# EUROPEAN PATENT APPLICATION

(11) **EP 4 454 706 A1**
(43) Date of publication of application: **30.10.2024**
(21) Application number: 23169557.8
(22) Date of filing: 24.04.2023
(51) Int. Cl.: A61P 35/00, C07K 16/28

(54) **NOVEL EGFR BINDING PROTEINS**

(71) Applicant: Universität Stuttgart, 70174 Stuttgart (DE)
(72) Inventor: KÜHL, Lennart, 72070 Tübingen (DE); KONTERMANN, Roland, 72622 Nürtingen (DE); OLAYIOYE, Monilola, 70469 Stuttgart (DE); SEIFERT, Oliver, 70437 Stuttgart (DE); KUHN, Philipp, 28355 Bremen (DE); RUSSO, Giulio, 9724 JZ Groningen (NL)
(74) Representative: Zwicker, Jörk

(57) **Abstract**

The present invention pertains to antigen-binding proteins binding to the ligand-binding site of EGFR and to clinically relevant EGFR escape mutations. Specifically, the present invention provides an antigen-binding protein comprising a first antibody variable domain and a second antibody variable domain, wherein the variable domains form a binding site that: specifically binds to a human epidermal growth factor (EGFR) variant having one or more of the mutations V441D, S464L, G465R, and S492R with a KD < 10⁻⁵ molar determined by biolayer interferometry (BLI); and that does not bind to human EGFR having the mutation Q435P or double mutation F436A/I462A with a KD > 10⁻⁵ molar determined by biolayer interferometry (BLI). The binding of the antigen-binding protein to human EGFR blocks ligand-induced activation of human EGFR.

## Description

The present invention relates to antigen binding proteins binding to the ligand-binding site of Epidermal Growth Factor Receptor (EGFR) and to clinically relevant EGFR escape mutations. The antigen binding proteins are capable of blocking the activation of EGFR and of overcoming acquired resistance observed in cancer patients that have been treated with anti-EGFR antibodies.

### Background of the invention

EGFR (also referred to as HER1) as a member of the ErbB (HER) receptor family is a membrane-bound tyrosine kinase that is activated upon ligand binding, leading to a conformational change and the formation of homo- or heterotypic receptor dimers with EGFR or one of the other three members of the receptor family (HER2, HER3, HER4), respectively (Roskoski, 2014, Pharmacol. Res. 79; 34-74). The extracellular region of EGFR comprises four domains. Domains I and III are involved in ligand binding, while domains II and IV contribute to receptor dimerization. Among the ligands of EGFR are EGF (epidermal growth factor), EPG (epigen), TGFα (transforming growth factor-α), ARG (amphiregulin), BTC (betacellulin), HB-EGF (heparin-binding epidermal growth factor and EPR (epiregulin). These ligand-activated receptor dimers phosphorylate tyrosine residues in the intracellular signaling domain of the receptors that function as binding sites for adaptor molecules, which in turn activate downstream signaling pathways. These activated signaling pathways eventually lead to increased proliferation, migration and survival of the cancer cells contributing to the malignant phenotype (Cai et al., 2020, Front. Oncol.24: 1249).

Elevated EGFR expression levels and activation states were found to be elevated in lung (NSCLC), prostate, breast, colon and rectum, head and neck, esophagogastric, liver, glioblastoma, cervix, ovary, bladder, kidney and pancreas cancer (Thomas & Weihua, 2019, Front. Oncol. 9:800). This led to the development of drugs targeting EGFR, including antibodies blocking receptor activation and small molecules (typrosine-kinase inhibitors, TKIs) that bind and inhibit the kinase domain (Wieduwelt & Moasser, 2008, Cell. Mol. Life Sci. 65: 1566-1584; Nedergaard et al., 2012, Biodrugs 26: 83-99; Dokala & Thakur, 2017, Oncogene 36:2337-2344). For both classes, molecules have been approved for treatment and a multitude of additional molecules are being investigated in preclinical and clinical studies, underlining the potential of EGFR as a target in cancer therapy (Kwapiszewski et al., 2016, Targ. Oncol. 11: 739-752; Yamaoka et al., Molecular Sciences 2017).

Several anti-EGFR antibodies have been approved for the treatment of CRC (colorectal cancer), HNSCC (head and neck squamous cell cancer), and NSCLC (non-small cell lung cancer). This includes cetuximab, panitumumab, necitumumab and nimotuzumab, *i.e.* human or humanized antibodies, all binding to domain III of EGFR. Cetuximab, panitumumab, and necitumumab bind to the same region of domain III which is part of the ligand binding site (Li et al., 2005, Cancer Cell 7: 301-311; Sickmier et al., 2016, PLoS ONE 11: e163366; Bagchi et al., 2018, Mol. Cancer Ther. 17: 521-531), although the three antibodies differ in the residues directly involved in antigen binding.

Cetuximab is approved for the treatment of patients with K-Ras wild-type, EGFR-expressing, metastatic colorectal cancer and recurrent or metastatic squamous cell carcinoma of the head and neck. Panitumumab is approved for the treatment of patients with wild-type RAS metastatic colorectal cancer. Necitumumab is approved for the treatment of patients with advanced squamous non-small cell lung cancer. Nimotuzumab is approved for the treatment of patients with squamous cell carcinoma of the head and neck.

Further antibodies, which have been studied in clinical trials or are currently in clinical development, include tomuzotuximab (CeuGEX), imgatuzumab (GA201), matuzumab (EMD72000), zalutumumab (huMax-EGFr), GC1118, JMT101, SCT200, ZZ06, and ABT-806, as well as antibody mixtures such as modotuximab + futuximab and MM-151.

Furthermore, anti-EGFR antibodies have been used to generate bispecific antibodies targeting EGFR and other surface receptors, such as cMET (amivantamab, MCLA-129, EMB-01, GB-263), HER3 (SI-B001), HGFR (CKD-702), and LGR5 (MCLA-158) for dual targeting and signal inhibition. Other examples include bispecific antibodies targeting EGFR and CD28 (REGN7075) for targeted immune co-stimulation, and 4-1BB (HLX35) for targeted checkpoint inhibition in cancer immunotherapy. For cancer immunotherapy, EGFR antibodies have furthermore been used to generate bispecific antibodies for the recruitment of T-cells (T-cell engagers; TCE) or NK cells by binding with the second arm to CD3 or CD 16. EGFR targeting antibody therapeutics include furthermore mono- or bispecific antibody-drug conjugates (including an approved cetuximab derivative (cetuximab sarotolacan), antibody fusion proteins, and CAR-T cells (Cai et al., 2020, Front. Oncol.24: 1249).

Though treatments with EGFR antibodies improve the overall outcome for patients, it has been found that multiple pre-existing or acquired resistance mechanisms limit the benefits for the individual patient. Pre-existing resistances include mutations in the downstream signaling molecules as for example in K-Ras, and patients carrying these mutations are excluded from therapy by diagnostic analysis of the K-Ras mutation status. Acquired resistances include an increased expression of ligands binding to EGFR (e.g. TGFα) and a compensatory expression of other receptors, e.g. members of the ErbB family (e.g. HER3) or other receptor families (e.g. c-Met).

Notably, mutations in the ectodomain of EGFR, being part of the epitope of approved anti-EGFR antibodies such as cetuximab and panitumumab, are observed in about 15-20% of patients treated with approved EGFR antibodies leading to a loss of binding of the therapeutic antibody. Frequently observed mutations after cetuximab treatment include V441D, S442R, G465R, and S492R within domain III (numbering based on the full-length EGFR sequence, SEQ ID NO: 54. As the approved antibodies cetuximab, necitumumab and panitumumab share a common epitope, several of these mutations (V441D, S442R, G465R) can also lead to a reduced or lost binding for necitumumab and panitumumab, resulting in resistance to treatment with these antibodies (Strickler et al., Cancer Discov., 2018, 8: 164-173; Cai et al., 2020, Front. Oncol.24: 1249).

There are currently no antibodies available that can broadly overcome the observed resistance mutations in the extracellular region of EGFR described above.

Thus, there is a need for fully human antagonistic anti-EGFR binding proteins and specifically antibodies capable of binding to the ligand binding site of EGFR and thus blocking receptor activation, receptor signaling and cell proliferation, and which further tolerate mutations within the ectodomain of EGFR emerging as resistance to antibody treatment. There is further a need for such antigen binding proteins for use in treating patients with EGFR-expressing tumors.

### Summary of the invention

By selection of a human antibody phage display library, novel human anti-EGFR antigen-binding proteins were identified that bind to domain III of EGFR, efficiently inhibiting EGFR activation, signal transduction and cell proliferation, and capable of binding to several clinically relevant EGFR escape mutations, including V441D, S442R, S464L, G465R, and S492R, within the ligand binding site of domain III. These antigen-binding proteins bind to a unique epitope comprising amino acid residues G434, Q435, F436 and I462, and are cross-reactive with mouse EGFR. The antigen-binding proteins possess superior inhibitory effects compared to cetuximab, necitumumab, panitumumab, GC1118 and matuzumab using cell lines expressing either the wild-type EGFR or mutated variants such as the clinically relevant mutations G465R and S492R. Thus, the antigen-binding proteins of the present invention allow treating cancers with wild-type EGFR and EGFR with clinically relevant escape mutations.

According to a first aspect, the present invention provides an antigen-binding protein comprising a first antibody variable domain and a second antibody variable domain and wherein the variable domains form a binding site that a) specifically binds to a human epidermal growth factor receptor (EGFR) variant having one or more of the mutations V441D, S464L, G465R, and S492R with a K_{D} < 10⁻⁵ molar determined by biolayer interferometry (BLI); and b) does not bind specifically to human EGFR having the mutation Q435P or double mutation F436A/I462A, wherein binding of the antigen-binding protein to human EGFR blocks ligand-induced activation of human EGFR.

According to one embodiment, the variable domains form a binding site that specifically binds to domain III of human EGFR according to SEQ ID NO: 61.

According to a further embodiment, the antigen-binding protein is selected from the group consisting of an antibody or an antigen-binding fragment thereof, and a chimeric antigen receptor (CAR). Preferably, the antigen-binding protein is selected from the group consisting Fab, Fab', (Fab')2, Fv, disulfide-linked Fv, BsFv, dsFv, (dsFv)2, dsFv-dsFv', scFv, scFv dimer, single chain domain antibody, diabody, ds diabody, and a bivalent domain antibody.

According to a preferred embodiment, the antibody is selected from the group consisting of a chimeric antibody, a humanized antibody, a bispecific antibody, and a multispecific antibody. The antibody is preferably a human or humanized antibody.

According to yet another embodiment, the first antibody variable domain comprises the light chain complementary determining region CDRL1 (CDRL1), CDRL2, CDRL3 of the variable light chain region of SEQ ID NO: 83, 84 or 85 and/or the second variable domain comprises the CDRH1, CDRH2 and CDRH3 of the heavy chain variable region of SEQ ID NO: 74 or 75, wherein the CDRs are identified by the Kabat definition, the Chothia definition, the IMGT definition or the contact definition, and wherein each CDR may comprise one or two amino acid substitutions.

According to a preferred embodiment, the first antibody variable domain comprises a CDRL1 of SEQ ID NO: 86, CDRL2 of SEQ ID NO: 87, and CDRL3 of SEQ ID NO: 88; and the second antibody variable domain comprises a CDRH1 of SEQ ID NO: 71 CDRH2 of SEQ ID NO: 72, and CDRH3 of SEQ ID NO: 73, and wherein each CDR may comprise one or two amino acid substitutions.

According to another preferred embodiment, the first antibody variable domain comprises a CDRL1 of SEQ ID NO: 76 or 77, CDRL2 of SEQ ID NO: 78 or 79, and CDRL3 of SEQ ID NO: 80, 81 or 82; and the second antibody variable domain comprises a CDRH1 of SEQ ID NO: 71, CDRH2 of SEQ ID NO: 72, and CDRH3 of SEQ ID NO: 73, and wherein each CDR may comprise one or two amino acid substitutions.

According to another embodiment, the first antibody variable domain comprises one or more of the framework (FR) sequences of SEQ ID NO: 83, 84 or 85, or a variant of the FR sequence having at least 90% sequence identity to the FR sequences of SEQ ID NO: 83, 84 or 85, and/or the second antibody variable domain comprises one or more of the FR sequences of SEQ ID NO: 74 or 75, or a variant of the FR sequence having at least 90% sequence identity to the FR sequences of SEQ ID NO: 74 or 75, wherein the FR are identified by the Kabat definition, the Chothia definition, the IMGT definition or the contact definition.

According to yet another embodiment the first variable domain comprises one of the amino acid sequences according to SEQ ID NO: 83, 84 or 85, and the second variable domain comprises one of the amino acid sequences according to SEQ ID NO: 74 or 75. According to a preferred embodiment, the antigen-binding protein comprises one of the combinations: SEQ ID NO: 74 and SEQ ID NO: 83, SEQ ID NO: 75 and SEQ ID NO: 84, or SEQ ID NO: 74 and SEQ ID NO: 85, and wherein each CDR may comprise one or two amino acid substitutions.

According to a particularly preferred embodiment, the antigen-binding protein comprises one of the sequence combinations: a) CDRH1, CDRH2 and CDRH3 of SEQ ID NOs: 71, 72 and 73, and CDRL1, CDRL2 and CDRL3 of SEQ ID NOs: 76, 78 and 80; b) CDRH1, CDRH2 and CDRH3 of SEQ ID NOs: 71, 72 and 73, and CDRL1, CDRL2 and CDRL3 of SEQ ID NOs: 77, 79 and 81; or c) CDRH1, CDRH2 and CDRH3 of SEQ ID NOs: 71, 72 and 73, and CDRL1, CDRL2 and CDRL3 of SEQ ID NOs: 77, 79 and 82, and wherein each CDR may comprise one or two amino acid substitutions.

According to one embodiment the antigen binding protein either competes for binding to an IgG1 antibody comprising the VL of SEQ ID NO: 83, 84 or 85 and the VH of SEQ ID NO: 74 or 75; or binds to the same epitope as an IgG1 antibody comprising the VL of SEQ ID NO: 83, 84 or 85 and the VH of SEQ ID NO: 74 or 75, and wherein each CDR may comprise one or two amino acid substitutions.

According to a further aspect, the present invention provides a nucleic acid or nucleic acids encoding the antigen-binding protein according to the invention.

According to another aspect, the present invention provides a vector comprising the nucleic acid or nuclei acids according to the invention.

According to a further aspect, the present invention provides a recombinant cell expressing the antigen-binding protein, the nucleic acid or nucleic acids, or the vector according to the invention.

The present invention further provides a pharmaceutical composition comprising the antigen-binding protein, the nucleic acid or nucleic acids, the vector, or the recombinant cell according to the invention, and optionally a pharmaceutically acceptable excipient.

According to a further aspect, the present invention provides the antigen-binding protein, the nucleic acid or nucleic acids, the vector, the recombinant cell, or the pharmaceutical composition according to the invention for use in medicine.

According to yet another aspect, the present invention provides the antigen-binding protein, the nucleic acid or nucleic acids, the vector, the recombinant cell, or the pharmaceutical composition according to the invention for use in the treatment of cancer.

According to a preferred embodiment, the cancer is selected from the group consisting of lung cancer, prostate cancer, breast cancer, colon cancer, rectum cancer, head cancer, neck cancer, esophagogastric cancer, liver cancer, glioblastoma, cervix cancer, ovary cancer, bladder cancer, kidney cancer, and pancreas cancer, preferably head and neck squamous cell cancer, non-small cell lung cancer, metastatic colorectal cancer, recurrent or metastatic squamous cell carcinoma of the head and neck. According to another aspect, the present invention provides a method of treating cancer, the method comprising administering to a patient in need thereof a therapeutically effective amount of the antigen-binding protein, the nucleic acid or nucleic acids, the vector, the recombinant cell, or the pharmaceutical composition of the invention. The cancer is preferably selected from the group consisting of lung cancer, prostate cancer, breast cancer, colon cancer, rectum cancer, head cancer, neck cancer, esophagogastric cancer, liver cancer, glioblastoma, cervix cancer, ovary cancer, bladder cancer, kidney cancer, and pancreas cancer, preferably head and neck squamous cell cancer, non-small cell lung cancer, metastatic colorectal cancer, recurrent or metastatic squamous cell carcinoma of the head and neck.

Further aspects and embodiments are disclosed in the accompanying claims and are derivable from the following detailed description of the invention.

### Description of the Figures

In the following, the content of the figures comprised in this specification is described. In this context please also refer to the detailed description of the invention above and/or below.

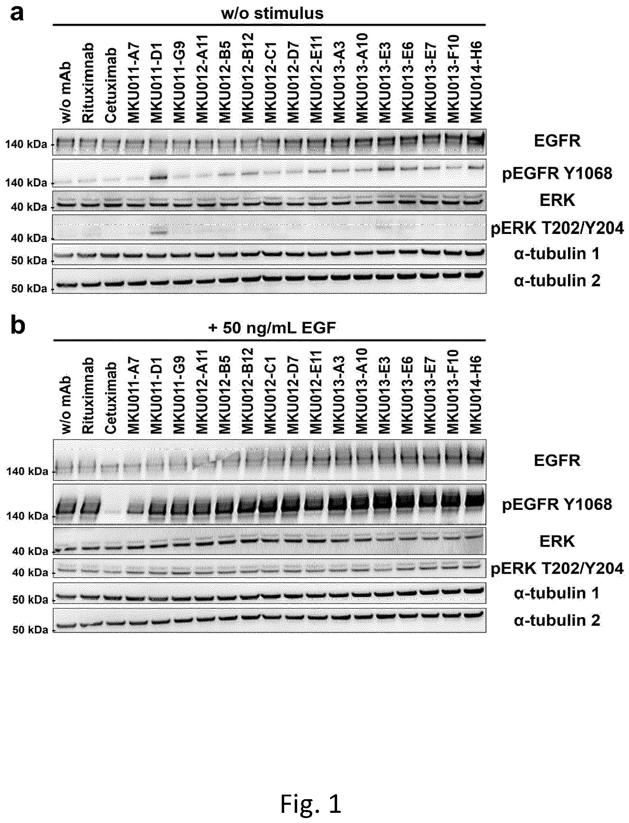

**Fig. 1** shows the inhibition of EGFR phosphorylation and downstream signaling for FaDu treated with 10 µg/mL anti-EGFR scFv-huFc either without stimulation (a) or for stimulation with 50 ng/mL EGF (b). (n=1)

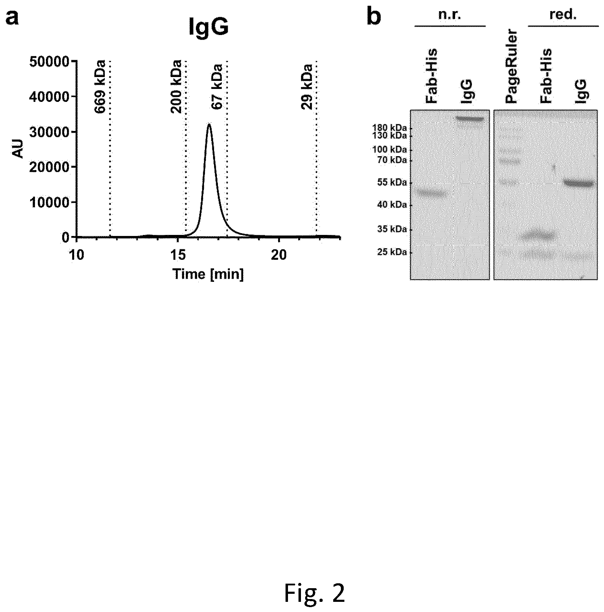

**Fig. 2** shows an SEC (a) and SDS-PAGE (b) analysis of IgG1 MKU011-A7 (a, b) and Fab-His MKU011-A7 (b) after transient expression in HEK293-6E and Protein A or Capture Select^{™} C_{H}1 XL purification.

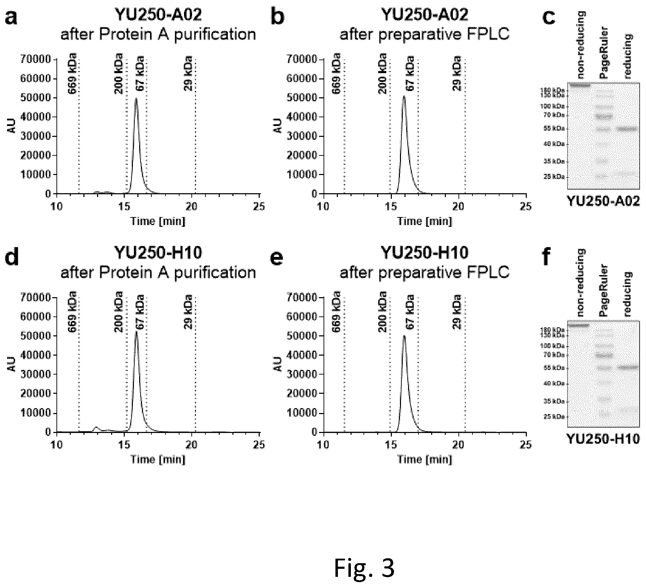

**Fig. 3** shows an SEC (a, b, d, e) and SDS-PAGE (c, f) analysis of IgG1 YU250-A02 (a - c) and YU250-H10 (d - e) after transient expression in HEK293-6E and Protein A purification.

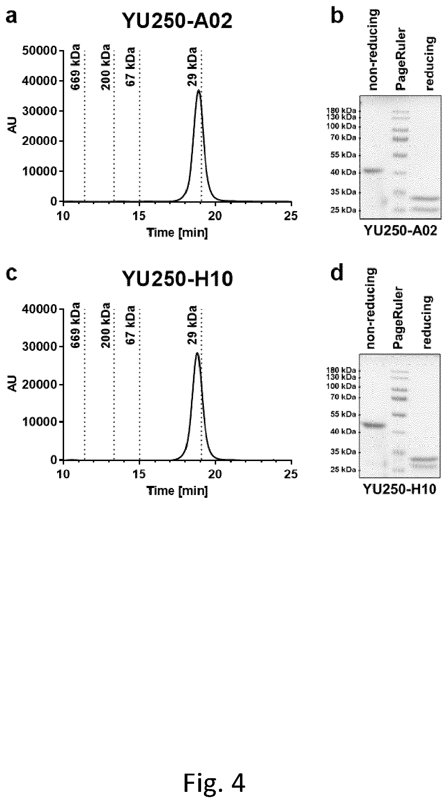

**Fig. 4** shows an SEC (a, c) and SDS-PAGE (b, d) analysis of Fab-His YU250-A02 (a, b) and YU250-H10 (c, d) after transient expression in HEK293-6E and Capture Select^{™} C_{H}1 XL purification.

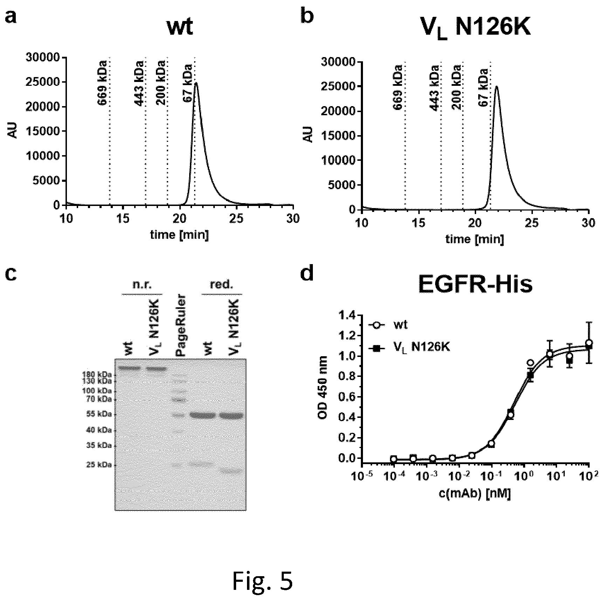

**Fig. 5** shows the analysis of IgG YU250-H10 wt and variant V_{L} N126K by SEC-HPLC (a, b), SDS-PAGE (c) and for binding to EGFR by ELISA (d) (n=1 - data shown as mean of duplicates ± S.D.).

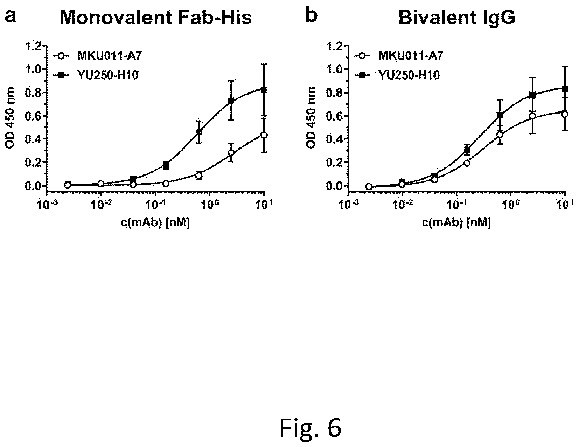

**Fig. 6** shows analysis of monovalent binding of Fab-His (a) and bivalent binding of IgG1 (b) for MKU011-A7 and YU250-H10 by EGFR-binding ELISA (n=3 - data shown as mean ± S.D.).

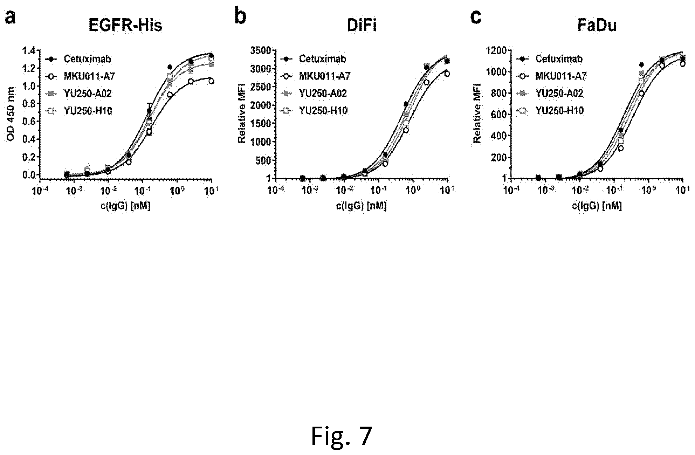

**Fig. 7** shows the analysis of binding to EGFR by ELISA (a) and to EGFR-expressing cancer cells DiFi (b) and FaDu (c) by flow cytometry for Cetuximab, IgG MKU011-A7, IgG YU250-A02 and IgG YU250-H10 (n=1 - data shown as mean of duplicate ± S.D.).

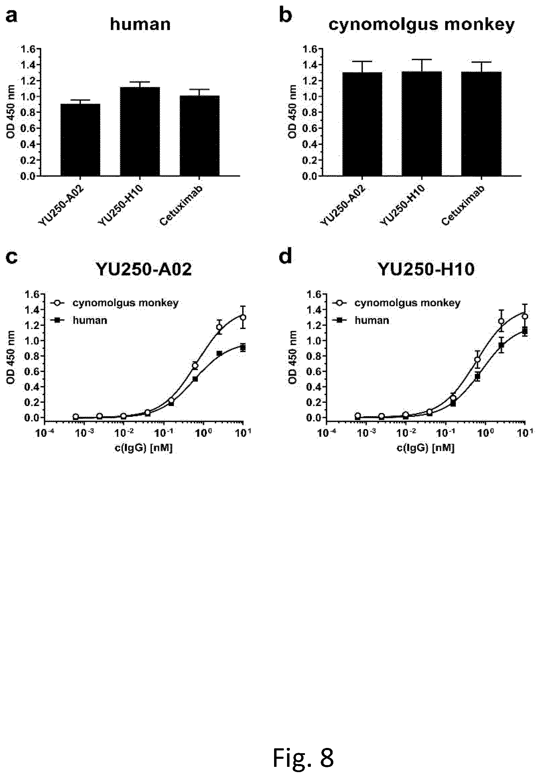

**Fig. 8** shows the analysis of binding to human (a) and cynomolgus monkey EGFR (b) for 10 nM IgG YU250-A02, IgG YU250-H10 and Cetuximab by ELISA. Determination of EC₅₀ values for both cynomolgus monkey and human EGFR for IgG YU250-A02 (c) and IgG YU250-H10 (d) by ELISA (n=3 - data shown as mean ± S.D.).

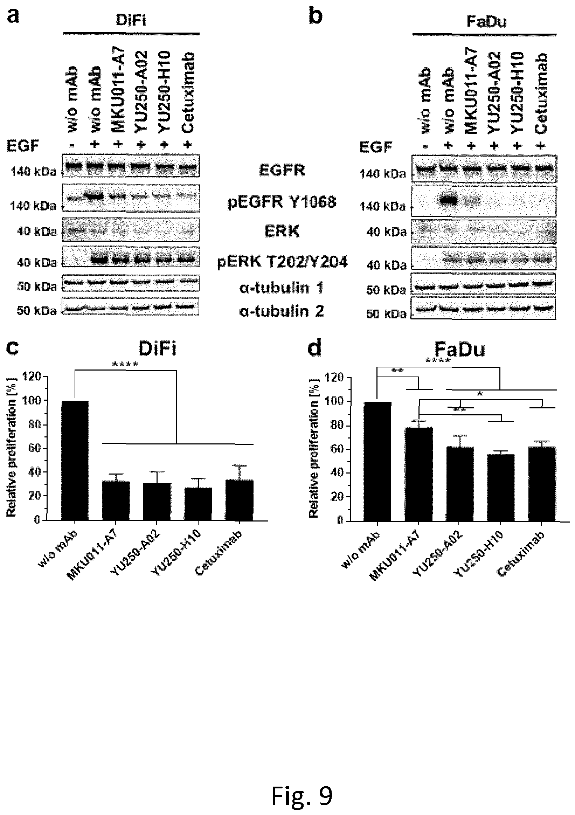

**Fig. 9** shows the inhibition of EGFR phosphorylation and MAPK pathway signaling for DiFi (a) and FaDu (b) for anti-EGFR IgG MKU011-A7, YU250-A02, YU250-H10 and Cetuximab. Analysis of proliferation for DiFi (c) and FaDu (d) treated with anti-EGFR IgG (n=3 - data shown as mean ± S.D. - normalized to cells w/o mAb - statistics: one-way ANOVA with Tukey post-hoc test - p=0.05 / * - p<0.05 / ** - p<0.01 / *** - p<0.001 / **** - p<0.0001).

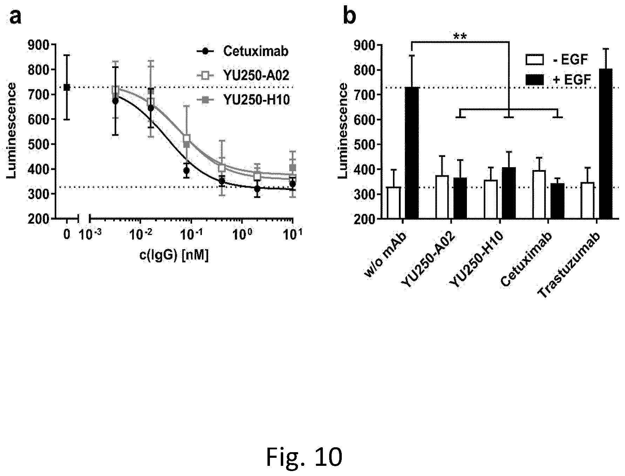

**Fig. 10** shows the inhibition of EGFR dimerization by anti-EGFR IgG in a β-galactosidase complementation assay analyzed for a concentration range (a) or at a concentration of 10 nM (b) using 30 ng/mL EGF as stimulus (n=3 - data shown as mean ± S.D. - statistics: one-way ANOVA with Tukey post-hoc test - p=0.05 / * - p<0.05 / ** - p<0.01).

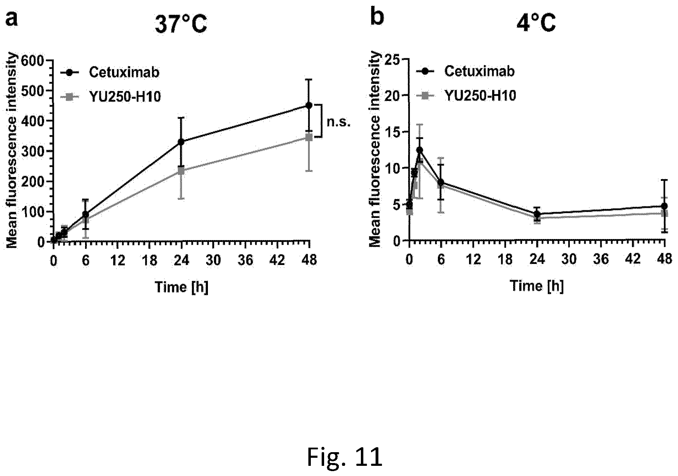

**Fig. 11** shows the analysis of internalization of pH-rodo labelled Cetuximab and IgG YU250-H10 into FaDu cells for 37°C (a) and 4°C (b) (n=3 - data shown as mean ± S.D. - statistics for 48 h at 37°C: t-test - p=0.05 / n.s. - not significant).

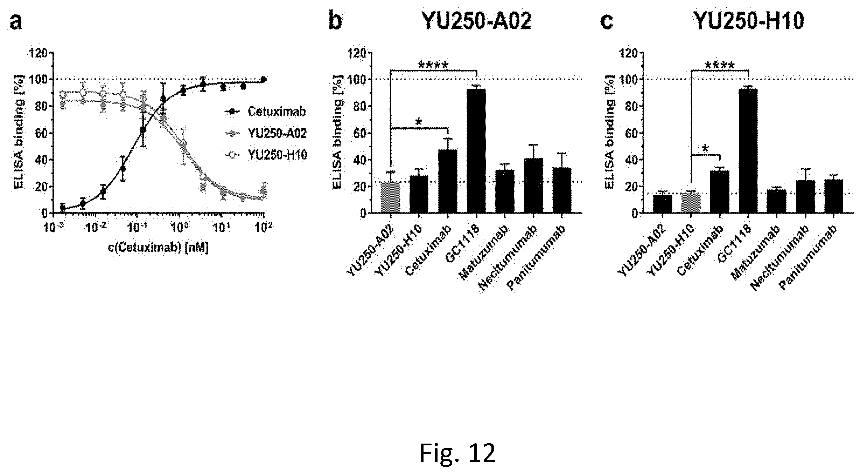

**Fig. 12** shows the analysis of competitive binding for Cetuximab versus scFv-huFc YU250-A02 and YU250-H10 (a) and for Fab-His YU250-A02 (b) or YU250-H10 (c) versus anti-EGFR IgG by ELISA (n=3 - data shown as mean ± S.D. - normalized to binding of Cetuximab at 100 nM (a) or to binding of anti-EGFR IgG without competitor (b, c) - statistics: one-way ANOVA with Tukey post-hoc test - p=0.05 / * - p<0.05 / ** - p<0.01 / *** - p<0.001 / **** - p<0.0001).

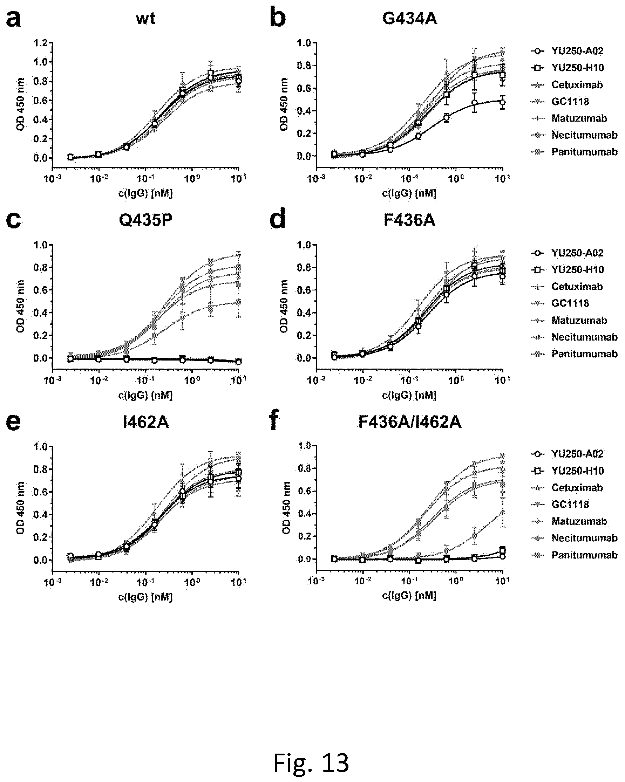

**Fig. 13** shows the analysis of binding to EGFR wt (a), G434A (b), Q435P (c), F436A (d), I462A (e) and F436A/I462A (f) for anti-EGFR IgG YU250-A02, YU250-H10, Cetuximab, GC1118, Matuzumab, Necitumumab and Panitumumab by ELISA (n=3 - data shown as mean ± S.D.).

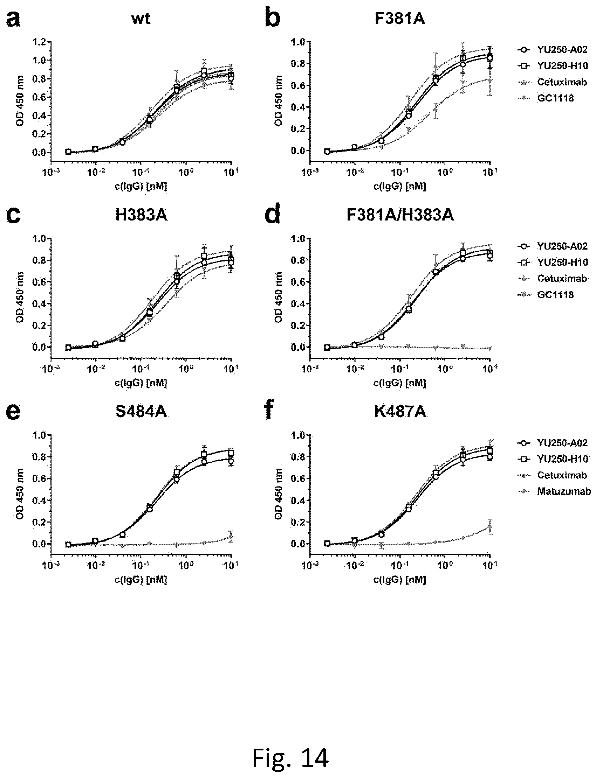

**Fig. 14** shows the analysis of binding to EGFR wt (a), F381A (b), H383A (c) and F381A/H383A (d) for anti-EGFR IgG YU250-A02, YU250-H10, Cetuximab and GC1118 as well as for EGFR S484A (e) and K487 (f) for anti-EGFR IgG YU250-A02, YU250-H10, Cetuximab and Matuzumab by ELISA (n=3 - data shown as mean ± S.D.).

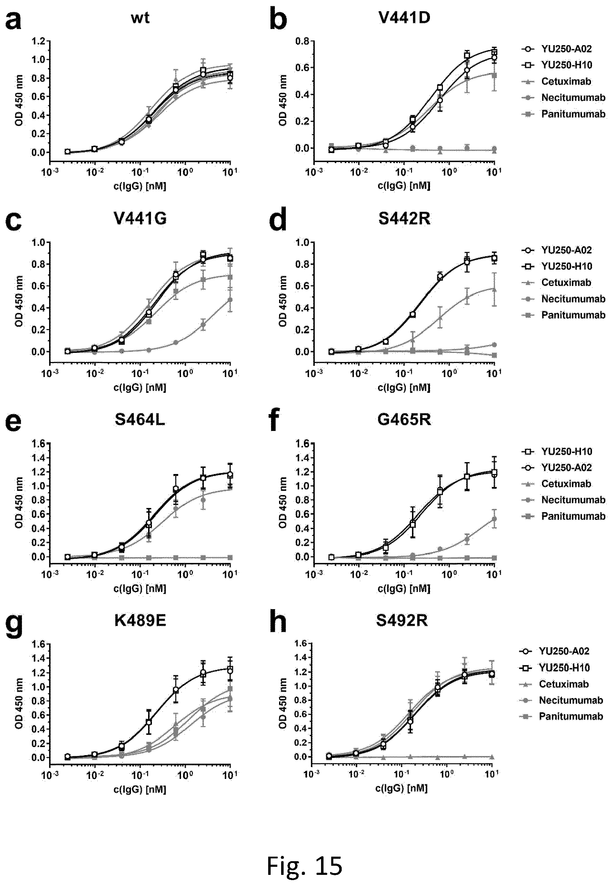

**Fig. 15** shows the analysis of binding to EGFR wt (a), V441D (b), V441G (c), S442R (d), S464L (e), G465R (f), K489E (g) and for S492R (h) for anti-EGFR IgG YU250-A02, YU250-H10, Cetuximab, Necitumumab and Panitumumab by ELISA (n=3 - data shown as mean ± S.D.).

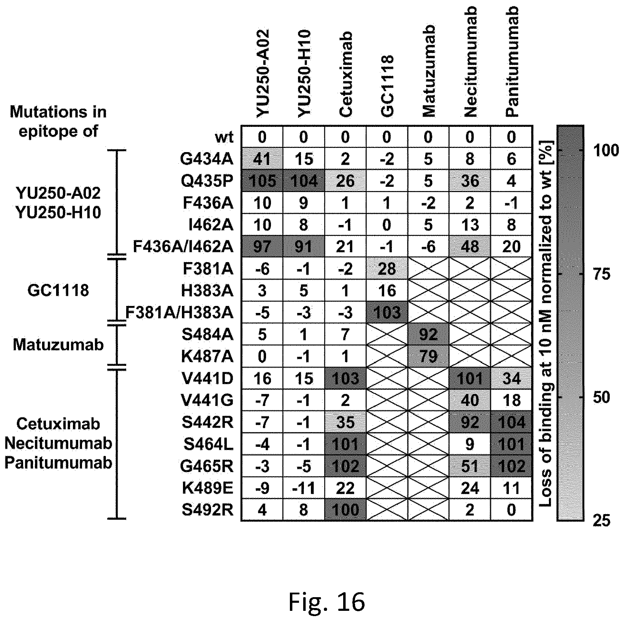

**Fig. 16** shows the summary of the epitope mapping by introduction of mutations into EGFR resulting in a loss of binding for anti-EGFR IgG YU250-A02, YU250-H10, Cetuximab, GC1118, Matuzumab, Necitumumab and Panitumumab (data shown as mean of n=3 for loss of binding normalized to wt [%] at a concentration of 10 nM).

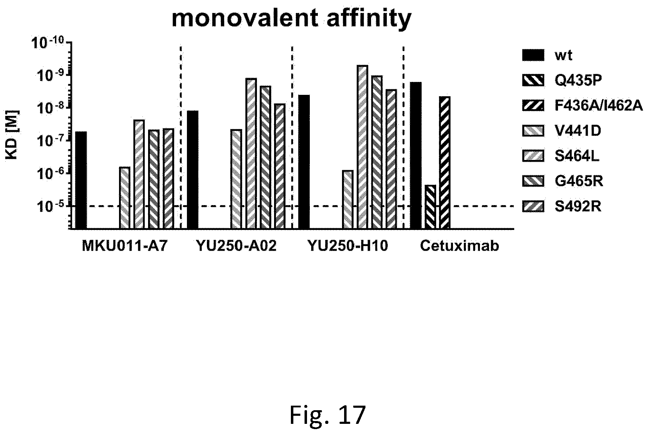

**Fig. 17** shows the affinity (K_{D}) of monovalent anti-EGFR Fab to His-tagged extracellular domains of EGFR analyzed by biolayer interferometry.

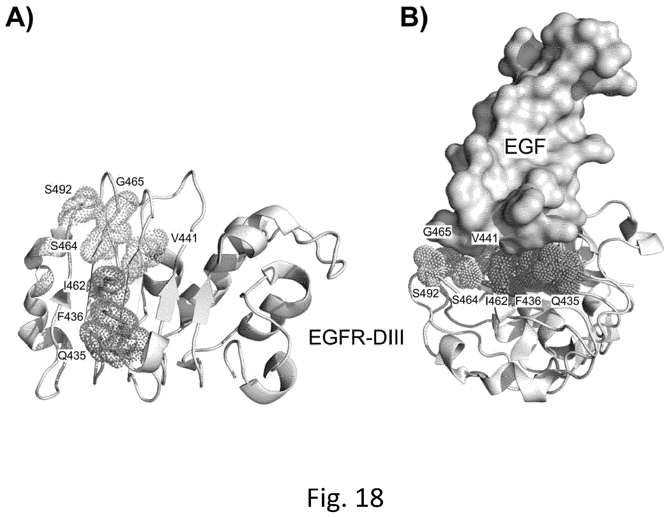

**Fig. 18** shows the structure of human EGFR domain III with the positions Q435P and F436A/I462A (cluster 1) affecting binding of antibodies MKU011-A7, YU250-A02 or YU250-H10, and positions V441D, S464L, G465R and S492R (cluster 2) affecting binding of cetuximab being highlighted. A) top view of domain III, B) side view of domain III in complex with EGF. The two clusters are structurally separated, but located at the EGF-EGFR-DIII interface.

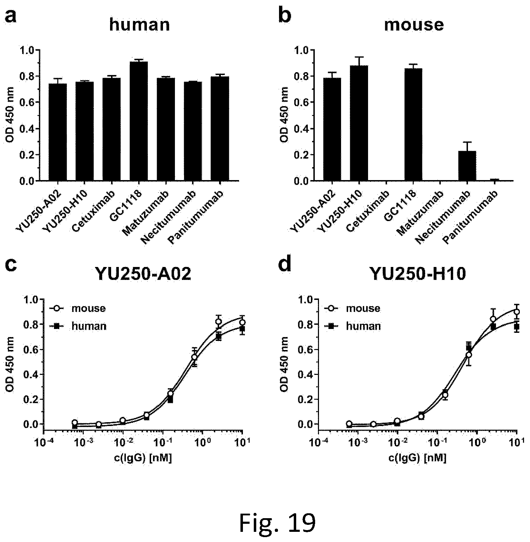

**Fig. 19** shows the analysis of binding to human (a) and murine EGFR (b) for 10 nM IgG YU250-A02, IgG YU250-H10, Cetuximab, GC1118, Matuzumab, Necitumumab and Panitumumab by ELISA. Determination of EC₅₀ values for both murine and human EGFR for IgG YU250-A02 (c) and IgG YU250-H10 (d) by ELISA (n=3 - data shown as mean ± S.D.).

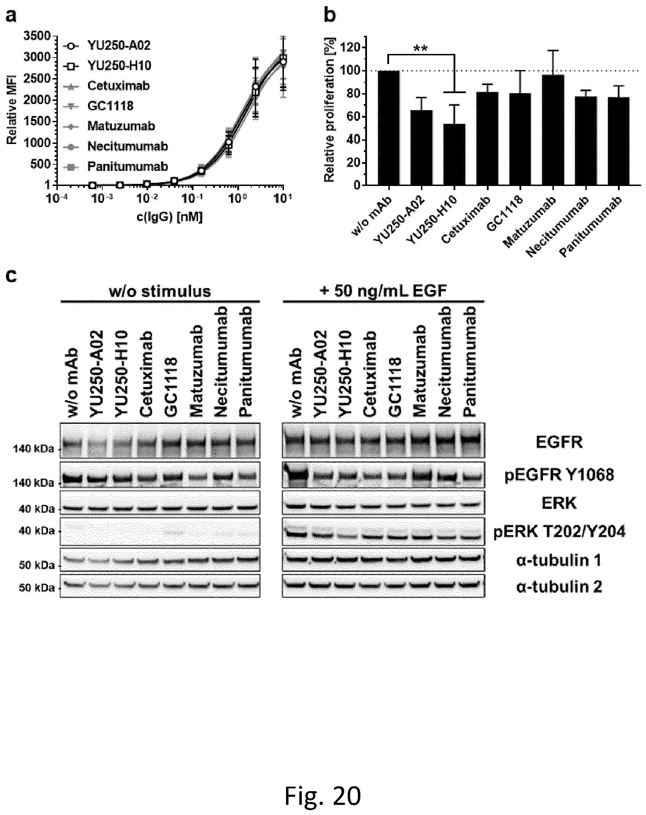

**Fig. 20** shows the analysis of binding for anti-EGFR IgG to A431 (a), inhibition of proliferation for A431 (b) and inhibition of EGFR phosphorylation and MAPK pathway signaling (c) (n=3 for binding and proliferation / n=1 for signaling inhibition - data shown as mean ± S.D. - proliferation normalized to cells w/o mAb - statistics: one-way ANOVA with Tukey post-hoc test - p=0.05 / * - p<0.05).

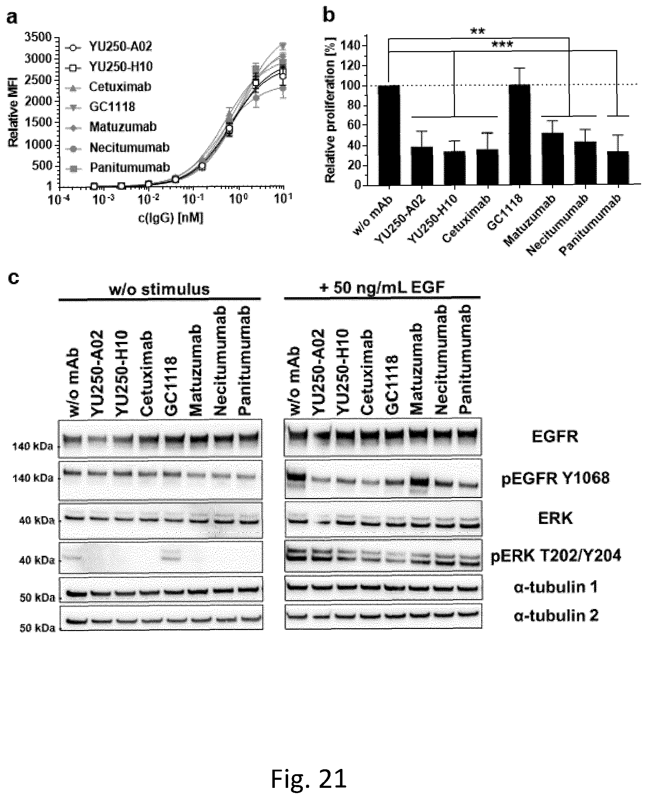

**Fig. 21** shows the analysis of binding for anti-EGFR IgG to DiFi (a), inhibition of proliferation for DiFi (b) and inhibition of EGFR phosphorylation and MAPK pathway signaling (c) (n=3 for binding and proliferation / n=2 for signaling inhibition - data shown as mean ± S.D. - proliferation normalized to cells w/o mAb - statistics: one-way ANOVA with Tukey post-hoc test - p=0.05 / * - p<0.05 / ** - p<0.01 / *** - p<0.001).

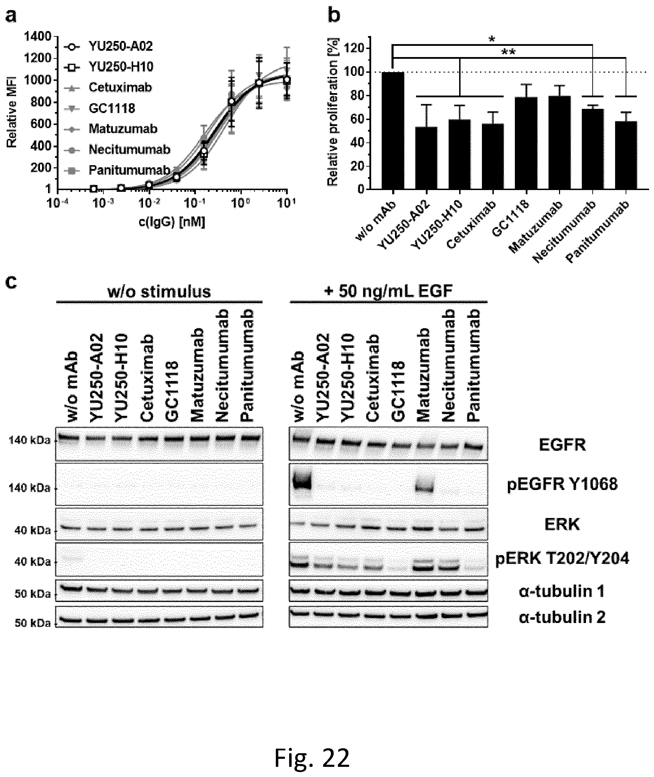

**Fig. 22** shows the analysis of binding for anti-EGFR IgG to FaDu (a), inhibition of proliferation for FaDu (b) and inhibition of EGFR phosphorylation and MAPK pathway signaling (c) (n=3 for binding and proliferation / n=1 for signaling inhibition - data shown as mean ± S.D. - proliferation normalized to cells w/o mAb - statistics: one-way ANOVA with Tukey post-hoc test - p=0.05 / * - p<0.05 / ** - p<0.01).

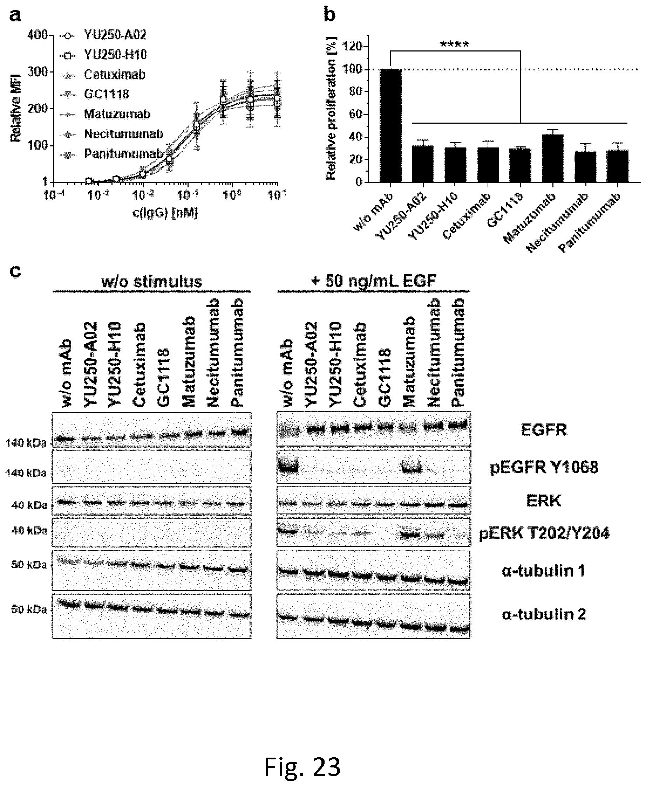

**Fig. 23** shows the analysis of binding for anti-EGFR IgG to LIM1215 (a), inhibition of proliferation for LIM1215 (b) and inhibition of EGFR phosphorylation and MAPK pathway signaling (c) (n=3 for binding and proliferation / n=1 for signaling inhibition - data shown as mean ± S.D. - proliferation normalized to cells w/o mAb - statistics: one-way ANOVA with Tukey post-hoc test - p=0.05 / * - p<0.05 / ** - p<0.01 / *** - p<0.001 / **** - p<0.0001).

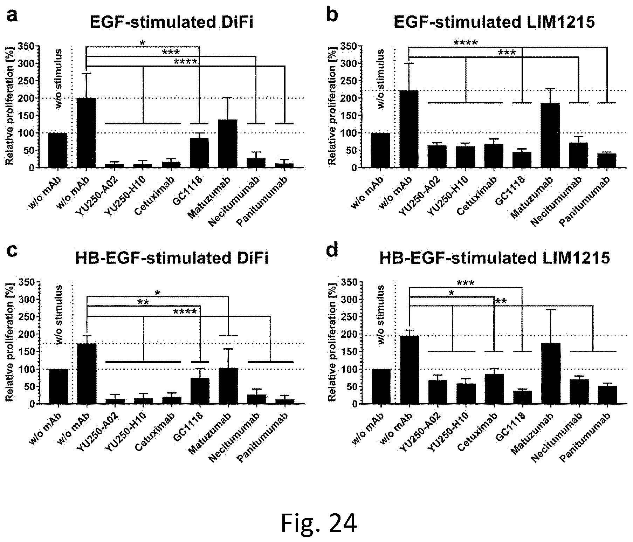

**Fig. 24** shows the inhibition of proliferation for human cancer cell lines DiFi (a / c) and LIM1215 (b / d) stimulated with EGF (a / b) or HB-EGF (c / d) (n=3 - normalized to cells w/o mAb - statistics: one-way ANOVA with Tukey post-hoc test - p=0.05 / * - p<0.05 / ** - p<0.01 / *** - p<0.001 / **** - p<0.0001).

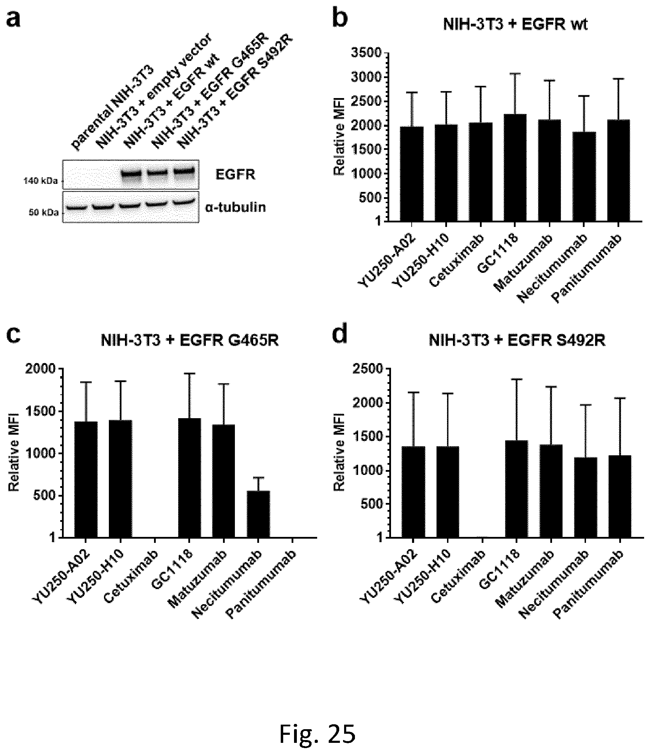

**Fig. 25** shows the analysis of EGFR expression for stable NIH-3T3 cell lines by Western blot (a) and binding of anti-EGFR IgG to the cell lines NIH-3T3 + EGFR wt (b), NIH-3T3 + EGFR G465R (c) and NIH-3T3 + EGFR S492R (d) analyzed by flow cytometry (data shown as mean ± S.D. - n=3).

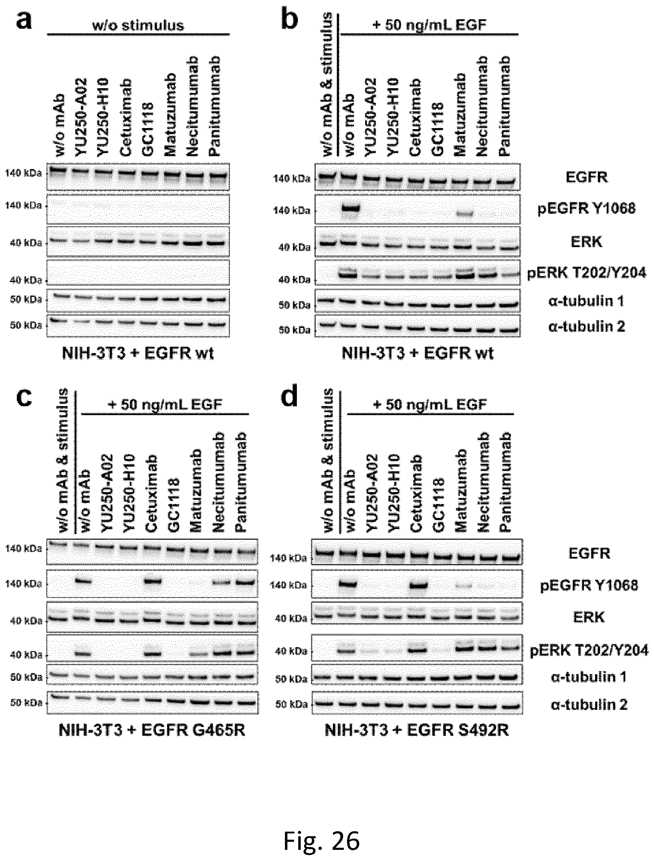

**Fig. 26** shows the inhibition of EGFR phosphorylation and activation of the MAPK pathway by anti-EGFR IgG for stable murine fibroblast cell lines NIH-3T3 + EGFR wt (a / b), NIH-3T3 + EGFR G465R (c) and NIH-3T3 + EGFR S492R (d) either without stimulus (a) or for stimulation with EGF (b - c) (n=1).

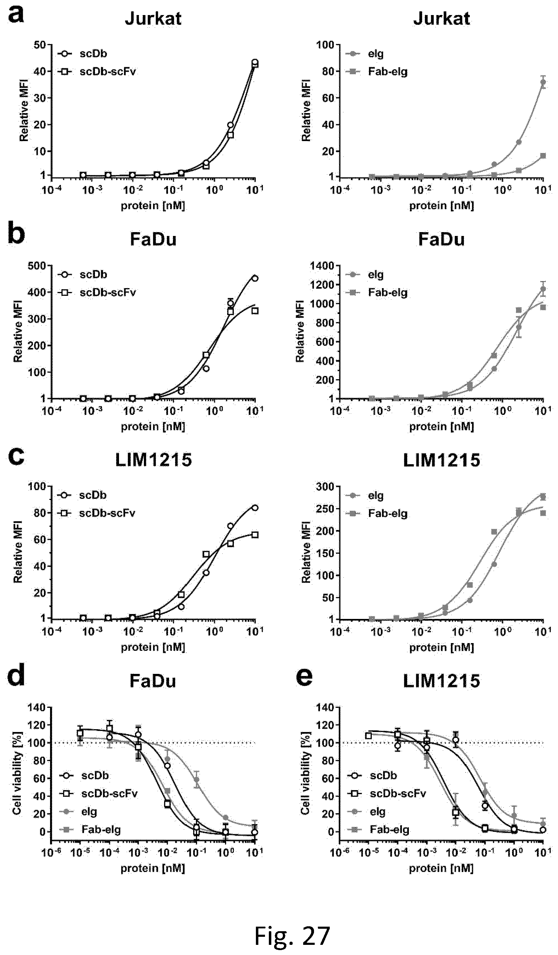

**Fig. 27** shows binding of bispecific EGFRxCD3 antibodies to CD3 on Jurkat cells (a) and to EGFR on cancer cells FaDu (b) and LIM1215 (c) analyzed by flow cytometry. Cytotoxicity assay on FaDu (d) and LIM1215 (e) for co-culture with PBMCs isolated from one healthy donors (n=1 for cell binding shown as mean of duplicate ± S.D. - n=3 for cytotoxicity shown as mean ± S.D. normalized to untreated target cells).

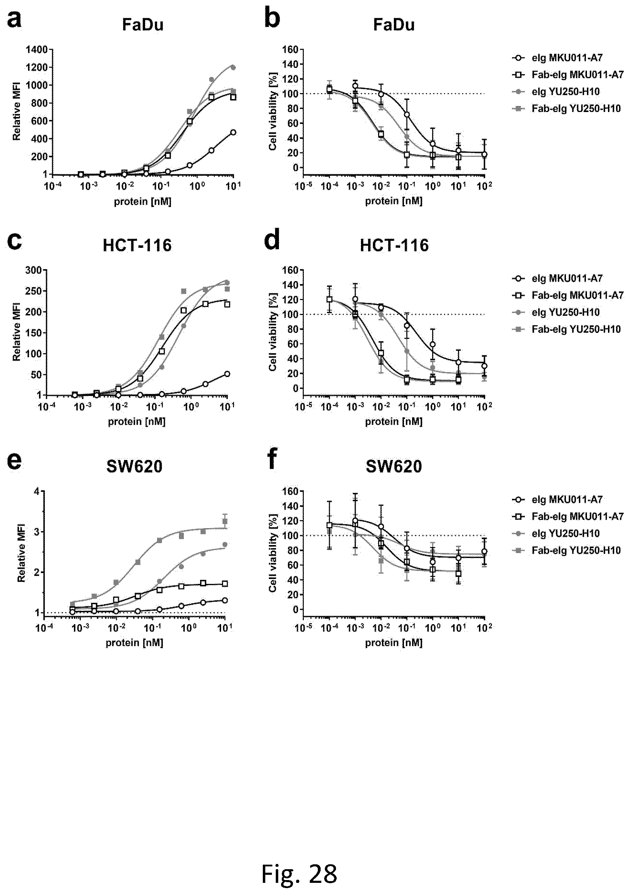

**Fig. 28** shows cell binding (a, c, e) and cytotoxicity (b, d, f) for bispecific EGFRxCD3 antibodies on FaDu (a, b), HCT-116 (c, d) and SW620 (e, f) (n=1 for cell binding - data shown as mean of duplicate ± S.D. / n=3 for cytotoxicity with three different donors normalized to untreated target cells - data shown as mean ± S.D.).

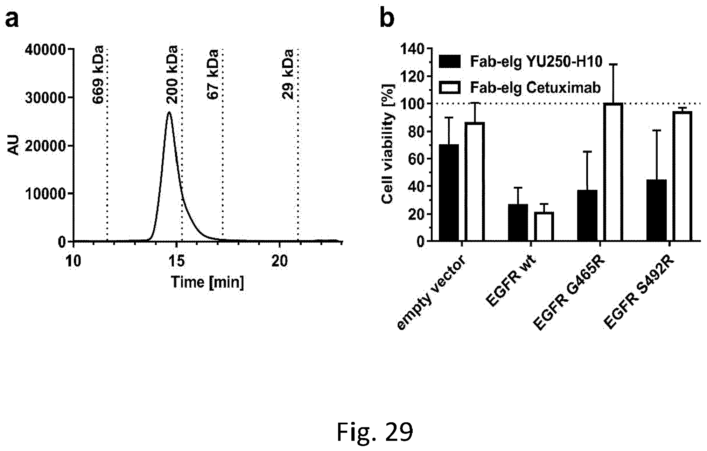

**Fig. 29** shows the quality control of Fab-eIg Cetuximab by SEC-HPLC (a). Analysis of cytotoxicity for bispecific EGFRxCD3 antibodies on stable NIH-3T3 cell lines expressing EGFR wt, G465R or S492R (b) comparing the two anti-EGFR antibodies YU250-H10 and Cetuximab (n=3 - three different donors normalized to untreated target cells - data shown as mean ± S.D.).

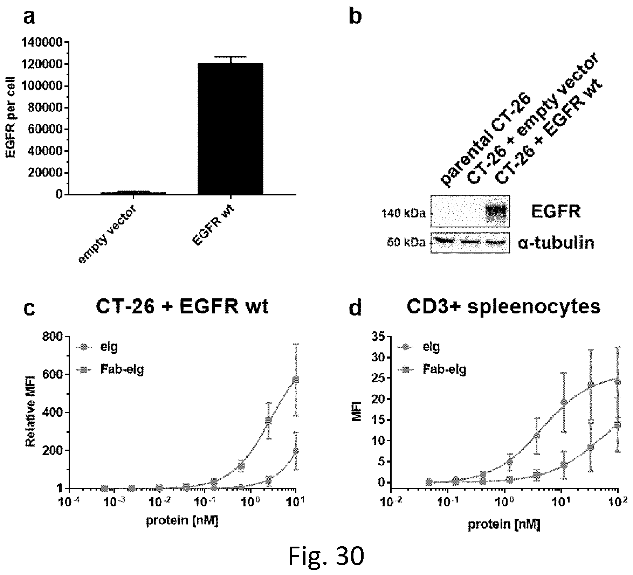

**Fig. 30** shows the quality control of stable CT-26 cell lines by flow cytometry quantifying the EGFR expression level (a) and by Western blot (b). Binding of bispecific EGFRxCD3 antibodies being cross-reactive to murine CD3 analyzed for binding to stable CT-26 + EGFR wt (c) and CD3+ spleenocytes (d) by flow cytometry (n=3 (a / c) / n=4 (d)- data shown as mean ± S.D.).

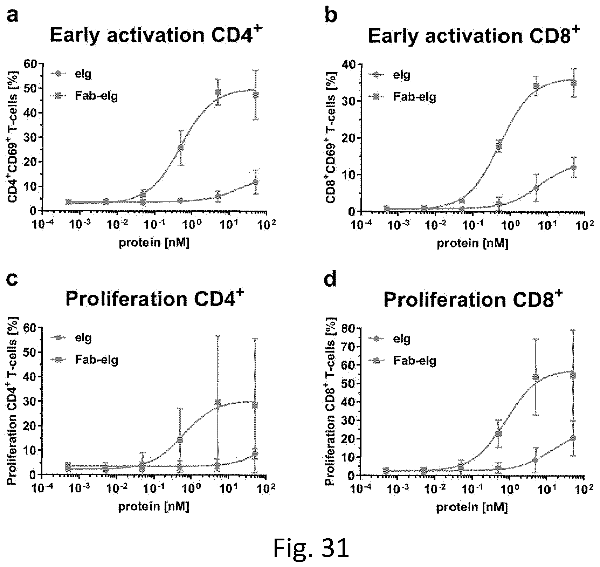

**Fig. 31** shows T-cell activation by surrogates of bispecific EGFRxCD3 antibodies analyzed for spleenocytes isolated from BALB/c: CD69⁺ T-cells of the CD4⁺ (a) and CD8⁺ (b) subtypes / T-cell proliferation for CD4⁺ (c) and CD8⁺ (d) subtypes (n=3 - data shown as mean ± S.D.).

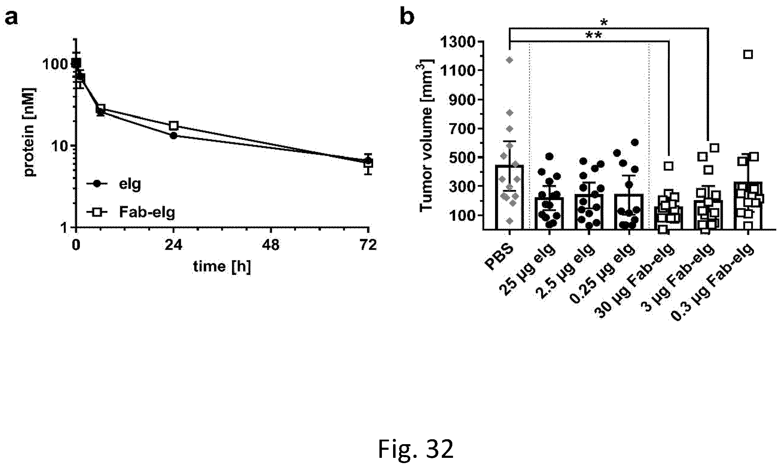

**Fig. 32** shows the investigation of surrogates for bispecific EGFRxCD3 antibodies *in vivo.* Pharmacokinetics for eIg and Fab-eIg was analyzed in BALB/c (a) and tumor volume of CT-26 + EGFR wt in BALB/c treated with different doses of eIg or Fab-eIg after 17 days (b) (three mice per group for pharmacokinetics - data shown as mean ± S.D. / six to seven mice per group for tumor model - data shown as mean ± 95% CI - statistics: one-way ANOVA with Tukey post-hoc test - p=0.05 / * - p<0.05 / ** - p<0.01).

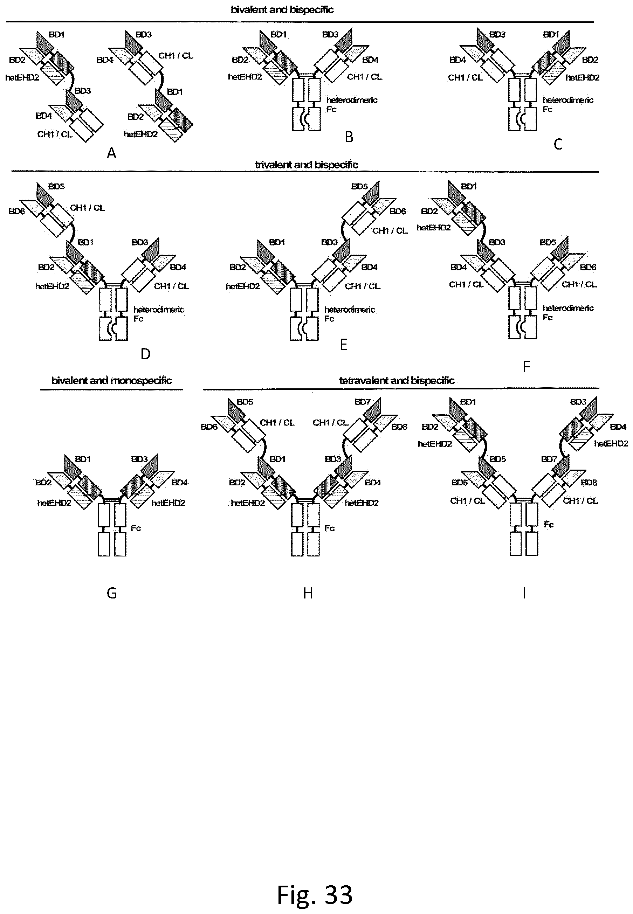

**Fig. 33** shows embodiments of formats of the antigen binding protein of the present invention comprising heterodimerization domains. The term "BD" denotes a binding domain, and two binding domains together form an antigen binding site, e.g. BD1 and BD2 together form a first binding site binding a first antigen, and BD3 and BD4 together form a second binding site binding the same or a different antigen.

### Sequences

The sequences referred to herein are listed in the accompanying sequence listing. Particularly relevant sequences and combinations thereof are additionally cited in the table below. CDR sequences listed in the table below have been defined in accordance with Kabat.

| SEQID NO | description | sequence |
|---|---|---|
| 1 to 3 | heavy chain leader sequences | |
| 4 and 10 | IgG MKU-011-A07 | |
| 4 and 5 | Fab-His MKU011-A7 | |
| 6 and 7 | IgG YU250-H10 | |
| 6 and 8 | Fab-His YU250-H10 | |
| 9 and 10 | IgG YU250-H10 | |
| 9 and 11 | Fab-His YU250-H10 | |
| 11 and 12 | IgG GC1118 | |
| 13 and 14 | IgG Matuzumab | |
| 15 and 16 | IgG Necitumumab | |
| 17 and 18 | IgG Panitumumab | |
| 19 | scDb-YU250-H10 x huU3 | |
| 20 | scDb-YU250-H10 x huU3-scFv-YU250-H10 | |
| 9, 21, 23, 24 | eIg YU250-H10xhuU3 & MKU011-A7xhuU3 | |
| 9, 21, 22, 24 | Fab-elg YU250-H10xhuU3 & MKU011-A7xhuU3 | |
| 25, 26, 27,28 | elg YU250-H10(hetEHD2)x2C11(CH1 /CL) | |
| 25, 26, 27,29 | Fab-elg YU250-H10(hetEHD2)x2C11(CH1 /CL) | |
| 24, 30, 31, 32 | Fab-elg Cetuximab x huU3 | |
| 33 to 53 | EGFR variants | |
| 54 to 56 | expression vectors | |
| 57 and 58 | glycosylation variants of YU250-H10 VL and VH | |
| 59 | EGFR-α-peptide | |
| 60 | EGFR- α-acceptor | |
| 61 | human EGFR domain III | |
| 62 | VH Cetuximab CH1-His | |
| 63 to 68 | EGFR variants | |
| 69 | human EGFR extracellular domains I-IV | |
| 70 | murine EGFR extracellular domains I-IV | |
| 71 | VH CDR1 of A7, A02, and H10 | SYGMH |
| 72 | VH CDR2 of A7, A02, and H10 | FIRYDGSNKYYADSVKG |
| 73 | VH CDR3 of A7, A02, and H10 | DGNSGYDSMDY |
| 74 | VH of A7 and H10 | QVQLVQSGGGVVQPGGSLRLSCAASGFTFSSYGMHWVRQAPGK GLEWVAFIRYDGSNKYYADSVKGRFTISRDNSKNTLYLQMNSLRA EDTAVYYCAKDGNSGYDSMDYWGQGTLVTVSS QVQLQESGGGVVQPGGSLRLSCAASGFTFSSYGMHWVRQAPGK GLEWVAFIRYDGSNKYYADSVKGRFTISRDNSKNTLYLQMNSLRA EDTAVYYCAKDGNSGYDSMDYWGQGTLVTVSS |
| 75 | VH of A02 | QVQLQESGGGVVQPGGSLRLSCAASGFTFSSYGMHWVRQAPGK GLEWVAFIRYDGSNKYYADSVKGRFTISRDNSKNTLYLQMNSLRA EDTAVYYCAKDGNSGYDSMDYWGQGTLVTVSS |
| 76 | VL CDR1 of A7 | SGVSSNIGRNYVY |
| 77 | VL CDR1 of A02, H10 | SGGSSNVGRNGVN |
| 78 | VL CDR2 of A7 | ATHQRPS |
| 79 | VL CDR2 of A02 and H10 | YDAFLSS |
| 80 | VL CDR3 of A7 | AAWDDGVTGWV |
| 81 | VL CDR3 of A02 | ASWDDSLTGWV |
| 82 | VL CDR3 of H10 | AAWDDSLYSWV |
| 83 | VL of A7 | QPVLTQSPSASGTPGQRVTISCSGVSSNIGRNYVYWYQQFPGTAP KLLIYATHQRPSGVPDRFSGSKSGTSASLAISGLRSEDEANYYCAAW DDGVTGWVFGGGTKLTVL |
| 84 | VL of A02 | QSVLTQPPSVSGAPGQRVTISCSGGSSNVGRNGVNWFQHLPEKA PKLLIYYDAFLSSGVSDRFSGSKSGTSASLAIRGLQSEDEADYYCAS WDDSLTGWVFGGGTKLTVL |
| 85 | VL of H10 | QSVLTQPPSVSGAPGQRVTISCSGGSSNVGRNGVNWFQHLPEKA PKLLIYYDAFLSSGVSDRFSGSKSGTSASLAIRGLQSDDEADYYCAA WDDSLYSWVFGGGTNLTVL |
| 86 | CDRL1 consensus | SG(V,G)SSN(I,V)GRN(Y,G)V(Y,N) |
| 87 | CDRL2 consensus | (A,Y) (T, D) (H,A) (Q, F) (R, L) (P,S)S |
| 88 | CDRL3 consensus | A(A,S)WDD(G,S)(V,L)(T,Y)(G,S)WV |
| 89 | VL of MKU012-E11 | |
| 90 | VH of MKU012-E11 | |
| 91 | wt human EHD2 core sequence | |
| 92 | scFv YU250-A02 Fc | |
| 93 | scFv YU250-H10 Fc | |
| 94 | hetEHD2 (1^{st} domain) | |
| 95 | hetEHD2 (2^{nd} domain) | |
| 96 | VH of huU3 | |
| 97 | VL of huU3 | |
| 120 | VH of 2C11 | |
| 121 | VL of 2C11 | |

The following table indicates the CDR sequences comprised in the variable heavy chains of preferred antibodies of the invention as defined using the Kabat definition, the IMGT definition, the Chothia definition, or the Contact definition:

| | | **VH_CDR1** | **VH_CDR2** | **VH_CDR3** |
|---|---|---|---|---|
| Kabat | **A7** | SYGMH (SEQ ID NO: 71) | FIRYDGSNKYYADSVKG (SEQ ID NO: 72) | DGNSGYDSMDY (SEQ ID NO: 73) |
| | **A02** | SYGMH (SEQ ID NO: 71) | FIRYDGSNKYYADSVKG (SEQ ID NO: 72) | DGNSGYDSMDY (SEQ ID NO: 73) |
| | **H10** | SYGMH (SEQ ID NO: 71) | FIRYDGSNKYYADSVKG (SEQ ID NO: 72) | DGNSGYDSMDY (SEQ ID NO: 73) |

| | | **VH_CDR1** | **VH_CDR2** | **VH_CDR3** |
|---|---|---|---|---|
| IMGT | **A7** | GFTFSSYG (SEQ ID NO: 98) | IRYDGSNK (SEQ ID NO: 99) | AKDGNSGYDSMDY (SEQ ID NO: 100) |
| | **A02** | GFTFSSYG (SEQ ID NO: 98) | IRYDGSNK (SEQ ID NO: 99) | AKDGNSGYDSMDY (SEQ ID NO: 100) |
| | **H10** | GFTFSSYG (SEQ ID NO: 98) | IRYDGSNK (SEQ ID NO: 99) | AKDGNSGYDSMDY (SEQ ID NO: 100) |

| | | **VH_CDR1** | **VH_CDR2** | **VH_CDR3** |
|---|---|---|---|---|
| Chothia | **A7** | GFTFSSY (SEQ ID NO: 101) | RYDGSN (SEQ ID NO: 102) | DGNSGYDSMDY (SEQ ID NO: 103) |
| | **A02** | GFTFSSY (SEQ ID NO: 101) | RYDGSN (SEQ ID NO: 102) | DGNSGYDSMDY (SEQ ID NO: 103) |
| | **H10** | GFTFSSY (SEQ ID NO: 101) | RYDGSN (SEQ ID NO: 102) | DGNSGYDSMDY (SEQ ID NO: 103) |

| | | **VH_CDR1** | **VH_CDR2** | **VH_CDR3** |
|---|---|---|---|---|
| Contact | **A7** | SSYGMH (SEQ ID NO: 104) | WVAFIRYDGSNKY (SEQ ID NO: 105) | DGNSGYDSMD (SEQ ID NO: 106) |
| | **A02** | SSYGMH (SEQ ID NO: 104) | WVAFIRYDGSNKY (SEQ ID NO: 105) | DGNSGYDSMD (SEQ ID NO: 106) |
| | **H10** | SSYGMH (SEQ ID NO: 104) | WVAFIRYDGSNKY (SEQ ID NO: 105) | DGNSGYDSMD (SEQ ID NO: 106) |

The following table indicates the CDR sequences comprised in the variable light chain of preferred antibodies of the invention as defined using the Kabat definition, the IMGT definition, the Chothia definition, or the Contact definition:

| | | **VL_CDR1** | **VL_CDR2** | **VL_CDR3** |
|---|---|---|---|---|
| Kabat | **A7** | SGVSSNIGRNYVY (SEQ ID NO: 76) | ATHQRPS (SEQ ID NO: 78) | AAWDDGVTGWV (SEQ ID NO: 80) |
| | **A02** | SGGSSNVGRNGVN (SEQ ID NO: 77) | YDAFLSS (SEQ ID NO: 79) | ASWDDSLTGWV (SEQ ID NO: 81) |
| | **H10** | SGGSSNVGRNGVN (SEQ ID NO: 77) | YDAFLSS (SEQ ID NO: 79) | AAWDDSLYSWV (SEQ ID NO: 82) |

| | | **VL_CDR1** | **VL_CDR2** | **VL_CDR3** |
|---|---|---|---|---|
| IMGT | **A7** | SSNIGRNY (SEQ ID NO: 107) | ATH (SEQ ID NO: 109) | AAWDDGVTGWV (SEQ ID NO: 80) |
| | **A02** | SSNVGRNG (SEQ ID NO: 108) | YDA (SEQ ID NO: 110) | ASWDDSLTGWV (SEQ ID NO: 81) |
| | **H10** | SSNVGRNG (SEQ ID NO: 108) | YDA (SEQ ID NO: 110) | AAWDDSLYSWV (SEQ ID NO: 82) |

| | | **VL_CDR1** | **VL_CDR2** | **VL_CDR3** |
|---|---|---|---|---|
| Chothia | **A7** | VSSNIGRNY (SEQ ID NO: 111) | ATH (SEQ ID NO: 109) | WDDGVTGW (SEQ ID NO: 113) |
| | **A02** | GSSNVGRNG (SEQ ID NO: 112) | YDA (SEQ ID NO: 110) | WDDSLTGW (SEQ ID NO: 114) |
| | **H10** | GSSNVGRNG (SEQ ID NO: 112) | YDA (SEQ ID NO: 110) | WDDSLYSW (SEQ ID NO: 115) |

| | | **VL_CDR1** | **VL_CDR2** | **VL_CDR3** |
|---|---|---|---|---|
| Contact | **A7** | IGRNYVYW (SEQ ID NO: 116) | LLIYATHQRP (SEQ ID NO: 118) | AAWDDGVTGWV (SEQ ID NO: 80) |
| | **A02** | VGRNGVNW (SEQ ID NO: 117) | LLIYYDAFLS (SEQ ID NO: 119) | ASWDDSLTGWV (SEQ ID NO: 81) |
| | **H10** | VGRNGVNW (SEQ ID NO: 117) | LLIYYDAFLS (SEQ ID NO: 119) | AAWDDSLYSWV (SEQ ID NO: 82) |

### Detailed description of the invention

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Klbl, H. eds. (1995), Helvetica Chimica Acta, CH-4010 Basel, Switzerland).

The practice of the present invention will employ, unless otherwise indicated, conventional methods of biochemistry, cell biology, immunology, and recombinant DNA techniques which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, i.e. the subject-matter consists in the inclusion of a stated member, integer or step or group of members, integers or steps.

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein.

In the following, the elements of the present invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

In the following, some definitions of terms frequently used in this specification are provided. These terms will, in each instance of its use, in the remainder of the specification have the respectively defined meaning and preferred meanings.

As used in this specification and the appended claims, the singular forms "a", "an", and "the" include plural referents, unless the content clearly dictates otherwise.

The term "domain III of human EGFR" as used in the context of the present invention refers to the amino acid sequence as denoted in SEQ ID NO: 61, which corresponds to amino acid positions 311 to 480 of the amino acid sequence of SEQ ID NO: 69, showing the amino acid sequence of the extracellular domains I to IV of human EGFR. SEQ ID NO: 54 shows the amino acid sequence of full length human EGFR including the signal peptide includes domain III at positions 335 to 505. A respective definition of domain III of human EGFR is further provided in Roskoski et al., 2014.

The term "antigen-binding protein" as used herein refers to molecules that contain an antigen-binding site that immune-specifically binds an antigen. Also included are immunoglobulin-like proteins that are selected through techniques including, for example, phage display to specifically bind to a target molecule or target epitope. The binding and/or specificity of an antigen binding protein, e.g., an antibody or immunologically functional fragment thereof, is preferably assessed by determining whether the antigen-binding protein substantially inhibits binding of a ligand to its binding partner when an excess of the antigen-binding protein reduces the quantity of the binding partner bound to its ligand by at least about 1-20%, 20-30%, 30-40%, 40-50%, 50-60%, 60-70%, 70-80%, 80-85%, 85-90%, 90-95%, 95-97%, 97-98%, 98-99% or more (e.g. as measured in an *in vitro* competitive binding assay). The neutralizing ability may be described in terms of an IC₅₀ or EC₅₀ value.

The "IC₅₀" value refers to the half maximal inhibitory concentration of a substance and is thus a measure of the effectiveness of a substance in inhibiting a specific biological or biochemical function. The values are typically expressed as molar concentration. The IC₅₀ of a drug can be determined in functional antagonistic assays by constructing a dose-response curve and examining the inhibitory effect of the examined substance at different concentrations. Alternatively, competition binding assays may be performed in order to determine the IC₅₀ value. Typically, inhibitory antigen binding proteins exhibit an IC₅₀ value of between 50 nM-1 pM, *i.e.* 50 nM, 15 nM, 10 nM, 1 nM, 900 pM, 800 pM, 700 pM, 600 pM, 500 pM, 400 pM, 300 pM, 200 pM, 100 pM, 50 pM, 30 pM, or 1pM.

The "EC₅₀" value refers to half maximal effective concentration of a substance and is thus a measure of the concentration of said substance which induces a response halfway between the baseline and maximum after a specified exposure time. It is commonly used as a measure of drug's potency. The EC₅₀ of a graded dose response curve therefore represents the concentration of a substance where 50% of its maximal effect is observed. The EC₅₀ of a quantal dose response curve represents the concentration of a compound where 50% of the population exhibit a response, after a specified exposure duration. Typically, inhibitory antigen binding proteins exhibit an EC₅₀ value of between 50 nM-1 pM, *i.e.* 50 nM, 15 nM, 10 nM, 1 nM, 900 pM, 800 pM, 700 pM, 600 pM, 500 pM, 400 pM, 300 pM, 200 pM, 100 pM, 50 pM, 390 pM, or 1 pM.

The term "binding" according to the invention preferably relates to a specific binding. "Specific binding" means that a binding protein (e.g. an antibody) binds stronger to a target such as an epitope for which it is specific compared to the binding to another target. A binding protein binds stronger to a first target compared to a second target if it binds to the first target with a dissociation constant (K_{D}) which is lower than the dissociation constant for the second target. Preferably the dissociation constant (K_{D}) for the target to which the binding protein binds specifically is more than 10-fold, preferably more than 20-fold, more preferably more than 50-fold, even more preferably more than 100-fold, 200-fold, 500-fold or 1000-fold, 10⁴-fold, 10⁵-fold, or 10⁶-fold lower than the dissociation constant (K_{D}) for the target to which the binding protein does not bind specifically. Preferably, the term "specific binding" refers to the binding to a predetermined target with a K_{D} of 10⁻⁵ M or lower, 10⁻⁶ M or lower, 10⁻⁷ M or lower, 10⁻⁸ M or lower, 10⁻⁹ M or lower, or 10⁻¹⁰ M or lower.

As used herein, the term "K_{D}" (measured in mol/L, sometimes abbreviated as "M") is intended to refer to the dissociation equilibrium constant of the particular interaction between a binding protein (e.g. an antibody or fragment thereof) and a target molecule (e.g. an antigen or epitope thereof). Methods for determining binding affinities of compounds, i.e. for determining the dissociation constant K_{D}, are known to a person of ordinary skill in the art and can be selected for instance from the following methods known in the art: Surface Plasmon Resonance (SPR) based technology, Bio-layer interferometry (BLI), quartz crystal microbalance (QCM), enzyme-linked immunosorbent assay (ELISA), flow cytometry, isothermal titration calorimetry (ITC), analytical ultracentrifugation, radioimmunoassay (RIA or IRMA) and enhanced chemiluminescence (ECL). In the context of the present application, the K_{D} value is determined by biolayer interferometry (BLI). Preferably, biolayer interferometry is carried out as described in Example 15.

The term "binding site" or "binding domain" as used herein refers to a sequence of amino acids having the ability to specifically bind to an antigen and can be derived, for example, from antibodies or antigen binding fragments thereof. Examples encompassed within the term include but are not limited to Fab fragments, monovalent fragments consisting of the VL, VH domains; Fv fragments consisting of the VL and VH domains of a single arm of an antibody, dAb fragments (Ward et al., (1989) Nature 341: 544-546), which consist of a VH domain or a VL domain, a VHH, a Nanobody, or a variable domain of an IgNAR; isolated complementarity determining regions (CDR), and combinations of two or more isolated CDRs which may optionally be joined by a synthetic peptide linker. The term also refers to the variable domain or region of a TCR α- and a TCR β-chain or of a TCR γ-chain and a TCR δ-chain.

The term "complementary determining region" or "CDR" refers in the context of this invention to the non-contiguous antigen combining sites found within the variable domains of immunoglobulins, e.g. in V_{H}, V_{L}, V_{α} and V_{β}. CDRs have been described by Lefranc et al. (2003) Developmental and Comparative Immunology 27:55; Kabat et al., J. Biol. Chem. 252:6609-66I6 (1977); Kabat et al., U.S. Dept. of Health and Human Services, "Sequences of proteins of immunological interest", 1991; Chothia et al., J. Mol. Biol. I96:90I-917, 1987; and Contact annotation (MacCallum et al., J. Mol. Biol. 262:732-745 (1996) for the Contact annotation); Abhinandan and Martin, Mol. Immunol. (2008), 45(14):3832-9. for AbM annotation; IMGT (Lefranc MP. Unique database numbering system for immunogenetic analysis; Immunol Today (1997) 18:509), where the definitions include overlapping or subsets of amino acid residues when compared against each other. Nevertheless, application of either definition to refer to a CDR of an antibody or grafted antibodies or variants or fragments thereof, is intended to be within the scope of the term as defined and used herein. Accordingly, CDRs can be identified e.g. by the Kabat definition, the Chothia definition, the IMGT definition or the contact definition. Preferably, the positions of the CDRs and framework regions as defined herein are assigned according to Kabat or Chothia, in particular according to Kabat numbering. Thus, according to a particularly preferred embodiment of the present invention, the numbering of the V_{H} and V_{L} described herein is in accordance with Kabat.

It will be appreciated by those skilled in the art that in particular in the sequences of the CDRs, hypervariable and variable regions can be modified without losing the ability to bind to a target. For example, CDR regions may be either identical or highly homologous to the CDRs disclosed herein. By "highly homologous" it is contemplated that from 1 to 3, preferably from 1 to 2, or 1 substitution may be made in the CDRs.

The term "antigen" as used herein relates to an agent comprising an epitope which is recognized by an antigen binding domain. The term "antigen" includes in particular proteins and peptides. An antigen is preferably a product which corresponds to or is derived from a naturally occurring antigen. Such naturally occurring antigens may include or may be derived from allergens, viruses, bacteria, fungi, parasites and other infectious agents and pathogens or an antigen may also be a tumor antigen. An antigen may correspond to a naturally occurring product, for example, a viral protein, or a part thereof, or a tumor protein.

The variable heavy chain domain (V_{H}) and the variable light chain domain (V_{L}) used in the context of the present invention are preferably antibody or immunoglobulin derived. Such "antibody" or "immunoglobulin" may be a natural or conventional antibody having a "Y"-shaped form and consisting of four polypeptide chains; two identical heavy chains and two identical light chains connected by disulfide bonds. Each chain is composed of structural domains. Two heavy chains are linked to each other by disulfide bonds and each heavy chain is linked to a light chain by a disulfide bond. There are two types of light chains, lambda (λ) and kappa (k), and five main heavy chain classes (or isotypes) which determine the functional activity of an antibody molecule: IgM, IgD, IgG, IgA and IgE. Each chain contains distinct sequence domains. The light chain includes two domains or regions, a V_{L} and a constant domain (CL). The heavy chain includes four domains, a V_{H} and three constant domains (CH1, CH2 and CH3, collectively referred to as CH), or in case of IgE and IgM five domains, a V_{H} and four constant domains (CH1, CH2, CH3, CH4). The V_{L} and V_{H} determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The "arms" of the "Y"-shaped antibody contain the sites that can bind to specific molecules, enabling recognition of specific antigens. This region of the antibody is called the Fab (fragment, antigen-binding) region. It is composed of one constant and one variable domain from each heavy and light chain of the antibody. The base of the Y plays a role in modulating immune cell activity. This region is called the Fc (fragment, crystallizable) region, and is composed of two heavy chains that contribute two or three constant domains depending on the class of the antibody. The Fv fragment is the N-terminal part of the Fab region of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or CDRs. Occasionally, residues from non-hypervariable or framework regions (FR) influence the overall domain structure and hence the combining site. As noted above CDRs refer to amino acid sequences that together define the binding affinity and specificity of the natural Fv region of a native immunoglobulin binding site. The light and heavy chains of an immunoglobulin each have three CDRs, designated CDRL1, CDRL2, CDRL3 and CDRH1, CDRH2, CDRH3, respectively. A conventional antibody antigen-binding site, therefore, includes six CDRs, comprising the CDR set from each of a heavy and a light chain variable ("V") region.

Antibodies and antigen-binding fragments thereof usable in the present invention may be from any animal origin including birds and mammals. Preferably, the antibodies or fragments are from human, chimpanzee, rodent (e.g. mouse, rat, guinea pig, or rabbit), chicken, turkey, pig, sheep, goat, camel, cow, horse, donkey, cat, or dog origin. It is particularly preferred that the antibodies are of human or murine origin. Antibodies of the invention also include chimeric molecules in which an antibody constant region derived from one species, preferably human, is combined with the antigen binding site derived from another species, e.g. mouse. Moreover, antibodies of the invention include humanized molecules in which the antigen binding sites of an antibody derived from a non-human species (e.g. from mouse) are combined with constant and framework regions of human origin.

As exemplified herein, antibodies can be obtained directly from hybridomas which express the antibody, or can be cloned and recombinantly expressed in a host cell (e.g., a CHO cell, or a lymphocytic cell). Further examples of host cells are microorganisms, such as *E*. *coli,* and fungi, such as yeast. Alternatively, they can be produced recombinantly in a transgenic non-human animal or plant.

Antibodies and antigen-binding fragments thereof suitable in the context of the present invention include, but are not limited to, polyclonal, monoclonal, monovalent, bispecific, heteroconjugate, multispecific, recombinant, heterologous, heterohybrid, chimeric, humanized (in particular CDR-grafted), deimmunized, or human antibodies, Fab fragments, Fab' fragments, F(ab')2 fragments, fragments produced by a Fab expression library, Fd, Fv, disulfide-linked Fvs (dsFv), single chain antibodies (e.g. scFv), diabodies or tetrabodies (Holliger P. et al. (1993) Proc. Natl. Acad. Sci. U.S.A. 90(14), 6444-6448), nanobodies (also known as single domain antibodies), anti-idiotypic (anti-Id) antibodies (including, e.g., anti-Id antibodies to antibodies of the invention), and epitope-binding fragments of any of the above.

The term "humanized antibody" refers in the context of the present invention to an antibody which is completely or partially of non-human origin and which has been modified to replace certain amino acids, in particular in the framework regions of the heavy and light chains, in order to avoid or minimize an immune response in humans. The constant domains of a humanized antibody are mainly human heavy and light chain domains. Methods for humanization of antibody sequences are known in the art; (Almagro & Fransson (2008) Front Biosci. 13: 1619-1633). One commonly used method is CDR grafting, or antibody reshaping, which involves grafting of the CDR sequences of a donor antibody, generally a mouse antibody, into the framework scaffold of a human antibody of different specificity. Since CDR grafting may reduce the binding specificity and affinity, and thus, the biological activity of a CDR grafted non-human antibody, back mutations may be introduced at selected positions of the CDR grafted antibody in order to retain the binding specificity and affinity of the parent antibody. Amino acid residues that are part of a CDR will typically not be altered, although in certain cases it may be desirable to alter individual CDR amino acid residues, for example to remove a glycosylation site, a deamidation site, an isomerization site, or an undesired cysteine residue. N-linked glycosylation occurs by attachment of an oligosaccharide chain to an asparagine residue in the tripeptide sequence Asn-X-Ser or Asn-X-Thr, where X may be any amino acid except Pro. Removal of an N-glycosylation site may be achieved by mutating either the Asn or the Ser/Thr residue to a different residue, in particular by way of conservative substitution. Deamidation of asparagine and glutamine residues can occur depending on factors such as pH and surface exposure. Asparagine residues are particularly susceptible to deamidation, primarily when present in the sequence Asn-Gly, and to a lesser extent in other dipeptide sequences such as Asn-Ser. When such a deamidation site, in particular Asn-Gly, is present in a CDR sequence, it may, therefore, be desirable to remove the site, typically by conservative substitution to remove one of the implicated residues. Substitution in a CDR sequence to remove one of the implicated residues is also intended to be encompassed by the present invention.

The term "dimerization domain" as used herein refers to a domain capable of forming a dimer of two peptide or protein chains, wherein at least one dimerization domain is present on the first chain and at least a second dimerization domain is present on the second chain. A dimerization domain can be selected from the group consisting of an Fc region, a heterodimerizing Fc region, CH1, CL, the second heavy chain constant domain (CH2) of IgE and IgM (EHD2, MHD2), modified EHD2, the last heavy chain constant domain (CH3 or CH4) of IgG, IgD, IgA, IgM, or IgE and heterodimerizing derivatives thereof, and the constant domains C-alpha and C-beta of a T-cell receptor. Depending on the respective dimerization domain, the C-terminus and N-terminus of the dimerization domain may vary. If the dimerization domain is derived from a naturally occurring protein, e.g. an immunoglobulin, the dimerization domain is preferably directly linked to the variable domain in the sense of the present invention, i.e. it is linked without a peptide linker, if there are no non-naturally occurring amino acids at its C- or N-terminus. The dimerization domains as used in the context of the present invention preferably form one or more covalent bonds between each other, preferably one covalent bond or two covalent bonds, most preferably one covalent bond.

The term "eIg" as referred to herein denotes a heterodimerization domain comprising a modified IgE heavy chain domain 2 (EHD2). Examples of eIg domains are described e.g. in WO 2021/058804, which is herein incorporated by reference. As used herein, the term "EHD2" and "EHD2 domain" refers to the second constant domain of the heavy chain of IgE (Seifert et al., 2012, Protein Eng Des Sel.; 25:603-12; and Seifert et al., 2014, Mol Cancer Ther.;13: 101-11). Likewise, the term "modified EHD2 domain" refers to a part of the EHD2 domain, which sequence has been modified compared the original EHD2 sequence from which it is derived. Modifications include amino acid deletions, substitutions, and insertions. The modification preferably includes one or more amino acid substitutions. Preferred substitutions include C14S and C102S, respectively, with respect to the wildtype human EHD2 core amino acid sequence as denoted in SEQ ID NO: 91. As used herein, the term "hetEHD2" denotes a heterodimer of two modified EHD2 domains, *i.e.* a dimer containing a first and a second EHD2 domain that differ from each other in at least one amino acid position. Exemplary hetEHD2 domains to be used in the context of the present invention comprise or consist of an amino acid sequence as denoted in SEQ ID NOs: 94 and 95. The skilled person will understand that a heterodimerization domain requires a first domain and a second domain to which the first domain binds. In order to prevent homodimerizations, the two domains are different to each other, allowing binding of a first heterodimerization domain to a respective different second heterodimerization domain only. Thus, according to a preferred embodiment of the present invention, the first heterodimerization domain comprises an amino acid sequence as set forth in SEQ ID NO: 94 and the respective second heterodimerization domain comprises an amino acid sequence as set forth in SEQ ID NO: 95. The eIg format described herein is preferably used for bivalent bispecific antigen binding proteins. Constructs having the eIg format are described in more detail in WO 2021/058804. Specific embodiments of the antigen binding proteins of the present invention using hetEHD2 domains are shown in Fig. 33.

The term "Fab-eIg" as referred to herein denotes a Fab directly or indirectly linked to an eIg as described herein. The Fab-eIg format is based on applying the engineered heterodimerization module eIg for dimerization of VH/VL instead of CH1/CL as described in WO 2021/058804. The Fab-eIg format is preferably used for trivalent bispecific antigen binding proteins. To this end, first antigen binding moieties are generated by fusing a first variable heavy chain to CH1 and a first variable light chain to CL. Second antigen binding moieties are generated by fusing a second variable heavy chain to a first hetEHD2 and a second variable light chain to the respective second hetEHD2 as described above. Specific embodiments of the antigen binding proteins of the present invention using the Fab-eIg format are shown in Fig. 33D.

The term "heterodimerizing Fc" relates to variants of a Fc, which are able to form heterodimers. Examples of heterodimerizing Fc include the knob-into-hole technology and other variants of the Fc described in literature for the generation of heterodimeric Fc (Krah et al., 2007; Ha et al., 2016; Mimoto et al., 2016; Brinkmann & Kontermann, 2017). The knob-into-hole or also called knobs-into-holes technology preferably refers to mutations Y349C, T366S, L368A and Y407V (Hole) and S354C and T366W (Knob) both in the CH3-CH3 interface to promote hetero-multimer formation, and has been described in US 5,731,168 and US 8,216,805, notably, both of which are herein incorporated by reference.

As used herein, the term "diabody" refers to a bivalent molecule that can bind to two antigens, either of the same type (monospecific) or to different antigens (bispecific). Diabodies are described e.g. in Holliger et al. (1993) Proc. Natl. Acad. Sci. U.S.A. 90(14), 6444-6448, the contents of which are hereby incorporated by reference.

As used herein, the term "single-chain diabody (scDb)" refers to derivatives of diabodies in which the four variable domains of one or two antibodies are connected by three linkers.

The term "compete(s)" when used in the context of antigen binding proteins (e.g., neutralizing antigen binding proteins or neutralizing antibodies) that compete for the same epitope means competition between antigen binding proteins as determined by an assay in which the antigen binding protein (e.g., antibody or immunologically functional fragment thereof) being tested prevents or inhibits (e.g., reduces) specific binding of a reference antigen binding protein (e.g., a ligand, or a reference antibody) to a common antigen. Numerous types of competitive binding assays can be used to determine if one antigen binding protein competes with another, for example: solid phase direct or indirect radioimmunoassay (RIA), solid phase direct or indirect enzyme immunoassay (EIA), sandwich competition assay (see, e.g., Stahli et al., 1983, Methods in Enzymology .2:242-253); solid phase direct biotin-avidin EIA (see, e.g., Kirkland et al., 1986, J. Immunol. 137:3614-3619) solid phase direct labelled assay, solid phase direct labelled sandwich assay (see, e.g., Harlow and Lane, 1988, Antibodies, A Laboratory Manual, Cold Spring Harbor Press); solid phase direct label RIA using I-125 label (see, e.g., Morel et al., 1988, Molec. Immunol. 25:7-15); solid phase direct biotinavidin EIA (see, e.g., Cheung, et al., 1990, Virology 176:546-552); and direct labelled RIA (Moldenhauer et al., 1990, Scand. J Immunol. 32:77-82). Typically, such an assay involves the use of purified antigen bound to a solid surface or cells bearing either of these, an unlabelled test antigen binding protein and a labelled reference antigen binding protein. Competitive inhibition is measured by determining the amount of label bound to the solid surface or cells in the presence of the test antigen binding protein. Usually, the test antigen binding protein is present in excess. Antigen binding proteins identified by competition assay (competing antigen binding proteins) include antigen binding proteins binding to the same epitope as the reference antigen binding proteins, and antigen binding proteins binding to an adjacent epitope sufficiently proximal to the epitope bound by the reference antigen binding protein for steric hindrance to occur. Usually, when a competing antigen binding protein is present in excess, it will inhibit (e.g., reduce) specific binding of a reference antigen binding protein to a common antigen by at least 40-45%, 45-50%, 50-55%, 55-60%, 60-65%, 65-70%, 70-75% or 75% or more. In some instances, binding is inhibited by at least 80-85%, 85-90%, 90-95%, 95-97%, or 97% or more.

The terms "nucleic acid" and "nucleic acids" as well as "nucleic acid molecule(s)" are used synonymously herein and are understood as single or double-stranded oligo- or polymers of deoxyribonucleotide or ribonucleotide bases or both. Nucleotide monomers are composed of a nucleobase, a five-carbon sugar (such as but not limited to ribose or 2'-deoxyribose), and one to three phosphate groups. Typically, a nucleic acid is formed through phosphodiester bonds between the individual nucleotide monomers, In the context of the present invention, the term nucleic acid includes but is not limited to ribonucleic acid (RNA) and deoxyribonucleic acid (DNA) molecules but also includes synthetic forms of nucleic acids comprising other linkages (e.g., peptide nucleic acids as described in Nielsen et al. (Science 254: 1497-1500, 1991). Typically, nucleic acids are single- or double-stranded molecules and are composed of naturally occurring nucleotides. The depiction of a single strand of a nucleic acid also defines (at least partially) the sequence of the complementary strand. The nucleic acid may be single or double stranded, or may contain portions of both double and single stranded sequences. Exemplified, double-stranded nucleic acid molecules can have 3 ` or 5 ` overhangs and as such are not required or assumed to be completely double-stranded over their entire length. The nucleic acid may be obtained by biological, biochemical or chemical synthesis methods or any of the methods known in the art, including but not limited to methods of amplification, and reverse transcription of RNA. The term nucleic acid comprises chromosomes or chromosomal segments, vectors (e.g., expression vectors), expression cassettes, naked DNA or RNA polymer, primers, probes, cDNA, genomic DNA, recombinant DNA, cRNA, mRNA, tRNA, microRNA (miRNA) or small interfering RNA (siRNA). A nucleic acid can be, e.g., single-stranded, double-stranded, or triple-stranded and is not limited to any particular length. Unless otherwise indicated, a particular nucleic acid sequence comprises or encodes complementary sequences, in addition to any sequence explicitly indicated.

The term "pharmaceutical composition" as used herein refers to a substance and/or a combination of substances being used for the identification, prevention or treatment of a disease or tissue status. The pharmaceutical composition is formulated to be suitable for administration to a patient in order to prevent and/or treat a disease. A pharmaceutical composition refers to the combination of an active agent with a carrier, inert or active, making the composition suitable for therapeutic use. Pharmaceutical compositions can be formulated for oral, parenteral, topical, inhalative, rectal, sublingual, transdermal, subcutaneous or vaginal application routes according to their chemical and physical properties. Pharmaceutical compositions comprise solid, semisolid, liquid, transdermal therapeutic systems (TTS). Solid compositions are selected from the group consisting of tablets, coated tablets, powder, granulate, pellets, capsules, effervescent tablets or transdermal therapeutic systems. Also comprised are liquid compositions, selected from the group consisting of solutions, syrups, infusions, extracts, solutions for intravenous application, solutions for infusion or solutions of the carrier systems of the present invention. Semisolid compositions that can be used in the context of the invention comprise emulsion, suspension, creams, lotions, gels, globules, buccal tablets and suppositories.

The term "active agent" refers to the substance in a pharmaceutical composition or formulation that is biologically active, *i.e.* that provides pharmaceutical value. According to the present invention, a pharmaceutical composition comprises at least the binding protein, the nucleic acid or acids, the vector or the recombinant cell according to the invention as active agent. A pharmaceutical composition may comprise more than one active agent which may act in conjunction with or independently of each other. The active agent can be formulated e.g. as neutral or salt forms. Pharmaceutically acceptable salts include those formed with free amino groups such as those derived from hydrochloric, phosphoric, acetic, oxalic, tartaric acids, etc., and those formed with free carboxyl groups such as but not limited to those derived from sodium, potassium, ammonium, calcium, ferric hydroxides, isopropylamine, triethylamine, 2-ethylamino ethanol, histidine, procaine, and the like.

The term "identity" also referred to as "amino acid sequence identity" refers in the context of the present invention to the percentage of sequence identity and is determined by comparing two optimally aligned sequences over a comparison window, wherein the portion of the sequence in the comparison window can comprise additions or deletions (*i.e.* gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.

The term "identical" refers in the context of two or more polypeptides or nucleic acid sequences, refers to two or more sequences or subsequences that are the same, *i.e.* comprise the same sequence of amino acids or nucleic acids. Sequences are substantially identical to each other if they have a specified percentage of amino acid residues that are the same (e.g., at least 90%, at least 91%, at least 92%, at least 93%, at least 94%, at least 95%, at least 96%, at least 97%, at least 98%, or at least 99% identity over a specified region), when compared and aligned for maximum correspondence over a comparison window, or designated region as measured using one of the following sequence comparison algorithms or by manual alignment and visual inspection. These definitions also refer to the complement of a test sequence. Accordingly, the term "at least 90% sequence identity" is used throughout the specification with regard to polypeptide and polynucleotide sequence comparisons. This expression preferably refers to a sequence identity of at least 90%, at least 91%, at least 92%, at least 93%, at least 94%, at least 95%, at least 96%, at least 97%, at least 98%, or at least 99% to the respective reference polypeptide or to the respective reference polynucleotide.

Comparing two sequences refers in the context of the present invention to a process wherein one sequence acts as a reference sequence, to which a test sequences are compared. When using a sequence comparison algorithm, test and reference sequences are inputted into a computer, if necessary subsequence coordinates are designated, and sequence algorithm program parameters are designated. Default program parameters are commonly used, or alternative parameters can be designated. The sequence comparison algorithm then calculates the percent sequence identities or similarities for the test sequences relative to the reference sequence, based on the program parameters. In case where two sequences are compared and the reference sequence is not specified in comparison to which the sequence identity percentage is to be calculated, the sequence identity is to be calculated with reference to the longer of the two sequences to be compared, if not specifically indicated otherwise. If the reference sequence is indicated, the sequence identity is determined on the basis of the full length of the reference sequence indicated by its SEQ ID number, if not specifically indicated otherwise.

Methods of alignment of sequences for comparison are well known in the art. Optimal alignment of sequences for comparison can be conducted, for example, by the local homology algorithm of Smith and Waterman (Adv. Appl. Math. 2:482, 1970), by the homology alignment algorithm of Needleman and Wunsch (J. Mol. Biol. 48:443, 1970), by the search for similarity method of Pearson and Lipman (Proc. Natl. Acad. Sci. USA 85:2444, 1988), by computerized implementations of these algorithms (e.g., GAP, BESTFIT, FASTA, and TFASTA in the Wisconsin Genetics Software Package, Genetics Computer Group, 575 Science Dr., Madison, Wis.), or by manual alignment and visual inspection (see, e.g., Ausubel et al., Current Protocols in Molecular Biology (1995 supplement)). Algorithms suitable for determining percent sequence identity and sequence similarity are the BLAST and BLAST 2.0 algorithms, which are described in Altschul et al. (Nuc. Acids Res. 25:3389-402, 1977), and Altschul et al. (J. Mol. Biol. 215:403-10, 1990), respectively. Software for performing BLAST analyses is publicly available through the National Center for Biotechnology Information (http://www.ncbi.nlm.nih.gov/).

All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

Several documents are cited throughout the text of this specification. Each of the documents cited herein (including all patents, patent applications, scientific publications, manufacturer's specifications, instructions etc.), whether supra or infra, is hereby incorporated by reference in its entirety. Nothing herein is to be construed as an admission that the invention is not entitled to antedate such disclosure by virtue of prior invention. Some of the documents cited herein are characterized as being "incorporated by reference". In the event of a conflict between the definitions or teachings of such incorporated references and definitions or teachings recited in the present specification, the text of the present specification takes precedence.

In the following, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

In a first aspect, the present invention provides an antigen-binding protein comprising a first antibody variable domain and a second antibody variable domain and wherein the variable domains form a binding site that a) specifically binds to a human epidermal growth factor receptor (EGFR) variant having one or more of the mutations V441D, S464L, G465R, and S492R with a K_{D} < 10⁻⁵ molar determined by biolayer interferometry (BLI); and b) does not specifically bind to human EGFR having the mutation Q435P or double mutation F436A/I462A determined by biolayer interferometry (BLI), wherein binding of the antigen-binding protein to human EGFR blocks ligand-induced activation of human EGFR. An antigen-binding protein of the invention does not specifically bind to human EGFR having the mutation Q435P or double mutation F436A/I462A, if its K_{D} > 10⁻⁵ molar with a K_{D} > 10⁻⁵ molar determined by biolayer interferometry (BLI), preferably > 10⁻⁴ molar, more preferably > 10⁻³ molar, and even more preferably > 10⁻² molar. Preferably, the first antibody variable domain is a V_{L} and the second antibody variable domain is a V_{H}.

According to a preferred embodiment, the antigen-binding protein of the present invention specifically binds to a human epidermal growth factor receptor (EGFR) variant having one or more of the mutations V441D, V441G, S442R, S464L, G465R, K489E and S492R with a KD < 10⁻⁵ molar determined by biolayer interferometry (BLI), and does not specifically bind to human EGFR having the mutation Q435P or double mutation F436A/I462A.

The amino acid sequence of human epidermal growth factor receptor (EGFR) including the signal peptide is as denoted in SEQ ID NO: 54. The positions of the mutations in human EGFR and in particular mutations V441D, S464L, G465R, and S492R, as well as Q435P and F436A/I462A cited herein refer to SEQ ID NO: 54.

Typical ligands of EGFR are EGF, EPG, TGFα, AR, BTC, HB-EGF and EPR (Roskoski, Pharmacological Research 2014: 79:34-74). The blocking of ligand-induced activation of EGFR can be determined by methods known in the art. One exemplary method includes Western Blotting for detecting the phosphorylation of EGFR, such as phosphorylation at position Y1068 and/or Y1173, with one or more phosphor-specific antibodies (Roskoski et al. 2014, *supra*)*.*

The antigen-binding proteins of the present invention preferably bind to a unique epitope in human EGFR comprising amino acid residues G434, Q435, F436 and I462 with respect to SEQ ID NO: 54.

According to the present invention, the variable domains form a binding site that specifically binds to domain III of human EGFR (SEQ ID NO: 61) having one or more of the mutations V441D, S464L, G465R, and S492R. The term "specifically binds to domain III of human EGFR" used in the present context means binding with a K_{D} < 10⁻⁵ molar as determined by biolayer interferometry (BLI), preferably binding with a K_{D} < 10⁻⁶ molar, more preferably binding with a K_{D} < 10⁻⁷ molar. According to a preferred embodiment, the binding site specifically binds to domain III of human EGFR having one or more of the mutations V441D, S464L, G465R, and S492R with a K_{D} of below 1 µM, preferably between 0.1 nM and 999 nM, more preferably with a K_{D} of between 0.5 nM and 850 nM as determined by biolayer interferometry (BLI). The specific positions and in particular mutations V441D, S464L, G465R and S492R cited herein refer to SEQ ID NO: 54. Since the amino acid sequence of domain III of human EGFR as identified herein is comprised within SEQ ID NO: 54, the positions of the mutations cited herein can also be identified in SEQ ID NO: 61 denoting the amino acid sequence of domain III of human EGFR. Accordingly, the mutation V441D as referred to herein corresponds to V107D in SEQ ID NO: 61. Likewise, mutation S464L as referred to herein corresponds to S130L in SEQ ID NO: 61, mutation G465R as referred to herein corresponds to G131R in SEQ ID NO: 61, mutation S492R as referred to herein corresponds to S158R in SEQ ID NO: 61. Therefore, according to one embodiment, the variable domains of the antigen-binding protein of the present invention form a binding site that specifically binds to domain III of human EGFR according to SEQ ID NO: 61 and having one or more of the mutations V107D, S130L, G131R, and S158R.

In accordance therewith, the other mutations Q435P and F436A/I462A in EGFR cited herein with respect to SEQ ID NO: 54 are identical to mutations Q101P, F102A/I128A with respect to SEQ ID NO: 61. Thus, in accordance with an embodiment, the antigen-binding protein of the present invention does not bind specifically to human EGFR having the mutation Q101P or double mutation F102A/I128A with reference to SEQ ID NO: 61. Likewise, amino acids G434, Q435, F436 and I462 as being comprised by the unique epitope to which the antigen-binding proteins of the present invention preferably binds, refer to SEQ ID NO: 54 and correspond to amino acids G100, Q101, F102 and I128 of SEQ ID NO: 61, respectively.

By simple comparison (e.g. aligning) of SEQ ID NO: 54 with SEQ ID NO: 61 or with any other amino acid sequence, all amino acid positions referred to herein can be associated to said other amino acid sequence, such as to the amino acid sequence as denoted in SEQ ID NO: 61.

According to the present invention, the antigen-binding protein is not limited to any specific form or format. The antigen binding protein can consist of only a single polypeptide chain, comprising the first and the second variable domain, e.g. in the form of a scFv, or the antigen binding protein comprises the first and second variable domain on two separate polypeptides. The antigen-binding protein can comprise further identical or different polypeptide chains, e.g. a total of three, four, five or six polypeptide chains. This or these polypeptide chain(s) of the antigen-binding protein of the present invention can comprise further functionalities that are directly or indirectly linked to the first and/or second variable domain, e.g. via a peptide linker. Exemplary functionalities are described in the following in more detail and can comprise:
(i) one or more antigen binding sites with the same or different specificity(ies);
(ii) homo- or heterodimerization domains;
(iii) trimerization domains or tetramerization domains;
(iv) a CH₁ or C_{L} domain.

The antigen-binding protein of the present invention is preferably selected from the group consisting of but not limited to Fab, Fab', (Fab')2, Fv, disulfide-linked Fv, BsFv, dsFv, (dsFv)2, dsFv-dsFv', scFv, scFv dimer, single chain (sc) domain antibody, diabody (Db), scDb, dsDb, and a bivalent domain antibody. Also included are forms that retain the ability to specifically bind to domain III of human EGFR. Examples include Fab fragments, F(ab')2 fragments, Fd fragments, Fv fragments, dAb fragments, etc. The antigen binding protein of the present invention can also be present in form of a chimeric antigen receptor (CAR). The most preferred form of the antigen-binding protein of the present invention is an antibody. According to a further preferred embodiment of the present invention, the antibody is selected from the group consisting of a chimeric antibody, a bispecific antibody, and a multispecific antibody. The antibody is preferably a human or a humanized antibody.

In particular embodiments of the present invention, the antigen binding protein is monospecific, bispecific or multispecific. In particular embodiments, the bispecific or multispecific antigen binding protein specifically binds to a further second cellular target. In preferred embodiments, the second cellular target is selected from the group consisting of a protein expressed on the surface of an immune cell such as CD2, CD3, CD16, CD44, CD64, CD69, CD89, Me114, Ly-6.2C, TCR-complex, Vy9V52 TCR, and NKG2D, preferably CD3. A particularly preferred anti-CD3 antigen binding protein comprises the variable light and heavy chains of anti-human CD3 antibody UCHT1 (huU3) (SEQ ID NOs: 96 and 97). According to a further preferred embodiment, the second cellular target is selected from the group consisting of human epidermal growth factor receptor 2 (HER2), human epidermal growth factor receptor 3 (HER3), human epidermal growth factor receptor 4 (HER4), insulin-like growth factor 1-receptor (IGF-1R), hepatocyte growth factor receptor (HGFR, c-MET), and derivatives thereof, preferably HER2. According to a particularly preferred embodiment, a bispecific or multispecific binding protein according to the invention specifically binds to domain III of human EGFR and to CD3, wherein specifically binds means binding with a K_{D} < 10⁻⁵ molar as determined by biolayer interferometry (BLI).

In particular embodiments of the present invention, the antigen binding protein is tri- or tetravalent. In particular embodiments, the antigen binding protein comprises an effector domain which is in particular bound by Fc receptors, neonatal Fc receptor (FcRn) or the complement system. In particular embodiments, the Fc domain is a domain bound by Fc gamma receptors, in particular by CD16, CD32, and/or CD64. In particular embodiments, the Fc domain is a domain activating the complement system, in particular by binding to C1q of the complement system.

In a preferred embodiment of the present invention, the antigen binding protein is bivalent. Unless indicated otherwise, the configurations of the antigen binding protein embodiments described herein are written from N-terminus on the left to C-terminus on the right. It is further preferred that the antigen binding protein is bivalent and bispecific. In a further preferred embodiment, the bivalent and bispecific antigen binding protein is a diabody. A bispecific diabody comprises two chains, each comprising a VH and VL domain from different antigen binding molecules. The two variable domains VH and VL are preferably connected by a short linker of 3 to 5 residues.

A diabody according to the present invention may be a two-chain diabody (Db) or a single-chain diabody (scDb). For the two-chain diabody, the two chains may have the configuration V_{H}A-V_{L}B and V_{H}B-V_{L}A or V_{L}A-V_{H}B and V_{L}B-V_{H}A, wherein A and B represent the two different specificities. For the single-chain diabody, the first chain, V_{H}A-V_{L}B or V_{L}A-V_{H}B, and the second chain, V_{H}B-V_{L}A or V_{L}B-V_{H}A, are covalently connected to each other. Preferably, the first and second chain are connected via a peptide linker having a length of 10 to 15 amino acids. Preferably, the bispecific diabody is a scDb. Preferably, the antigen binding protein has the configuration (V_{H}A-V_{L}B-V_{H}B-V_{L}A)scDb. In a particularly preferred embodiment, the antigen binding protein comprises or consists of the amino acid sequence of SEQ ID NO: 19.

In a further preferred embodiment, the antigen binding protein is a bispecific Db or bispecific scDb, preferably a bispecific scDb, connected to one or more scFvs, preferably to one or two scFvs. Two or more scFvs may be connected in tandem. A scFv comprises the VH and VL domain of the same antigen binding protein, preferably connected with peptide linker of about 10 to 25 amino acids. An scFv may have the configuration V_{H}-V_{L} or V_{L}-V_{H}. Preferably, the one or more scFvs have one or both of the specificities of the bispecific Db or bispecific scDb. Thus, the scFvs preferably have the configuration V_{H}A-V_{L}A or V_{L}A-V_{H}A or alternatively have the configuration V_{H}B-V_{L}B or V_{L}B-V_{H}B. In a further preferred embodiment, the one or more scFvs may have a specificity different to the specificities of the bispecific Db or bispecific scDb. Accordingly, the one or more scFvs may have the configurations V_{H}C-V_{L}C or V_{L}C-V_{H}C, or V_{H}D-V_{L}D or V_{L}D-V_{H}D, and so on. In a preferred embodiment, the antigen binding protein is a bispecific trivalent antigen binding protein. Preferably the antigen binding protein has the configuration (V_{H}A-V_{L}B-V_{H}B-V_{L}A)scDb-(V_{H}A-V_{L}A)scFv. In a particularly preferred embodiment, the antigen binding protein comprises or consists of the amino acid sequence of SEQ ID NO: 20.

In a further preferred embodiment, the antigen binding protein is comprised of two bispecific Dbs or bispecific scDbs, preferably bispecific scDbs, each connected to an Fc region, wherein the Fc region serves as homodimerization domain. In a preferred embodiment, the antigen binding protein comprises two moieties of the configuration (V_{H}A-V_{L}B-V_{H}B-V_{L}A)scDb-Fc. The two moieties may be covalently or non-covalently bound.

In a further preferred embodiment of a bispecific antigen binding protein, an additional V_{H} domain and V_{L} domain of a second specificity is connected to a light chain and a heavy chain, respectively, wherein the Fc region of the heavy chain serves as dimerization domain. The two V_{H} domains and the two V_{L} domains of different speficities may be connected in various combinations to the light chain and heavy chain, respectively, resulting in different configurations. In a preferred embodiment of the antigen binding protein, the light chain has the configuration V_{H}A-V_{H}B-C_{L}k and the heavy chain has the configuration V_{L}A-V_{L}B-CH1-CH2-CH3. Certain configurations allow a crossover pairing of the V_{H} and V_{L} domains. In a preferred embodiment the light chain has the configuration V_{H}A-V_{L}B-C_{L}k and the heavy chain has the configuration V_{H}B-V_{L}A-CH1-CH2-CH3. In a preferred embodiment, the light chain has the configuration V_{L}A-V_{L}B-C_{L}k and the heavy chain has the configuration V_{H}B-V_{H}A-CH1-CH2-CH3.

In each of above examples, the letters "A", "B", "C" and "D" symbolize an antigen specificity of the antigen binding proteins of the present invention. At least one of "A", "B", "C" and "D" within each antigen binding protein of the invention specifically binds to a conformational epitope formed by domain III of human epidermal growth factor receptor EGFR according to SEQ ID NO: 61. The other specificities may be the same or different. Preferred second and further specificities are outlined below.

Further examples for bispecific antibodies are described in Brinkmann U & Kontermann RE, MABS, 2017, 9(2), 182-212 and in WO 2021/058804, both of which are specifically incorporated herein by reference.

Particularly preferred formats of the antigen-binding protein of the present invention include bivalent and monospecific, bivalent and bispecific, trivalent and bispecific, and tetravalent and bispecific. Particularly preferred formats of the antigen-binding protein of the present invention are shown in Fig. 33.

In a particular embodiment, the antigen binding protein of the present invention comprises multimerization domains. Preferred examples are dimerization domains, trimerization domains or tetramerization domains. If two protein chains are bound to each other, each comprises at least one dimerization domain capable of binding to at least one dimerization domain in the other protein. Accordingly, if the antigen binding protein comprises three protein chains, each comprises at least one treimization domain capable of interacting with the respective other trimerization domains of the other chains. In a particular embodiment, the dimerization domains are selected from the group consisting of heavy chain domain 2 (CH2) of IgM (MHD2) or IgE (EHD2), immunoglobulin Fc region, heavy chain domain 3 (CH3) of IgG or IgA, heavy chain domain 4 (CH4) of IgM or IgE, Fab, Fab2, leucine zipper motifs, barnase-barstar dimers, mini-antibodies, and ZIP mini-antibodies.

In a particular embodiment, the trimerization domain is selected from the group consisting of tenascin C (TNC), the trimerization region of the C-terminal noncollagenous domain (NC1) of collagen XVIII, Fab3 like molecules, and TriBi-minibodies.

In a particular embodiment, the tetramerization domains are selected from the group consisting of the tetramerization domain of p53, the tetramerization domain of the general control protein 4 (GCN4), the tetramerization domain of VASP (vasodilator stimulated phosphoprotein), tandem diabodies, and di-diabodies.

In some embodiments, the use of heterodimerization domains is preferred, in particular if two protein chains with different antigen specificities are present. Particularly preferred heterodimerization domains are eIg and Fab-eIg as described herein.

The present invention particularly provides an antigen binding molecule comprising a first antibody variable domain and a second antibody variable domain. The first antibody variable domain preferably comprises the light chain complementary determining regions CDRL1, CDRL2, CDRL3 of the variable light chain region of SEQ ID NO: 83, 84 or 85. The second antibody variable domain preferably comprises the heavy chain complementary determining regions CDRH1, CDRH2 and CDRH3 of the heavy chain variable region of SEQ ID NO: 74 or 75 and wherein each CDR may comprise one or two amino acid substitutions. Preferably, the CDRL1 comprises one or two, preferably one substitution, and/or the CDRL3 comprises one or two, preferably one substitution. Preferably, the CDRH1 comprises one or two, preferably one substitution and/or the CDRH3 comprises one or two, preferably one substitution. In each case it is preferred that the substitution is a conservative amino acid substitution.

Thus, according to a preferred embodiment, the antigen binding molecule comprises the LCDRs of SEQ ID NO: 83 and the HCDRs of SEQ ID NO: 74 and wherein each CDR may comprise one or two amino acid substitutions. Preferably, the CDRL1 comprises one or two, preferably one substitution, and/or the CDRL3 comprises one or two, preferably one substitution. Preferably, the CDRH1 comprises one or two, preferably one substitution and/or the CDRH3 comprises one or two, preferably one substitution. In each case it is preferred that the substitution is a conservative amino acid substitution.

According to a further preferred embodiment, the antigen binding molecule comprises the LCDRs of SEQ ID NO: 84 and the HCDRs of SEQ ID NO: 74 and wherein each CDR may comprise one or two amino acid substitutions. Preferably, the CDRL1 comprises one or two, preferably one substitution, and/or the CDRL3 comprises one or two, preferably one substitution. Preferably, the CDRH1 comprises one or two, preferably one substitution and/or the CDRH3 comprises one or two, preferably one substitution. In each case it is preferred that the substitution is a conservative amino acid substitution.

According to a further preferred embodiment, the antigen binding molecule comprises the LCDRs of SEQ ID NO: 85 and the HCDRs of SEQ ID NO: 74 and wherein each CDR may comprise one or two amino acid substitutions. Preferably, the CDRL1 comprises one or two, preferably one substitution, and/or the CDRL3 comprises one or two, preferably one substitution. Preferably, the CDRH1 comprises one or two, preferably one substitution and/or the CDRH3 comprises one or two, preferably one substitution. In each case it is preferred that the substitution is a conservative amino acid substitution.

According to a further preferred embodiment, the antigen binding molecule comprises the LCDRs of SEQ ID NO: 83 and the HCDRs of SEQ ID NO: 75 and wherein each CDR may comprise one or two amino acid substitutions. Preferably, the CDRL1 comprises one or two, preferably one substitution, and/or the CDRL3 comprises one or two, preferably one substitution. Preferably, the CDRH1 comprises one or two, preferably one substitution and/or the CDRH3 comprises one or two, preferably one substitution. In each case it is preferred that the substitution is a conservative amino acid substitution.

According to a further preferred embodiment, the antigen binding molecule comprises the LCDRs of SEQ ID NO: 84 and the and wherein each CDR may comprise one or two amino acid substitutions HCDRs of SEQ ID NO: 75 and wherein each CDR may comprise one or two amino acid substitutions. Preferably, the CDRL1 comprises one or two, preferably one substitution, and/or the CDRL3 comprises one or two, preferably one substitution. Preferably, the CDRH1 comprises one or two, preferably one substitution and/or the CDRH3 comprises one or two, preferably one substitution. In each case it is preferred that the substitution is a conservative amino acid substitution.

According to a further preferred embodiment, the antigen binding molecule comprises the LCDRs of SEQ ID NO: 85 and the HCDRs of SEQ ID NO: 75 and wherein each CDR may comprise one or two amino acid substitutions. Preferably, the CDRL1 comprises one or two, preferably one substitution, and/or the CDRL3 comprises one or two, preferably one substitution. Preferably, the CDRH1 comprises one or two, preferably one substitution and/or the CDRH3 comprises one or two, preferably one substitution. In each case it is preferred that the substitution is a conservative amino acid substitution.

The sequences and positions of amino acid residues when referring to CDRs are preferably identified by the Kabat definition, the Chothia definition, the IMGT definition or the contact definition. According to a preferred embodiment, the CDRs are identified by the Kabat definition.

The antigen binding molecule according to the present invention can also be defined by the following complementary determining regions of the variable heavy and light chains.

According to a preferred embodiment, the first antibody variable domain comprises a CDRL1 of SEQ ID NO: 86, CDRL2 of SEQ ID NO: 87, and CDRL3 of SEQ ID NO: 88; and the second antibody variable domain comprises a CDRH1 of SEQ ID NO: 71 CDRH2 of SEQ ID NO: 72, and CDRH3 of SEQ ID NO: 73 and wherein each CDR may comprise one or two amino acid substitutions. Preferably, the CDRL1 comprises one or two, preferably one substitution, and/or the CDRL3 comprises one or two, preferably one substitution. Preferably, the CDRH1 comprises one or two, preferably one substitution and/or the CDRH3 comprises one or two, preferably one substitution. In each case it is preferred that the substitution is a conservative amino acid substitution.

According to another preferred embodiment, the first antibody variable domain comprises a CDRL1 of SEQ ID NO: 76 or 77, CDRL2 of SEQ ID NO: 78 or 79, and CDRL3 of SEQ ID NO: 80, 81 or 82; and the second antibody variable domain comprises a CDRH1 of SEQ ID NO: 71, CDRH2 of SEQ ID NO: 72, and CDRH3 of SEQ ID NO: 73. Preferably, the CDRL1 comprises one or two, preferably one substitution, and/or the CDRL3 comprises one or two, preferably one substitution. Preferably, the CDRH1 comprises one or two, preferably one substitution and/or the CDRH3 comprises one or two, preferably one substitution. In each case it is preferred that the substitution is a conservative amino acid substitution.

Thus, according to a preferred embodiment, the antigen binding molecule comprises an antibody variable domain comprising a CDRH1 of SEQ ID NO: 71, CDRH2 of SEQ ID NO: 72, and CDRH3 of SEQ ID NO: 73, in combination with an antibody variable domain comprising one of the following combinations of CDRLs:
(i) CDRL1 of SEQ ID NO: 76, CDRL2 of SEQ ID NO: 78, and CDRL3 of SEQ ID NO: 80;
(ii) CDRL1 of SEQ ID NO: 76, CDRL2 of SEQ ID NO: 78, and CDRL3 of SEQ ID NO: 81;
(iii) CDRL1 of SEQ ID NO: 76, CDRL2 of SEQ ID NO: 78, and CDRL3 of SEQ ID NO: 82;
(iv) CDRL1 of SEQ ID NO: 76, CDRL2 of SEQ ID NO: 79, and CDRL3 of SEQ ID NO: 80;
(v) CDRL1 of SEQ ID NO: 76, CDRL2 of SEQ ID NO: 79, and CDRL3 of SEQ ID NO: 81;
(vi) CDRL1 of SEQ ID NO: 76, CDRL2 of SEQ ID NO: 79, and CDRL3 of SEQ ID NO: 82;
(vii) CDRL1 of SEQ ID NO: 77, CDRL2 of SEQ ID NO: 78, and CDRL3 of SEQ ID NO: 80;
(viii) CDRL1 of SEQ ID NO: 77, CDRL2 of SEQ ID NO: 78, and CDRL3 of SEQ ID NO: 81;
(ix) CDRL1 of SEQ ID NO: 77, CDRL2 of SEQ ID NO: 78, and CDRL3 of SEQ ID NO: 82;
(x) CDRL1 of SEQ ID NO: 77, CDRL2 of SEQ ID NO: 79, and CDRL3 of SEQ ID NO: 80;
(xi) CDRL1 of SEQ ID NO: 77, CDRL2 of SEQ ID NO: 79, and CDRL3 of SEQ ID NO: 81;
(xii) CDRL1 of SEQ ID NO: 77, CDRL2 of SEQ ID NO: 79, and CDRL3 of SEQ ID NO: 82;
and wherein each CDR may comprise one or two amino acid substitutions. Preferably, the CDRL1 comprises one or two, preferably one substitution, and/or the CDRL3 comprises one or two, preferably one substitution. Preferably, the CDRH1 comprises one or two, preferably one substitution and/or the CDRH3 comprises one or two, preferably one substitution. In each case it is preferred that the substitution is a conservative amino acid substitution.

According to another embodiment, the first antibody variable domain comprises one or more of the framework (FR) sequences of SEQ ID NO: 83, 84 or 85, or a variant of the FR sequences having at least 80%, at least 85%, at least 90%, or at least 95% sequence identity to the FR sequences of SEQ ID NO: 83, 84 or 85, and/or the second antibody variable domain comprises one or more of the FR sequences of SEQ ID NO: 74 or 75, or a variant of the FR sequences having at least 80%, at least 85%, at least 90%, or at least 95% sequence identity to the FR sequences of SEQ ID NO: 74 or 75, wherein the FR are identified by the Kabat definition, the Chothia definition, the IMGT definition or the contact definition. According to a preferred embodiment of the present invention, the FRs are identified by the Kabat definition. The sequence identity of the FR sequences is preferably determined for each FR individually. This means that in the V_{H}, each of FR1, FR2, FR3 and FR4 has at least 80%, at least 85%, at least 90%, or at least 95% sequence identity to the respective FR1, FR2, FR3 and FR4 sequences of SEQ ID NO: 74 or 75. Likewise, in the V_{L}, each of FR1, FR2, FR3 and FR4 has at least 80%, at least 85%, at least 90%, or at least 95% sequence identity to the respective FR1, FR2, FR3 and FR4 sequences of SEQ ID NO: 83, 84 or 85.

According to yet another embodiment the first variable domain comprises one of the amino acid sequences according to SEQ ID NO: 83, 84 or 85 or a variant thereof that has at least 90%, preferably at least 92%, more preferably at least 95% or 98% sequence identity over the entire length and which preferably maintains the three CDRLs as defined above, and the second variable domain comprises one of the amino acid sequences according to SEQ ID NO: 74 or 75 or a variant thereof that has at least 90%, preferably at least 92%, more preferably at least 95% or 98% sequence identity over the entire length and which preferably maintains the three CDRHs as defined above. According to a preferred embodiment, the antigen-binding protein comprises one of the following combinations: SEQ ID NO: 74 and SEQ ID NO: 84, SEQ ID NO: 75 and SEQ ID NO: 83, SEQ ID NO: 75 and SEQ ID NO: 85, SEQ ID NO: 74 and SEQ ID NO: 83, SEQ ID NO: 75 and SEQ ID NO: 84, or SEQ ID NO: 74 and SEQ ID NO: 85. Particularly preferred combinations of variable domains are SEQ ID NO: 74 and SEQ ID NO: 83 (A7), SEQ ID NO: 75 and SEQ ID NO: 84 (A02), or SEQ ID NO: 74 and SEQ ID NO: 85 (H10), or a variant of each of the specified SEQ ID NOs that has at least 90%, preferably at least 92%, more preferably at least 95% or 98% sequence identity over the entire length and which preferably maintains the respective CDRs as defined above. According to a particularly preferred embodiment of the invention, the antigen-binding protein comprises the combination SEQ ID NO: 74 and SEQ ID NO: 83, or the combination SEQ ID NO: 75 and SEQ ID NO: 84, or the combination SEQ ID NO: 74 and SEQ ID NO: 85, or a variant of each of the specified SEQ ID NOs that has at least 90%, preferably at least 92%, more preferably at least 95% or 98% sequence identity over the entire length and which preferably maintains the respective CDRs as defined above.

According to a particularly preferred embodiment, the antigen-binding protein comprises one of the sequence combinations: a) CDRH1, CDRH2 and CDRH3 of SEQ ID NOs: 71, 72 and 73, and CDRL1, CDRL2 and CDRL3 of SEQ ID NOs: 76, 78 and 80 (A7); b) CDRH1, CDRH2 and CDRH3 of SEQ ID NOs: 71, 72 and 73, and CDRL1, CDRL2 and CDRL3 of SEQ ID NOs: 77, 79 and 81 (A02); or c) CDRH1, CDRH2 and CDRH3 of SEQ ID NOs: 71, 72 and 73, and CDRL1, CDRL2 and CDRL3 of SEQ ID NOs: 77, 79 and 82 (H10), wherein each CDR may comprise one or two amino acid substitutions. Preferably, the CDRL1 comprises one or two, preferably one substitution, and/or the CDRL3 comprises one or two, preferably one substitution. Preferably, the CDRH1 comprises one or two, preferably one substitution and/or the CDRH3 comprises one or two, preferably one substitution. In each case it is preferred that the substitution is a conservative amino acid substitution.

The present invention also provides antigen binding proteins such as antibodies that compete for binding with anantigen binding protein of the present invention, preferably a reference antigen binding protein comprising the VL of SEQ ID NO: 83, 84 or 85 and the VH of SEQ ID NO: 74 or 75, which serves as a reference antigen binding protein; or that bind to the same epitope as an antigen binding protein of the present invention preferably comprising the VL of SEQ ID NO: 83, 84 or 85 and the VH of SEQ ID NO: 74 or 75. Thus, according to one embodiment of the present invention, the antigen binding protein either competes for binding with an antigen binding protein comprising the VL of SEQ ID NO: 83 and the VH of SEQ ID NO: 74; or binds to the same epitope as an antigen binding protein comprising the VL of SEQ ID NO: 83 and the VH of SEQ ID NO: 74. According to a further embodiment of the invention, the antigen binding protein either competes for binding to an antigen binding protein comprising the VL of SEQ ID NO: 84 and the VH of SEQ ID NO: 74; or binds to the same epitope as an antigen binding protein comprising the VL of SEQ ID NO: 84 and the VH of SEQ ID NO: 74. According to a further embodiment of the invention, the antigen binding protein either competes for binding to an antigen binding protein comprising the VL of SEQ ID NO: 85 and the VH of SEQ ID NO: 74; or binds to the same epitope as an antigen binding protein comprising the VL of SEQ ID NO: 85 and the VH of SEQ ID NO: 74. According to a further embodiment of the present invention, the antigen binding protein either competes for binding to an antigen binding protein comprising the VL of SEQ ID NO: 83 and the VH of SEQ ID NO: 75; or binds to the same epitope as an antigen binding protein comprising the VL of SEQ ID NO: 83 and the VH of SEQ ID NO: 75. According to a further embodiment of the invention, the antigen binding protein either competes for binding to an antigen binding protein comprising the VL of SEQ ID NO: 84 and the VH of SEQ ID NO: 75; or binds to the same epitope as an antigen binding protein comprising the VL of SEQ ID NO: 84 and the VH of SEQ ID NO: 75. According to a further embodiment of the invention, the antigen binding protein either competes for binding to an antigen binding protein comprising the VL of SEQ ID NO: 85 and the VH of SEQ ID NO: 75; or binds to the same epitope as an antigen binding protein comprising the VL of SEQ ID NO: 85 and the VH of SEQ ID NO: 75. In each of the above embodiments it is preferred that the antigen binding protein of the present invention that serves as a reference is in the scFv format. Suitable competition assays are described in the examples. For example, if the antigen binding protein of the present invention that serves as a reference is present in a competition assay at a concentration of 1 nM it is preferred if the competing antibody has an IC₅₀ of 100 nM or less, more preferably of 50 nM, and even more preferably of 10 nM or less.

According to a particularly preferred embodiment, the bispecific antigen binding proteins of the present invention are in the format scDb, scDb-scFc, eIg or Fab-eIg.

According to a further particularly preferred embodiment, bispecific antigen binding proteins of the present invention comprise the anti-EGFR antibody variable domains of the present invention and in addition anti-CD3 antibody variable domains.

Particularly preferred embodiments of the present invention are bispecific anti-EGFR and anti-CD3 antigen binding proteins comprising an amino acid sequence as set forth in SEQ ID NO: 19 (scDb), SEQ ID NO: 20 (scDb-scFv), the combination of SEQ ID NOs: 9, 21, 23 and 24 (eIg), or the combination of SEQ ID NOs: 9, 21, 22 and 24 (Fab-eIg).

The present invention further provides in a second aspect a nucleic acid or nucleic acids or one or more sets of nucleic acids (referred to as nucleic acid or nucleic acids herein) encoding the antigen-binding protein according to the invention. A nucleic acid may be degraded by endonucleases or exonucleases, in particular by DNases and RNases which can be found in the cell. It may, therefore, be advantageous to modify the nucleic acids of the invention in order to stabilize them against degradation, thereby ensuring that a high concentration of the nucleic acid is maintained in the cell over a long period of time. Typically, such stabilization can be obtained by introducing one or more internucleotide phosphorus groups or by introducing one or more non-phosphorus internucleotides. Accordingly, nucleic acids can be composed of non-naturally occurring nucleotides and/or modifications to naturally occurring nucleotides, and/or changes to the backbone of the molecule. Modified internucleotide phosphate radicals and/or non-phosphorus bridges in a nucleic acid include but are not limited to methyl phosphonate, phosphorothioate, phosphoramidate, phosphorodithioate and/or phosphate esters, whereas non-phosphorus internucleotide analogues include but are not limited to, siloxane bridges, carbonate bridges, carboxymethyl esters, acetamidate bridges and/or thioether bridges. Further examples of nucleotide modifications include but are not limited to: phosphorylation of 5' or 3' nucleotides to allow for ligation or prevention of exonuclease degradation/polymerase extension, respectively; amino, thiol, alkyne, or biotinyl modifications for covalent and near covalent attachments; fluorphores and quenchers; and modified bases such as deoxyInosine (dI), 5-Bromo-deoxyuridine (5-Bromo-dU), deoxyUridine, 2-Aminopurine, 2,6-Diaminopurine, inverted dT, inverted Dideoxy-T, dideoxyCytidine (ddC 5-Methyl deoxyCytidine (5-Methyl dC), locked nucleic acids (LNA's), 5-Nitroindole, Iso-dC and -dG bases, 2`-O-Methyl RNA bases, Hydroxmethyl dC, 5-hydroxybutynl-2'-deoxyuridine, 8-aza-7-deazaguanosineand Fluorine Modified Bases. Thus, the nucleic acid can also be an artificial nucleic acid which includes but is not limited to polyamide or peptide nucleic acid (PNA), morpholino and locked nucleic acid (LNA), as well as glycol nucleic acid (GNA) and threose nucleic acid (TNA).

According to a further aspect, the present invention provides a vector comprising the nucleic acid or set of nucleic acids of the invention. Suitable vectors for expressing nucleic acids in host cells are well known in the art.

The present invention also relates to a method of producing a recombinant host cell expressing a binding molecule according to the invention, said method comprising the steps consisting of (i) introducing in vitro or ex vivo the nucleic acid or set of nucleic acids or the vector as described above into a competent host cell, (ii) culturing in vitro or ex vivo the recombinant host cell obtained and (iii), optionally, selecting the cells which express and/or secrete said binding molecule. Accordingly, the present invention further provides a recombinant cell or host cell comprising the nucleic acid, nucleic acids or vector according to the present invention. The present invention further provides a recombinant cell or host cell expressing the antigen-binding protein, the nucleic acid or nucleic acids, or the vector according to the invention. Suitable cells in this context are *E. coli* cells, insect cells, and mammalian cells. Examples of cells to be used as recombinant or host cells include, without limitation, prokaryotic cells (such as bacteria) and eukaryotic cells (such as yeast cells, mammalian cells, insect cells, plant cells, etc.). Specific examples include *E. coli,* Kluyveromyces or *Saccharomyces* yeasts, mammalian cell lines (e.g., Vero cells, CHO cells, 3T3 cells, COS cells, etc.) as well as primary or established mammalian cell cultures (e.g., produced from lymphoblasts, fibroblasts, embryonic cells, epithelial cells, nervous cells, adipocytes, etc.). Examples also include mouse SP2/0-Ag14 cell (ATCC CRL1581), mouse P3X63-Ag8.653 cell (ATCC CRL1580), CHO cell in which a dihydrofolate reductase gene (hereinafter referred to as "DHFR gene") is defective (Urlaub G et al; 1980), rat YB2/3HL.P2.G11.16Ag.20 cell (ATCC CRL1662, hereinafter referred to as "YB2/0 cell"), HEK293 cells, and the like. Preferred cells are HEK293-6E cells (Durocher et al., 2002, Nucl. Acids Res. 30(2)e9) or CHO cells.

According to a further aspect, the present invention provides a pharmaceutical composition comprising the antigen-binding molecule, the nucleic acid or nucleic acids, the vector, or the recombinant cell or host cell according to the present invention as active agent, and optionally a pharmaceutically acceptable carrier and/or suitable excipient. The pharmaceutical composition may further comprise one or more further active agents. The pharmaceutical composition is preferably selected from the group consisting of solid, liquid, semi-solid or transdermal therapeutic systems.

In a further aspect, the present invention relates to the antigen binding molecule, the nucleic acid or nucleic acids, the vector, the recombinant cell or host cell, or the pharmaceutical composition of the present invention for use in medicine.

The antigen binding molecule, the nucleic acid or set of nucleic acids, the vector, the recombinant cell or host cell, or the pharmaceutical composition of the present invention is particularly suitable for use in treating cancer. According to a preferred embodiment, the cancer is selected from the group consisting of lung cancer, prostate cancer, breast cancer, colon cancer, rectum cancer, head cancer, neck cancer, esophagogastric cancer, liver cancer, glioblastoma, cervix cancer, ovary cancer, bladder cancer, kidney cancer, and pancreas cancer, preferably head and neck squamous cell cancer, non-small cell lung cancer, metastatic colorectal cancer, recurrent or metastatic squamous cell carcinoma of the head and neck.

The cancer is preferably selected from the group consisting of lung (NSCLC), prostate, breast, colon and rectum, head and neck, esophagogastric, liver, glioblastoma, cervix, ovary, bladder, kidney and pancreas cancer (Thomas & Weihua, 2019, Front. Oncol. 9:800).

According to another aspect, the present invention provides a method of treating cancer, the method comprising administering to a patient in need thereof a therapeutically effective amount of the antigen-binding protein, the nucleic acid or nucleic acids, the vector, the recombinant cell or host cell, or the pharmaceutical composition of the invention. According to a preferred embodiment, the cancer is selected from the group consisting of lung cancer, prostate cancer, breast cancer, colon cancer, rectum cancer, head cancer, neck cancer, esophagogastric cancer, liver cancer, glioblastoma, cervix cancer, ovary cancer, bladder cancer, kidney cancer, and pancreas cancer, preferably head and neck squamous cell cancer, non-small cell lung cancer, metastatic colorectal cancer, recurrent or metastatic squamous cell carcinoma of the head and neck.

In accordance with the above, the invention preferably relates to the following items:
Item 1. An antigen-binding protein comprising a first antibody variable domain and a second antibody variable domain and wherein the variable domains form a binding site that:
   a) specifically binds to a human epidermal growth factor (EGFR) variant having one or more of the mutations V441D, S464L, G465R, and S492R with a KD < 10⁻⁵ molar determined by biolayer interferometry (BLI);
   b) does not bind specifically to human EGFR having the mutation Q435P or double mutation F436A/I462A; and
   wherein binding of the antigen-binding protein to human EGFR blocks ligand-induced activation of human EGFR.
Item 2. The antigen-binding protein according to item 1, wherein the variable domains form a binding site that specifically binds to domain III of EGFR (SEQ ID NO: 61).
Item 3. The antigen-binding protein according to item 1 or 2, wherein the antigen-binding molecule is selected from the group consisting of an antibody or an antigen-binding fragment thereof, and a chimeric antigen receptor (CAR);
   wherein the antigen-binding molecule is preferably selected from the group consisting Fab, Fab', (Fab')2, Fv, disulfide-linked Fv, BsFv, dsFv, (dsFv)2, dsFv-dsFv', scFv, scFv dimer, single chain domain antibody, diabody, ds diabody, bivalent domain antibody.
Item 4. The antigen-binding protein according to item 3, wherein the antibody is selected from the group consisting of a chimeric antibody, a humanized antibody, a bispecific antibody, and a multispecific antibody; preferably wherein the antibody is a human antibody.
Item 5. The antigen-binding protein according to any one of items 1 to 4, wherein the first antibody variable domain comprises the light chain complementary determining region (CDRL) 1 (CDRL1), CDRL2, CDRL3 of the variable light chain region of SEQ ID NO: 83, 84 or 85 and/or the second variable domain comprises the CDRH1, CDRH2 and CDRH3 of the heavy chain variable region of SEQ ID NO: 74 or 75, wherein the CDRs are identified by the Kabat definition, the Chothia definition, the IMGT definition or the contact definition.
Item 6. The antigen-binding protein according to any one of items 1 to 4, wherein:
   (a) the first antibody variable domain comprises a
      CDRL1 of SEQ ID NO: 86,
      CDRL2 of SEQ ID NO: 87, and
      CDRL3 of SEQ ID NO: 88;
   (b) the second antibody variable domain comprises a
      CDRH1 of SEQ ID NO: 71
      CDRH2 of SEQ ID NO: 72, and
      CDRH3 of SEQ ID NO: 73;
   wherein each CDR may comprise one or two amino acid substitutions.
Item 7. The antigen-binding protein according to item 6, wherein
   (a) the first antibody variable domain comprises a
      CDRL1 of SEQ ID NO: 76 or 77,
      CDRL2 of SEQ ID NO: 78 or 79, and
      CDRL3 of SEQ ID NO: 80, 81 or 82;
   (b) the second antibody variable domain comprises a
      CDRH1 of SEQ ID NO: 71,
      CDRH2 of SEQ ID NO: 72, and
      CDRH3 of SEQ ID NO: 73;
   wherein each CDR may comprise one or two amino acid substitutions.
Item 8. The antigen-binding protein according to any one of items 1 to 7, wherein the first antibody variable domain comprises one or more of the framework (FR) sequences of SEQ ID NO: 83, 84 or 85, or a variant of the FR sequence having at least 90% sequence identity to the FR sequences of SEQ ID NO: 83, 84 or 85, and/or wherein the second antibody variable domain comprises one or more of the FR sequences of SEQ ID NO: 74 or 75, or a variant of the FR sequence having at least 90% sequence identity to the FR sequences of SEQ ID NO: 74 or 75, wherein the FR are identified by the Kabat definition, the Chothia definition, the IMGT definition or the contact definition, wherein each CDR may comprise one or two amino acid substitutions.
Item 9. The antigen-binding protein according to any one of items 1 to 8, wherein the first variable domain comprises one of the amino acid sequences according to SEQ ID NO: 83, 84 or 85, and wherein the second variable domain comprises one of the amino acid sequences according to SEQ ID NO: 74 or 75; preferably wherein the antigen-binding protein comprises one of the combinations:
   a) SEQ ID NO: 74 and SEQ ID NO: 83,
   b) SEQ ID NO: 75 and SEQ ID NO: 84, or
   c) SEQ ID NO: 74 and SEQ ID NO: 85;
   wherein each CDR may comprise one or two amino acid substitutions.
Item 10. The antigen-binding protein according to any one of items 1 to 9, comprising the following sequences:
   a) CDRH1, CDRH2 and CDRH3 of SEQ ID NOs: 71, 72 and 73, and CDRL1, CDRL2 and CDRL3 of SEQ ID NOs: 76, 78 and 80,
   b) CDRH1, CDRH2 and CDRH3 of SEQ ID NOs: 71, 72 and 73, and CDRL1, CDRL2 and CDRL3 of SEQ ID NOs: 77, 79 and 81, or
   c) CDRH1, CDRH2 and CDRH3 of SEQ ID NOs: 71, 72 and 73, and CDRL1, CDRL2 and CDRL3 of SEQ ID NOs: 77, 79 and 82;
   wherein each CDR may comprise one or two amino acid substitutions.
Item 11. The antigen-binding protein according to any of items 1 to 10, wherein the antigen binding protein:
   (i) competes for binding to an IgG1 antibody comprising the VL of SEQ ID NO: 83, 84 or 85 and the VH of SEQ ID NO: 74 or 75, or
   (ii) binds to the same epitope as an IgG1 antibody comprising the VL of SEQ ID NO: 83, 84 or 85 and the VH of SEQ ID NO: 74 or 75.
Item 12. A nucleic acid or nucleic acids encoding the antigen-binding protein according to any one of items 1 to 11.
Item 13. A vector comprising the nucleic acid or nuclei acids according to item 12.
Item 14. A recombinant cell expressing the antigen-binding protein according to any one of items 1 to 11, the nucleic acid or nucleic acids according to item 12, or the vector according to item 13.
Item 15. A pharmaceutical composition comprising the antigen-binding protein according to any one of items 1 to 11, the nucleic acid or nucleic acids according to item 12, the vector according to item 13, or the recombinant cell according to item 14, and a pharmaceutically acceptable excipient.
Item 16. The antigen-binding protein according to any one of items 1 to 11, the nucleic acid or nucleic acids according to item 12, the vector according to item 13, the recombinant cell according to item 14, or the pharmaceutical composition according to item 15, for use in medicine.
Item 17. The antigen-binding protein according to any one of items 1 to 11, the nucleic acid or nucleic acids according to item 12, the vector according to item 13, the recombinant cell according to item 14, or the pharmaceutical composition according to item 15, for use in the treatment of cancer.
Item 18. The antigen-binding protein, the nucleic acid or nucleic acids, the vector, the recombinant cell, or the pharmaceutical composition for use according to item 17, wherein the cancer is selected from the group consisting of According to a preferred embodiment, the cancer is selected from the group consisting of lung cancer, prostate cancer, breast cancer, colon cancer, rectum cancer, head cancer, neck cancer, esophagogastric cancer, liver cancer, glioblastoma, cervix cancer, ovary cancer, bladder cancer, kidney cancer, and pancreas cancer, preferably head and neck squamous cell cancer, non-small cell lung cancer, metastatic colorectal cancer, recurrent or metastatic squamous cell carcinoma of the head and neck.
Item 19. A method of treating cancer, the method comprising administering to a patient in need thereof a therapeutically effective amount of the antigen-binding protein according to any one of items 1 to 11, the nucleic acid or nucleic acids according to item 12, the vector according to item 13, the recombinant cell according to item 14, or the pharmaceutical composition according to item 15.
Item 20. The method according to item 19, wherein the cancer is selected from the group consisting of lung cancer, prostate cancer, breast cancer, colon cancer, rectum cancer, head cancer, neck cancer, esophagogastric cancer, liver cancer, glioblastoma, cervix cancer, ovary cancer, bladder cancer, kidney cancer, and pancreas cancer, preferably head and neck squamous cell cancer, non-small cell lung cancer, metastatic colorectal cancer, recurrent or metastatic squamous cell carcinoma of the head and neck.

### Examples

### Example 1: EGFR antibodies selection via phage display

For the selection of human single chain fragment variable (scFv) antibodies specific to EGFR, the extracellular domain of human EGFR or EGFRvIII fused to human Fc were used as antigens (huEGFR-/huEGFRvIII-huFc). The selection of monoclonal scFv antibodies (panning) was performed in 96 well microtiter plates (MTP), hence against surface-immobilized EGFR-/EGFRvIII-huFc. Antibodies have been selected from a combination of libraries of Hyperphage packaged human naive antibody genes, named HAL9/10 libraries (Kügler et al., 2015), throughout three consecutive selection rounds.

In brief, 2 µg/well of huEGFR-Fc or huEGFRvIII-huFc were immobilized overnight at 4°C in 150 µL PBS each in two separate wells of a flat bottom MTP (High Binding Costar/Corning, New York, USA). Additionally, four other wells were coated in the same conditions, but with IgG1-huFc protein, for the antibody phage library preclearance. Afterwards, all coated wells were emptied and blocked with 300 µL 2% MPBS-T (2% milk powder in 1x PBS + 0.1% Tween-20) for 1 h at RT and then washed. The human naïve antibody gene library input was mixed 1:1 in panningblock solution (1% BSA, 1% MPBS-T) at a final concentration of 10¹¹ cfu/well in 200 µL. Removal of huFc-specific antibody-phage particles was performed by incubating the library in the preclearance wells 1 h at RT. Afterwards, the precleared antibody phage libraries were incubated in the wells with immobilized huEGFR-huFc or huEGFRvIII-huFc for 2 h at RT.

To further eliminate huFc-specific antibody-phages, 1 µg/well of huIgG1-Fc soluble protein has been added for competition during the library incubation on huEGFR-huFc or huEGFRvIII-huFc immobilized antigens. In a different selection strategy, to select EGFRvIII- or EGFR-specific antibodies, respectively huEGFR-huFc or huEGFRvIII-huFc protein at 1 µg/well were used for competition instead of the solely hIgG1-Fc.

Terminated the incubation, stringent plate bottom washing (1st and 2nd panning round: 20x, 3rd panning round: 30x) was applied to remove unbound antibody-phages. From the remaining antigen-bound antibody-phages, the corresponding phage particles were eluted with 150 µL of 10 µg/ml trypsin in PBS for 30 min at 37°C. The eluted phage were rescued by infection of *E. coli* (150 µL) at OD600 of 0.5 for 30 min at 37°C, followed by incubation for 30 min at 37°C and 650 rpm. After that, new selective media was added (1000 µL 2xYT and 150 µL 10x glucose / ampicillin) to allow the propagation of infected *E.coli* for 1 h at 37°C and 650 rpm. For the production of new antibody-phage particles, M13K07 helperphage was added (1.5×10E12 cfu/mL) to the bacteria. Again, infection was performed for 30 min at 37°C, followed by incubation for 30 min at 37°C and 650 rpm. After that, the cells were centrifuged at 3,220 × g for 10 min and the culture medium replaced to 2xTY (tryptone-yeast) medium containing ampicillin and kanamycin for the induction of antibody-phage production (overnight at 30°C and 650 rpm).

The next day, the bacterial culture was centrifuged (3,220 × g, 10 min) to separate bacteria from antibody-phage particles in the supernatant. The supernatant was removed and used for a second cycle of panning. The amplified antibody-phage particles from each of the four selection strategies (50 µL each) were mixed separately with panningblock to a final volume of 200 µL. This antibody-phage preparation has been used as input to panning round 2 and 3 instead of the human naive antibody library. All other steps have been performed as described above, except that after the 3^{rd} panning round, eluted phage have not been amplified. Instead, *E*. *coli* infected with the eluted phage particles, were directly plated onto 2xYT-GA agar plates at: 10E-4, 10E-5 and 10E-6 dilution and incubated at 37 °C overnight.

### Antibody screening and sequence analysis

In total, 92 single bacterial colonies, corresponding to as many antibody clones, were picked per each of the four panning strategies and used to inoculate 150 µL 2xYT-GA medium in U-shape MTPs. Plates were incubated at 37°C and 800 rpm overnight.

Afterwards, 10 µL of each well were used to inoculate 180 µL 2xYT-GA into a new U-shape MTP. Wells H3 and H9 were not inoculated (negative control); wells H6 and H12 were inoculated with a known antibody culture (positive control). After 2 h incubation at 37°C and 800 rpm, plates were centrifuged for 10 min at 3,220 × g. Bacterial pellet was resuspended in fresh 180 µL production media supplemented with 50 µM IPTG to induce scFv expression and incubated at 800 rpm, at 30°C overnight. The morning after, bacteria were pelleted down again 10 min at 3,220 × g and the supernatant used for antigen binding analysis in ELISA.

For that purpose, 96-well ELISA plates were coated with 1 µg/mL huEGFR-huFc, huEGFRvIII-huFc, or huIgG1-Fc in 100 µL/well. After washing and blocking of the ELISA plates, the soluble scFv in crude bacterial supernatant mixed to MPBS-T were added, incubated for 1 h at RT and binding of the scFv was detected via anti-myc-tag (clone 9E10, produced by University of Braunschweig) and HRP-conjugated secondary antibodies (Sigma A0168). The binding signal was quantified and measured via TMB reaction, followed by absorbance reading at 450 nm and 620 nm as reference wavelength.

For analysis, signal-to-noise (S/N) ratios were calculated between the ELISA binding signals of the positive and negative antigens. Antibody clones showing S/N ratios above 3 and negligible binding to huIgG1-Fc were selected as potential lead candidates. A total of 94 clones fulfilled these selection criteria and were used to confirm the previously obtained result in screening ELISA and then sequence analysis. A total of 22 clones revealed a unique antibody sequence, one has been discarded due to the presence of an amber stop codon in VH gene.

### Antibody production and testing

The remaining 21 scFv antibody genes were cloned in fusion to mouse IgG2a-Fc gene to produce scFv-moFc fusion proteins in mammalian cells and purified by Protein A affinity chromatography for preliminary binding analysis in titration ELISA and on A549 cells. No antibody was found to be selective for EGFRvIII over EGFR in ELISA, while four showed selectivity for the WT protein. Of the 21 tested antibodies, 15 showed specific binding to A549 cells.

### Example 2: Screening for neutralizing anti-EGFR antibodies

Antibodies screened as scFv by phage display were cloned into expression vectors fusing the selected scFv to a human Fc part. Plasmids were transiently transfected into HEK293 cells. Antibodies were purified from supernatant by Protein A affinity chromatography.

Inhibition of EGFR signaling was analyzed by Western blotting using the cancer cell lines FaDu (Figure 1). Therefor, 200,000 cells per 6-well were seeded in DMEM + 10% FCS + P/S and incubated for 24 h before starving cells in medium containing 0.2% FCS for another 24 h. Serum-starved cells were treated with 10 µg/mL anti-EGFR scFv-huFc for 1 h prior to stimulation with 50 ng/mL EGF (biotechne [236-EG-200]) for 15 min. After cell lysis (150 mM NaCl, 1% Nonident P-40, 10 mM NaF, 20 mM β-glycero-phosphate, 1 mM EDTA, 1 mM NasVOs, 0.5 mM PMSF, 0.25% deoxycholic acid sodium salt, 0.1% SDS, 50 mM Tris pH 7.5, cOmplete^{™} protease inhibitors (Roche)), the concentration of the whole cell lysates was determined (DC protein assay - Bio-Rad [5000116]), equal amounts loaded onto 4-12% Nu-PAGE^{™} gels (Thermo Fisher [WG1402BOX] and Western blotting was performed using iBlot 2 Dry Blotting System (Thermo Fisher [IB23001]. Blots were blocked (0.5% (v/v) Roche blocking reagent (Merck) in PBS, 0.05% (v/v) Tween-20) and signals detected with Fusion Solo S (Vilber) using primary antibodies directed against EGFR (Santa Cruz [sc-03]), pEGFR Y1068 (Cell Signaling [CS#3777]), ERK (Cell Signaling [CS#9107]), pERK T202/Y204 (Cell Signaling [CS#9101]) and α-tubulin (Sigma-Aldrich - clone B5-1-2) as well as secondary HRP-conjugated antibodies directed against mouse IgG (Sigma [A2554]) or rabbit IgG (Cell Signaling [CS#7074]).

For cells treated with anti-EGFR scFv-huFc MKU011-A7 and scFv-huFc MKU012-E11, a reduced phosphorylation of EGFR was observed compared to the control without antibody treatment though the inhibition was not as efficient as for treatment with Cetuximab (Figure 1 b). This inhibitory effect was stronger for MKU011-A7 than for MKU012-E11. Treatment of non-stimulated cells with the anti-EGFR scFv-huFc MKU011-A7 and MKU012-E11 did not induce either a phosphorylation of EGFR or an activation of the MAPK pathway analyzed by phosphorylation of ERK (Figure 1 a). All other tested anti-EGFR scFv-huFc did not inhibit EGFR phosphorylation.

Taken together, anti-EGFR scFv-huFc MKU011-A7 and MKU012-E11 were identified as antagonistic antibodies that block ligand-induced phosphorylation of EGFR in FaDu cells.

### Example 3: Affinity maturation of MKU011-A7 by light chain shuffling

### Library generation

The VH gene of 2 EGFR-specific antibodies (MKU011-A7 (variable heavy and light chains according to SEQ ID NOs: 4 and 5), MKU012-E11 (variable heavy and light chains according to SEQ ID NOs: 89 and 90)) was amplified by PCR and purified by gel extraction method.

The purified VH DNA was then cloned individually into antibody-phage display vectors, containing either a human kappa or lambda VL gene library isolated from human B-cells. The insertion of the VH gene into the vector lead to a genetic fusion of the VH gene with a peptide linker and the VL gene, thus, generating a scFv antibody gene.

The cloning products were pooled and desalted using Amicon Ultra centrifugation columns. After that, commercially available electrocompetent E. coli was mixed with the desalted DNA and cells were transformed with phagemid DNA by electroporation. The electroporated bacteria were recovered in SOC media and propagated for 1 h at 37°C and 650 rpm. The theoretical diversity of the scFv library was determined by serial dilution and colony counting.

For the scFv lambda library, a total diversity of 3.0×10⁸ transformants was calculated. For the scFv kappa library, a total diversity of 0.8×10⁸ transformants was obtained.

Remaining bacterial culture was used for antibody-phage production. For that purpose, the bacteria were propagated again in selective culture medium until OD600 of 0.5 was reached. Then, bacteria were infected with M13K07 helperphage. After another growth phase of the infected bacteria in selective media (30 min at 37°C and 30 min at 37°C and 250 rpm), the culture media was exchanged and antibody-phage production was induced by incubation overnight at 25°C and 250 rpm.

The bacteria were separated from antibody-phage particles by centrifugation and purified by PEG precipitation. In brief, 20% (v/v) of a PEG/NaCl solution was added to the cleared culture supernatant and incubated for 1 h on ice. After centrifugation for 1 h at 4 °C and 4,000 × g, the supernatant was discarded and the antibody-phage pellet was resuspended in PBS. The resuspended antibody-phage were again centrifuged at 17,000 × g for 10 min and the soluble fraction was recovered and stored at 4°C.

To determine the antibody-phage concentration, a serial dilution was prepared first and used for the infection of *E. coli* at OD600 of 0.5. After that, the infected cells were cultivated on selective media and the number of transformants determined by colony counting.

For both scFv libraries, an antibody-phage concentration of approximately 4×10¹¹ cfu/ml was calculated (~3×10¹¹ cfu in total).

The display of the scFv on the antibody-phage surface was confirmed by SDS-PAGE of 10E9 antibody-phage particles, followed by western blotting and immunoblot staining with an anti-pIlI antibody.

### Antibody-phage selection

To select VL-shuffled antibodies with improved binding characteristics, the antibody-phage libraries were used for biopanning with antigen limitation and competition. In brief, antibody-phage particles (~4×10E10 cfu) were diluted in 2% BSA-PBST (~1 mL) and incubated with magnetic Dynabeads^{™} M-280 Streptavidin (25 µL, Thermo Scientific, #11206D) and mouse IgG (10 µg) for 1 h at RT under rotation. The beads were separated from the supernatant by magnetic force, thereby separating cross-reactive antibody-phage from the solution. After clearance of the library, the biotinylated target antigen was added. As a target antigen, the extracellular domain (ECD) of the EGFR protein was used, which was fused to a mouse IgG2a Fc. In the first cycle of biopanning, a final concentration of 20 nM antigen was used and incubated with the cleared library for 1 h at RT under rotation. After that, magnetic Dynabeads^{™} M-280 Streptavidin (25 µl) were added and incubated for 30 min at RT under rotation. Antibody-phage that bound to the biotinylated target antigen were separated from the non-bound antibody-phage in solution via the Dynabeads by magnetic force. The supernatant was removed and beads were washed 3x with 1 ml 2% BSA-PBST. After that, the beads were incubated for 15 min in 1 mL 2% BSA-PBST, before washing was repeated 3x with 1 ml PBST. Antibody-phage that were still bound to the antigen-bead complex, were eluted by incubation with Trypsin (10 µg/ml, 150 µL) for 30 min at 37°C.

The eluted antibody-phage were rescued by infection of *E. coli* (1 mL) at OD600 of 0.5 for 30 min at 37°C, followed by incubation for 30 min at 37°C and 500 rpm. After that, new selective media was added (~4.5 ml) to allow the propagation of infected *E. coli* for 1 h at 37 °C and 500 rpm. For the production of new antibody-phage particles, M13K07 helper phage was added (MOI: -1:20) to the bacteria. Again, infection was performed for 30 min at 37°C, followed by incubation for 30 min at 37°C and 500 rpm.

After that, the cells were centrifuged at 3,220 × g for 10 min and the culture medium replaced to 2xTY medium containing ampicillin and kanamycin for the induction of antibody-phage production (overnight at 30°C and 500 rpm). The next day, the bacterial culture was centrifuged (3,220 × g, 10 min) to separate bacteria from antibody-phage particles in the supernatant. The supernatant was removed and used (100 µL each) for a second cycle of biopanning under more stringent conditions.

In cycle two, two different selection strategies were performed with the amplified antibody-phage from cycle one. In one strategy, the amount of biotinylated target antigen was reduced to 2 nM. In the other strategy, the amount of biotinylated target antigen was reduced to 0.2 nM. After incubation of the antibody-phage and the biotinylated target antigen for 1 h at RT under rotation, the non-biotinylated target antigen was added as a soluble competitor (75 nM) to the selection mix. The incubation was continued for another 30 min before washing, elution and rescuing was performed as described above.

### Antibody screening and sequence analysis

Bacteria, that were infected with eluted antibody-phage from the second selection round, were transferred on selective media and incubated at 37 °C until single colonies were observed. Monoclonal colonies were picked and transferred into 96-well plates with selective culture medium (180 µL/well) and incubated overnight at 30 °C and 300 rpm. The overnight cultures (15 µL) were transferred into new 96-well plates, pre-filled with production medium (180 µL/well) and soluble scFv production was induced overnight at 30°C and 300 rpm.

The binding characteristics of the scFv containing bacterial cultures was measured by ELISA. For that purpose, 384-well ELISA plates were coated with Streptavidin (40 ng/well) and the biotinylated target antigen (20 ng/well), Streptavidin only (40 ng/well), mouse IgG (20 ng/well) or a 2% BSA solution. After washing and blocking of the ELISA plates, the scFv were added, incubated for 1 h at RT and binding of the scFv was detected via secondary antibodies. The binding signal was quantified and measured via TMB reaction, followed by absorbance reading at 450 nm and 620 nm as reference wavelength.

For analysis, signal-to-noise (S/N) ratios were calculated between the ELISA binding signals of the positive and negative antigens. Antibody clones that were selected from the most stringent selection strategy (0.2 nM target antigen for positive selection) and very high ELISA S/N ratios (>100) were selected as potential lead candidates. A total of 88 clones fulfilled these selection criteria and were used for DNA-sequence analysis. 13 clones revealed a unique antibody sequence.

### Antibody production and testing

At first, 12 of the identified antibodies were produced as scFv-human Fc fusion proteins in mammalian cells and purified by Protein A affinity chromatography. After initial testing for EGFR binding by ELISA, two lead antibodies derived from parental antibody MKU011-A7 were identified (YU250-A02, YU250-H10) which were subsequently produced as human IgG1 antibodies.

### Example 4: Production of anti-EGFR IgG and Fab-His for MKU011-A7, YU250-A02 and YU250-H10

Fully human monospecific IgG1 (SEQ ID NOs: 4 and 10) and Fab-His (SEQ ID NOs: 4 and 5) comprising the anti-EGFR antibody MKU011-A7 variable domain sequences were cloned and expressed in suspension culture adapted HEK293-6E cells. To this end, HEK293-6E with a density of 1×10⁶/mL in FreeStyle^{™} F17 expression medium (Thermo Fisher, A1383501) supplemented with 4 mM GlutaMAX^{™} (Thermo Fisher, 35050061) and 0.1% Kolliphor^{®} P188 (Sigma, 15759-1KG) were transiently transfected using polyethylamin (PEI, 25 kDa, linear - Polysciences). For 100 mL of cells, 100 µg DNA and 200 µg PEI were mixed in 10 mL medium and incubated for 15 min before adding to the cells. The cells were incubated at 120 rpm / 5% CO₂ at 37°C in a humified incubator for 24 h before adding 5 mL 20% (w/v) trypton N1 (Organo Technie) solved in medium to the 100 mL culture to initiate the protein production. After additional 4 days of incubation at 120 rpm / 5% CO₂ at 37°C in a humified incubator, the supernatant containing the protein was harvested by centrifugation. The protein was purified by Protein A for IgG or CaptureSelect^{™} C_{H}1 XL (Thermo Fisher) affinity chromatography for Fab-His using 100 mM glycine (pH 3.5) as elution buffer. Size exclusion chromatography using a TSKgel SuperSW mAb HR column (Tosoh) confirmed the integrity of the IgG after Protein A purification (Figure 2 a). SDS-PAGE analysis of the proteins showed single bands under non-reducing conditions for Fab-His (50 kDa) and IgG (150 kDa). Under reducing conditions, the presence of the expected chains was confirmed for Fab-His (two chains at 25 kDa) and IgG (heavy chain at 50 kDa and light chain at 25 kDa) (Figure 2 b).

Fully human IgG1 molecules (IgG YU250-A02 and YU250-H10) comprising the YU250-A02 variable domain sequences (SEQ ID NOs: 6 and 7) or the YU250-H10 variable domain sequences (SEQ ID NOs: 9 and 10) were cloned and expressed in suspension culture adapted HEK293-6E cells as described above. The protein was purified from the supernatant of transiently transfected cells by Protein A affinity chromatography using 100 mM glycine (pH 3.5) as elution buffer. Size exclusion chromatography after Protein A purification using a TSKgel SuperSW mAb HR column (Tosoh) confirmed a purity of IgG YU250-A02 and YU250-H10 of 94.4% and 93.1%, respectively (Figure 3 a + d). No aggregates were detectable after a preparative SEC-FPLC (Figure 3 b + e). SDS-PAGE analysis of FPLC-purified IgG YU250-A02 and YU250-H10 showed single bands under non-reducing conditions (approximately 150 kDa) and two bands under reducing conditions corresponding to the heavy chain (50 kDa) and the light chain (25 kDa) (Figure 3 c + f).

Fully human Fab-His comprising the anti-EGFR antibody YU250-A02 variable domain sequences (SEQ ID NOs: 6 and 8) and YU250-H10 variable domain sequences (SEQ ID NOs: 5 and 9) were cloned and expressed in suspension culture adapted HEK293-6E cells as described above (fully human Fab-His comprising the anti-EGFR antibody MKU011-A7 with SEQ ID NOs: 4 and 5). The protein was purified from the supernatant by CaptureSelect^{™} C_{H}1 XL (Thermo Fisher) affinity chromatography using 100 mM glycine (pH 3.5) as elution buffer. Size exclusion chromatography using a TSKgel SuperSW mAb HR column (Tosoh) confirmed the integrity of the Fab-His after CaptureSelect^{™} C_{H}1 XL purification (Figure 4 a + c). SDS-PAGE analysis of the proteins showed single bands under non-reducing conditions at 50 kDa and two bands under reducing conditions at 25 kDa (Figure 4 b + d).

Taken together, the anti-EGFR antibodies MKU011-A7, YU250-A02 and YU250-H10 can be produced both as an IgG and as a Fab-His with a purity of >93.1% after a one-step purification using either Protein A or CaptureSelect^{™} C_{H}1 XL (Thermo Fisher) affinity chromatography, respectively. Increased yields were obtained for the V_{L}-shuffled variants YU250-A02 and YU250-H10 compared to their parental antibody MKU011-A7 (Table 1).

**Table 1: Yields of anti-EGFR IgG or Fab-His after Protein A and CaptureSelect^{™} C_{H}1 XL purification as well as after preparative SEC-FPLC if necessary.**

| **IgG** | **After Protein A** | **After preparative SEC-FPLC** |
|---|---|---|
| MKU011-A7 | 2.7 mg/L | - |
| YU250-A02 | 46 - 63 mg/L | 38 mg/L |
| YU250-H10 | 22 - 40 mg/L | 9 - 22 mg/L |

| **Fab-His** | **After CaptureSelect^{™} C_{H}1 XL** | **After preparative SEC-FPLC** |
|---|---|---|
| MKU011-A7 | 4.1 mg/L | - |
| YU250-A02 | 2.2 mg/L | - |
| YU250-H10 | 1.7 mg/L | - |

### Example 5: Deletion of glycosylation site in V_{L} of YU250-H10 increases productivity of IgG YU250-H10

A potential glycosylation site in V_{L} of IgG YU250-H10 was deleted by site-directed mutagenesis generating the variant V_{L} N126K. The parental antibody (wt) (SEQ ID NOs: 9 and 58) and the variant (V_{L} N126K) (SEQ ID NOs: 57 and 58) were expressed in suspension culture adapted HEK293-6E cells as described above. The protein was purified from the supernatant of transiently transfected cells by Protein A affinity chromatography using 100 mM glycine (pH 3.5) as elution buffer. Size exclusion chromatography after Protein A purification using a TSKgel SuperSW mAb HR column (Tosoh) confirmed the integrity of both proteins (Figure 5 a, b). Deletion of the potential glycosylation site resulted in an increased protein yield by -50% (16.8 mg/L for wt / 26.3 mg/L for V_{L} N126K). By SDS-PAGE, the integrity of both proteins was analyzed (Figure 5 c) showing a band under non-reducing conditions corresponding to approximately 150 kDa. For reducing conditions, both heavy (50 kDa) and light (25 kDa) chain were observed. Due to the deletion of the glycosylation site, a smaller apparent molecular weight was observed for the V_{L} N1216K variant. Binding of anti-EGFR IgG was analyzed by ELISA (Figure 5 d) using immobilized EGFR-His fusion protein comprising the extracellular domain (aa 25 - 645) of human EGFR (SEQ ID NO: 53). The EGFR-His fusion protein was coated onto polystyrene microtiter plates at 3 µg/mL in PBS. Remaining sites were blocked with PBS, 2% skimmed milk (MPBS). Plates were incubated with a serial dilution of antibodies in MPBS. Bound antibodies were detected with an HRP-conjugated anti-huFc antibody (1:5,000) diluted in MPBS. As a substrate for detection, 1% TMB and 0.06% H₂O₂ diluted in 100 mM sodium phosphate buffer (pH 6.0) was used. No difference for binding to EGFR was observed when deleting the glycosylation site in V_{L} of IgG YU250-H10 (EC50 0.49 ± 0.09 nM for wt / 0.53 ± 0.05 nM for V_{L} N126K).

### Example 6: Anti-EGFR antibody YU250-H10 exhibits an increased binding to EGFR compared to MKU011-A7

Monovalent binding of Fab-His (Figure 6 a) as well as bivalent binding of IgG1 (Figure 6 b) for anti-EGFR antibodies MKU011-A7 and YU250-H10 (sequences as defined in Example 4) was analyzed by ELISA using immobilized EGFR-moIgG2a-Fc fusion protein (SEQ ID NO: 33) comprising the extracellular domain (aa 25 - 645) of human EGFR. The EGFR-moIgG2a-Fc fusion protein was coated onto polystyrene microtiter plates at 3 µg/mL in PBS. Remaining sites were blocked with PBS, 2% skimmed milk (MPBS). Plates were incubated with a serial dilution row of antibodies in MPBS. Bound antibodies were detected with an HRP-conjugated anti-huFab antibody (1:20,000) diluted in MPBS. As a substrate for detection, 1% TMB and 0.06% H₂O₂ diluted in 100 mM sodium phosphate buffer (pH 6.0) was used (n=3).

Compared to anti-EGFR antibody MKU011-A7, YU250-H10 exhibited a 4.7-fold increased binding for the monovalent Fab-His (EC₅₀: 2.79 ± 1.19 nM vs. 0.59 ± 0.19 nM). For bivalent binding, no difference was observed (EC₅₀: 0.32 ± 0.08 nM vs. 0.27 ± 0.10 nM) (Table 2).

**Table 2: EC₅₀ values [nM] for monovalent and bivalent binding of MKU011-A7 and YU250-H10 to EGFR analyzed by ELISA (n=3).**

| | **MKU011-A7** | **YU250-H10** |
|---|---|---|
| **Fab-His** | 2.79 ± 1.19 | 0.59 ± 0.19 |
| **IgG** | 0.32 ± 0.08 | 0.27 ± 0.10 |

### Example 7: IgG YU250-A02 and YU250-H10 bind as efficiently to EGFR in ELISA and to cells analyzed by flow cytometry as Cetuximab

Binding of anti-EGFR IgG was analyzed by ELISA (Figure 7 a) using immobilized EGFR-His fusion protein comprising the extracellular domain (aa 25 - 645) of human EGFR (SEQ ID NO: 53). The EGFR-His fusion protein was coated onto polystyrene microtiter plates at 3 µg/mL in PBS. Remaining sites were blocked with PBS, 2% skimmed milk (MPBS). Plates were incubated with a serial dilution of antibodies in MPBS. Bound antibodies were detected with an HRP-conjugated anti-huFc antibody (1:5,000) diluted in MPBS. As a substrate for detection, 1% TMB and 0.06% H₂O₂ diluted in 100 mM sodium phosphate buffer (pH 6.0) was used.

Binding of anti-EGFR IgG to cells was analyzed by flow cytometry for DiFi (Figure 7 b) and FaDu (Figure 7 c). Therefor, 100,000 cells were harvested and incubated with a serial dilution of antibodies diluted in PBS + 2% (v/v) FCS + 0.02% NaN₃ (PBA) for 1 h in a U-bottom 96-well plate. After washing of each well three times (harvesting of cells by centrifugation for 3 min at 1,500 rpm / 4°C, discarding of supernatant and re-suspending cells in 175 µL PBA), cells were incubated with a PE-conjugated anti-huFc antibody (1:500 - dianova [109-115-098]) for 1 h. After washing of each well three times, the fluorescence was measured with MACSQuant^{®} VYB (Miltenyi Biotec). Anti-EGFR IgG MKU011-A7, YU250-A02 and YU250-H10 (sequences as defined in Example 4) bound to EGFR-His in ELISA and to human cancer cell lines DiFi and FaDu analyzed by flow cytometry with similar EC₅₀ values as Cetuximab (Table 3).

**Table 3: EC₅₀ values [nM] for binding of anti-EGFR IgG to EGFR-His in ELISA and to cancer cell lines DiFi and FaDu by flow cytometry (n=1).**

| | **EGFR-His** | **DiFi** | **FaDu** |
|---|---|---|---|
| **Cetuximab** | 0.14 ± 0.02 | 0.48 ± 0.01 | 0.19 ± 0.03 |
| **MKU011-A7** | 0.19 ± 0.02 | 0.81 ± 0.04 | 0.35 ± 0.04 |
| **YU250-A02** | 0.16 ± 0.01 | 0.64 ± 0.03 | 0.23 ± 0.04 |
| **YU250-H10** | 0.19 ± 0.02 | 0.78 ± 0.04 | 0.29 ± 0.04 |

### Example 8: Anti-EGFR antibodies YU250-A02 and YU250-H10 are cross-reactive to cynomolgus monkey EGFR

Binding of anti-EGFR IgG YU250-A02 and YU250-H10 (sequences as defined in Example 4) to cynomolgus monkey EGFR was analyzed at 10 nM in comparison to Cetuximab (Figure 8 a, b) as well as for a concentration-dependent binding for anti-EGFR IgG YU250-A02 and YU250-H10 (Figure 8 c, d). Binding was analyzed by ELISA using immobilized EGFR-His fusion protein comprising the extracellular domain of cynomolgus monkey EGFR (aa 1 - 645) (Sino Biological [SIN-90285-C08H-50]) (SEQ ID NO: 52). The fusion protein was coated onto polystyrene microtiter plates at 3 µg/mL in PBS. Remaining sites were blocked with PBS, 2% skimmed milk (MPBS). Plates were incubated with 10 nM anti-EGFR IgG (Figure 8 a, b) or a serial dilution of anti-EGFR IgG (Figure 8 c, d) in MPBS. Bound IgG was detected with an HRP-conjugated anti-huFc antibody (1:5,000) diluted in MPBS. As a substrate for detection, 1% TMB and 0.06% H₂O₂ diluted in 100 mM sodium phosphate buffer (pH 6.0) was used. Anti-EGFR IgG YU250-A02 and YU250-H10 were cross-reactive to cynomolgus monkey EGFR showing similar EC₅₀ values for both human and cynomolgus monkey EGFR (Table 4).

**Table 4: EC₅₀ values [nM] for binding of anti-EGFR IgG to human and cynomolgus monkey EGFR by ELISA (n=3).**

| | **human EGFR** | **cynomolgus monkey EGFR** |
|---|---|---|
| **YU250-A02** | 0.59 ± 0.05 | 0.69 ± 0.08 |
| **YU250-H10** | 0.78 ± 0.08 | 0.58 ± 0.09 |

### Example 9: Affinity-matured IgG YU250-A02 and YU250-H10 are more potent in inhibiting EGFR signaling and cell proliferation than IgG MKU011-A7

Inhibition of EGFR signaling was analyzed by Western blotting using the cancer cell lines DiFi and FaDu (Figure 9 a, b). Therefor, 200,000 cells per 6-well were seeded in RPMI + 10% FCS + P/S (DiFi) or DMEM + 10% FCS + P/S (FaDu) and incubated for 24 h before starving cells in medium containing 0.2% FCS for another 24 h. Serum-starved cells were treated with 10 nM anti-EGFR IgG for 1 h prior to stimulation with 50 ng/mL EGF (biotechne [236-EG-200]) for 15 min. After cell lysis (150 mM NaCl, 1% Nonident P-40, 10 mM NaF, 20 mM β-glycero-phosphate, 1 mM EDTA, 1 mM NasVOs, 0.5 mM PMSF, 0.25% deoxycholic acid sodium salt, 0.1% SDS, 50 mM Tris pH 7.5, cOmplete^{™} protease inhibitors (Roche)), the concentration of the whole cell lysates was determined (DC protein assay - Bio-Rad [5000116]), equal amounts loaded onto 4-12% Nu-PAGE^{™} gels (Thermo Fisher [WG1402BOX] and Western blotting was performed using iBlot 2 Dry Blotting System (Thermo Fisher [IB23001]. Blots were blocked (0.5% (v/v) Roche blocking reagent (Merck) in PBS, 0.05% (v/v) Tween-20) and signals detected with Fusion Solo S (Vilber) using primary antibodies directed against EGFR (Santa Cruz [sc-03]), pEGFR Y1068 (Cell Signaling [CS#3777]), ERK (Cell Signaling [CS#9107]), pERK T202/Y204 (Cell Signaling [CS#9101]) and α-tubulin (Sigma - clone B5-1-2) as well as secondary HRP-conjugated antibodies directed against mouse IgG (Sigma [A2554]) or rabbit IgG (Cell Signaling [CS#7074). For both cell lines treated with anti-EGFR IgG antibodies MKU011-A7, YU250-A02 and YU250-H10 inhibited phosphorylation of EGFR at position Y1068 compared to untreated cells. IgG YU250-A02 and YU250-H10 inhibited phosphorylation of EGFR at position Y1068 more efficiently than MKU011-A7 for both EGF-stimulated DiFi and FaDu. This inhibition was comparable to the inhibition that was observed for treatment with Cetuximab.

Inhibition of proliferation was analyzed for cancer cell lines DiFi and FaDu (Figure 9 c, d). Therefor, 2,000 cells per 96-well were seeded in RPMI + 10% FCS + P/S (DiFi) or DMEM + 10% FCS + P/S (FaDu) and incubated for 24 h before starving cells in medium containing 0.2% FCS for another 24 h. Serum-starved cells were treated with 10 nM anti-EGFR IgG for 5 d until quantification of cell number using CellTiter-Glo^{®} 2.0 Cell Viability Assay (Promega [G9242]). Anti-EGFR IgG MKU011-A7, YU250-A02 and YU250-H10 (sequences as defined in Example 4) lead to a statistically significant inhibition of proliferation for serum-starved DiFi compared to untreated cells. Anti-EGFR IgG MKU011-A7, YU250-A02 and YU250-H10 inhibited proliferation of serum-starved DiFi as efficiently as Cetuximab. Anti-EGFR IgG MKU011-A7, YU250-A02 and YU250-H10 lead to a statistically significant inhibition of proliferation for serum-starved FaDu compared to untreated cells. Anti-EGFR IgG YU250-A02 and YU250-H10 led to a statistically significant stronger inhibition of proliferation of serum-starved FaDu compared to cells treated with MKU011-A7.

Taken together, IgG YU250-A02 and YU250-H10 showed inhibitory effects as good as Cetuximab for inhibition of EGFR phosphorylation and proliferation of FaDu and DiFi. These inhibitory effects were superior to IgG MKU011-A7 and in a comparable range as Cetuximab.

### Example 10: Anti-EGFR IgG YU250-A02 and YU250-H10 inhibit EGFR dimerization as efficiently as Cetuximab in a β-galactosidase complementation assay

Stable murine fibroblast cell lines were generated using a retroviral MSCV-based vector system to study EGFR dimerization by β-galactosidase complementation. To this end, i) the extracellular and transmembrane domains of human EGFR (aa 1 - 679) were fused by a linker (AAAGSGGGGS) to the α-peptide of β-galactosidase (SEQ ID NO: 59) and cloned into a pMSCV-LTR-Puromycin vector, and ii) the extracellular and transmembrane domains of human EGFR (aa 1 - 679) were fused by a linker (AAAGSGGGGS) to α-acceptor of β-galactosidase (Δ1-31) and cloned into a pMSCV-LTR-HygomycinB vector (SEQ ID NO: 60). Transduced NIH-3T3 cells were selected using 1 µg/mL Puromycin (Sigma [P8833-25MG]) and 200 µg/mL Hygromycin B (Thermo Fisher [10687010]) in DMEM + 10% FCS + P/S. To monitor dimerization of EGFR, 20,000 stable cells were seeded per 96-well in DMEM + 10% FCS + P/S and incubated for 24 h before starving cells in medium containing 0.2% FCS for another 24 h. Serum-starved cells were treated with serial dilution (Figure 10 a) or 10 nM (Figure 10 b) IgG for 15 min prior to stimulation with 30 ng/mL EGF (biotechne [236-EG-200]) for 1 h. After washing cells once with PBS, luminescence was measured (TECAN Spark^{®}) according to instructions of Galacto-Light PlusTM β-Galactosidase Reporter Gene Assay System (Thermo Fisher [T1011]). Anti-EGFR IgG YU250-A02 and YU250-H10 (sequences as defined in Example 4) led to a statistically significant inhibition of EGF-mediated EGFR dimerization in the β-galactosidase complementation cell line, similar to Cetuximab, while Trastuzumab included as negative control had no effects.

### Example 11: Anti-EGFR IgG YU250-H10 is efficiently internalized into FaDu cells

Anti-EGFR IgG YU250-H10 (sequences as defined in Example 4) and Cetuximab were labeled with pHrodo^{™} Red (succinimidyl ester) according to instructions (Thermo Fisher [P36600]) to analyze antibody internalization. To this end, 20,000 FaDu were seeded per 96-well in DMEM + 10% FCS + P/S and incubated for 24 h before treatment with 50 nM pHrodo^{™} Red-labeled anti-EGFR IgG. After 5 min, 1 h, 2 h, 6 h, 24 h and 48 h cells incubated either at 37°C (Figure 11 a) or 4°C (Figure 11 b) were harvested by trypsinization, re-suspended in PBS + 2% (v/v) FCS + 0.02% NaN₃ (PBA) and fluorescence was measured with MACSQuant^{®} VYB (Miltenyi Biotec). The labeled anti-EGFR IgG YU250-H10 was rapidly internalized into FaDu like Cetuximab, while at 4 °C only a marginal uptake of antibodies was observed.

### Example 12: Anti-EGFR antibodies YU250-A02 and YU250-H10 compete with Cetuximab, Matuzumab, Necitumumab and Panitumumab for binding to EGFR

Competition for binding to EGFR with Cetuximab was analyzed by ELISA using immobilized EGFR-moIgG2a-Fc fusion protein (SEQ ID NO: 33) comprising the extracellular domain (aa 25-645) of human EGFR. The EGFR-moIgG2a-Fc fusion protein was coated onto polystyrene microtiter plates at 3 µg/mL in PBS. Remaining binding sites were blocked with PBS, 2% skimmed milk (MPBS). Plates were incubated with a serial dilution of Cetuximab in MPBS. After washing, 1 nM scFv-Fc YU250-A02 (SEQ ID NO: 92) or scFv-Fc YU250-H10 (SEQ ID NO: 93) spiked into dilutions of Cetuximab in MPBS were incubated. After washing, bound scFv-huFc were detected with rabbit serum generated against variable domains (Stork et al., JBC 2008, 12:7804-7812 - diluted 1:1,000) followed by incubation with an HRP-conjugated anti-rabbit-Fc antibody (Abcam - 1:5,000) diluted in MPBS. Bound Cetuximab was detected in separate wells by incubation with an HRP-conjugated anti-human-Fc antibody (1:5,000) diluted in MPBS. As a substrate for detection, 1% TMB and 0.06% H₂O₂ diluted in 100 mM sodium phosphate buffer (pH 6.0) was used. Binding of both IgG YU250-A02 and IgG YU250-H10 to EGFR in ELISA was efficiently blocked in a concentration-dependent manner by Cetuximab as shown below and in Figure 12 a.
IC₅₀ values as determined in the competition assay:
scFv-huFc YU250-A02: 1.29 ± 0.26 nM
scFv-huFc YU250-H10: 1.20 ± 0.17 nM.

Furthermore, competition for binding of Fab-His YU250-A02 or Fab-His YU250-H10 (sequences as defined in Example 4) to EGFR by Cetuximab, IgG GC1118 (SEQ ID NOs: 11 and 12), Matuzumab (SEQ ID NOs: 13 and 14), Necitumumab (SEQ ID NOs: 15 and 16) and Panitumumab (SEQ ID NOs: 17 and 18) was analyzed by ELISA using immobilized EGFR-moIgG2a-Fc fusion protein (SEQ ID NO: 33) comprising the extracellular domain (aa 25 - 645) of human EGFR. The EGFR-moIgG2a-Fc fusion protein was coated onto polystyrene microtiter plates at 3 µg/mL in PBS. Remaining sites were blocked with PBS, 2% skimmed milk (MPBS). Plates were incubated with 200 nM Fab-His YU250-A02 or Fab-His YU250-H10 in MPBS. After washing, 1 nM IgG YU250-A02, IgG YU250-H10 (sequences as defined in Example 4), Cetuximab, GC1118, Matuzumab, Necitumumab or Panitumumab spiked into 200 nM Fab-His YU250-A02 or Fab-His YU250-H10 in MPBS were incubated. After washing, bound IgG were detected by incubation with an HRP-conjugated anti-human-Fc antibody (1:5,000) diluted in MPBS. As a control, bound Fab-His was detected with an HRP-conjugated anti-His antibody (1: 1,000) diluted in MPBS. As a substrate for detection, 1% TMB and 0.06% H₂O₂ diluted in 100 mM sodium phosphate buffer (pH 6.0) was used. Fab-His YU250-A02 and YU250-H10 efficiently blocked binding of Matuzumab, Necitumumab and Panitumumab only a partial inhibition was observed for Cetuximab. IgG GC1118 was able to bind regardless of blocking with Fab-His YU250-A02 or Fab-His YU250-H10 (Figure 12 b, c).

In conclusion, anti-EGFR antibodies comprising the variable domains of YU250-A02 or YU250-H10 bind to an epitope identical or overlapping with the epitopes of Cetuximab, Matuzumab, Necitumumab and Panitumumab, while anti-EGFR antibodies comprising the variable domains of YU250-A02 or YU250-H10 have an epitope distinct from GC1118.

### Example 13: Anti-EGFR antibodies YU250-A02 and YU250-H10 utilize unique interactions to EGFR compared to Cetuximab, GC1118, Matuzumab, Necitumumab and Panitumumab

By site-directed mutagenesis, mutations were introduced into domain III of EGFR for EGFR-moIgG2a-Fc fusion proteins (SEQ ID NOs: 34, 35, 36, 37, 38) comprising the extracellular domain (aa 25 - 645) of human EGFR. Mutated antigens were produced by transient transfection of HEK293-6E as described above, purified from the supernatant by Protein A affinity chromatography (elution with 100 mM glycine buffer pH 3.5) and dialyzed against PBS. The EGFR-moIgG2a-Fc fusion proteins were coated onto polystyrene microtiter plates at 3 µg/mL in PBS. Remaining sites were blocked with PBS, 2% skimmed milk (MPBS). Plates were incubated with a serial dilution row of anti-EGFR IgG in MPBS. Bound IgG was detected with an HRP-conjugated anti-huFc antibody (1:5,000) diluted in MPBS. As a substrate for detection, 1% TMB and 0.06% H₂O₂ diluted in 100 mM sodium phosphate buffer (pH 6.0) was used.

All antibodies bound with similar EC₅₀ values to EGFR wt, F436A and I462A. A slightly reduced binding to G434A was observed for YU250-A02, while all other antibodies bound to this variant. No binding to Q435P was observed for YU250-A02 and YU250-H10, while all other antibodies showed binding to various extent. Furthermore, binding to the double mutant F436A/I462A was diminished for IgG YU250-A02 and IgG YU250-H10 (sequences as defined in Example 4), reduced for Necitumumab and not or only slightly affected for Cetuximab, GC1118, Matuzumab and Panitumumab (Figure 13).

Thus, binding of anti-EGFR antibodies comprising the variable domains of YU250-A02 or YU250-H10 to EGFR can be discriminated from that of Cetuximab, GC118, Matuzumab, Necitumumab and Panitumumab by the mutation Q435P and the double mutation F436A/I462A, which completely abrogated binding of YU250-A02 and YU250-H10, while binding to this mutant could be observed by all other antibodies, although reduced for Necitumumab.

Further mutations at position F381, H383, F381/H383, S484 and K487 (SEQ ID NOs: 39, 40, 41, 42, 43) were studied using substitutions by alanines (Figure 14). Of note, the double mutations F381A/H383A completely abrogated binding of GC118, while no effects were observed for IgG YU250-A02, IgG YU250-H10 and Cetuximab. Furthermore, the mutations S484A and K487A abrogated binding of Matuzumab, while no effects were observed for IgG YU250-A02, IgG YU250-H10 and Cetuximab.

### Example 14: Anti-EGFR antibodies YU250-A02 and YU250-H10 bind to clinically relevant EGFR mutations

In further experiments, we studied binding of IgG YU250-A02, IgG YU250-H10 (sequences as defined in Example 4) and other EGFR antibodies to clinically observed EGFR mutants, including V441D, V441G, S442R, S464L, G465R, K489E and S492R (SEQ ID NOs: 44, 45, 46, 47, 48, 49, 50) (Strickler et al., Cancer Discovery 2018) (Figure 15). IgG YU250-A02 and IgG YU250-H10 were capable of binding to V441D, V441G, S442R, S464L, G465R, K489E and S492R. Thus, anti-EGFR antibodies comprising the variable domains ofYU250-A02 or YU250-H10 are capable of binding seven clinically relevant EGFR mutants. Of note, Cetuximab showed no binding to V441D, S464L, G465R and S492R. Necitumumab showed no binding to V441D and S442R and reduced binding to V441G, G465R and K489E. Panitumumab showed no binding to S442R, S464L, G465R and a slightly reduced binding to K489E. Thus, anti-EGFR antibodies comprising the variable domains of YU250-A02 or YU250-H10 exhibit a distinct binding pattern compared to Cetuximab, Necitumumab and Panitumumab, capable of binding to several clinically relevant EGFR mutants.

The binding properties shown in Examples 13 and 14 are summarized in Figure 16, further highlighting the distinct and unique binding properties of anti-EGFR antibodies comprising the variable domains of YU250-A02 or YU250-H10.

### Example 15: Affinity measurement of binding of anti-EGFR Fab fragments to EGFR using biolayer interferometry

Affinity of monovalent anti-EGFR Fab was determined by biolayer interferometry using the Octet^{®} QK^{e} system (ForteBio). To this end, His-tagged anti-EGFR Fab comprising the anti-EGFR binding moieties MKU011-A7 (SEQ ID NOs: 4 and 5), YU250-A02 (SEQ ID NOs: 6 and 8), YU250-H10 (SEQ ID NOs: 5 and 9) or cetuximab (SEQ ID NOs: 32 and 62) and His-tagged extracellular domain of EGFR wt or comprising the mutations Q435P, F436A/I462A, V441D, S464L, G465R or S492R were used as antigen. FAB2G sensors were used for the measurement and activated by incubation in PBS supplemented with 2% BSA and 0.05% Tween20 for ≥ 10 min at RT. Baseline measurement in PBS supplemented with 2% BSA and 0.05% Tween20 was performed at 1,000 rpm for 120 seconds at RT before loading of Fab antibodies onto sensor by incubation for 180 seconds at RT in 2.5 µg/mL antibody diluted in PBS supplemented with 2% BSA and 0.05% Tween20. Baseline measurement in PBS supplemented with 2% BSA and 0.05% Tween20 was performed at 1,000 rpm for 120 seconds at RT. Measurement of antigen association was performed at 500 nM, 158 nM, 50 nM, 15.8 nM, 5 nM, 1.58 nM, 0.5 nM and 0 nM diluted in PBS supplemented with 2% BSA and 0.05% Tween20 at 1,000 rpm for 300 seconds at RT followed by measurement of antigen dissociation by incubation in PBS supplemented with 2% BSA and 0.05% Tween20 at 1,000 rpm for 300 seconds at RT. Analysis was performed by Data Analysis HT software (version 11.1.2.48). In brief, non-significant binding traces with a response of < 0.05 nm were excluded from the analysis. Additionally, only traces with antigen concentrations of ~10× above and ~10x below the K_{D} were used for calculation of the 1:1 binding model.

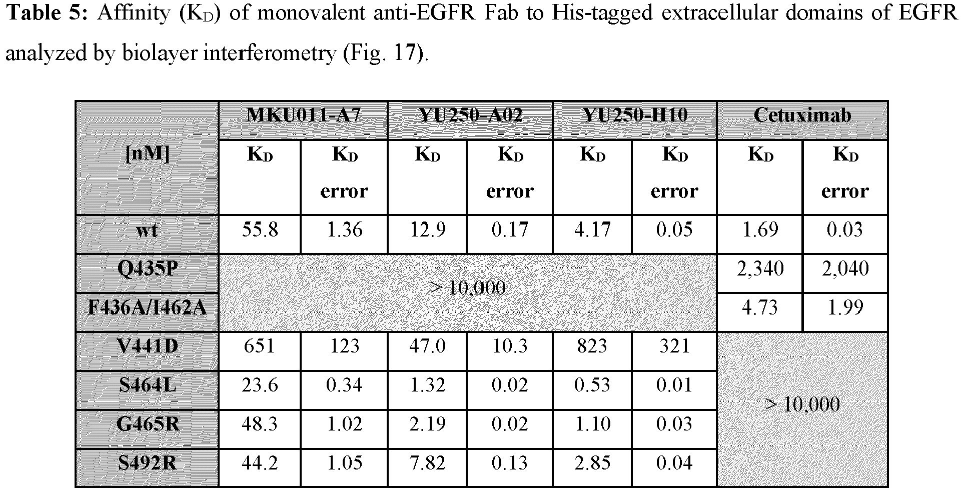

All Fab bound to EGFR wt with a K_{D} < 10⁻⁷ M. No binding (K_{D} > 10⁻⁵ M) was detected for Fab MKU011-A7, YU250-A02 and YU250-H10 to antigen comprising the mutation Q435P or the double mutation F436A/I462A. To these two variants, Fab cetuximab bound with a K_{D} < 10⁻⁵ M. Binding of Fab MKU011-A7, YU250-A02 and YU250-H10 to antigen comprising the mutation V441D, S464L, G465R, or S492R with a K_{D} < 10⁻⁵ M. To these four variants, Fab cetuximab did not bind (K_{D} > 10⁻⁵ M). A summary of the positions of the tested mutations in domain III of EGFR is shown in Figure 18.

These data show that the antibodies comprising the anti-EGFR binding sites of MKU011-A7, YU250-A02 or YU250-H10 bind to EGFR variants with the cetuximab escape mutations V441D, S464L, G465R, or S492R. No binding to Q435P or F436A/I462A EGFR variants was detectable for antibodies comprising the anti-EGFR binding sites of MKU011-A7, YU250-A02 or YU250-H10, indicating that the epitope of these antibodies comprises Q435, F436 and I462.

### Example 16: Anti-EGFR antibodies YU250-A02 and YU250-H10 are cross-reactive to murine EGFR

Binding of anti-EGFR antibodies IgG YU250-A02 and IgG YU250-H10 (sequences as defined in Example 4) and various other EGFR antibodies, including Cetuximab, GC1118, Matuzumab, Necitumumab and Panitumumab, to human and murine EGFR was analyzed by ELISA using immobilized EGFR-His fusion protein comprising the extracellular domain of human EGFR (aa 25 - 645) (SEQ ID NO: 53) or murine EGFR (aa 25 - 647 - Sino Biological [51091-M08H]) (SEQ ID NO: 51) (Figure 19). The antigens were coated onto polystyrene microtiter plates at 2 µg/mL in PBS. Remaining sites were blocked with PBS, 2% skimmed milk (MPBS). Plates were then incubated with 10 nM anti-EGFR IgG (Figure 19 a, b) or a serial dilution row of anti-EGFR IgG antibodies YU250-A02 and YU250-H10 (Figure 19 c, d) in MPBS. Bound IgG was detected with an HRP-conjugated anti-huFc antibody (1:5,000) diluted in MPBS. As a substrate for detection, 1% TMB and 0.06% H₂O₂ diluted in 100 mM sodium phosphate buffer (pH 6.0) was used. Anti-EGFR antibodies IgG YU250-A02 and IgG YU250-H10 bound to human EGFR and were cross-reactive to murine EGFR with similar EC₅₀ values (Table 6). Only GC1118 and to some extend Necitumumab were cross-reactive with mouse EGFR at 10 nM. All other antibodies (Cetuximab, Matuzumab, Panitumumab) did not show cross-reactivity with mouse EGFR at 10 nM.

**Table 6: EC₅₀ values [nM] for binding of anti-EGFR IgG to human and mouse EGFR by ELISA (n=3).**

| | **human EGFR** | **mouse EGFR** |
|---|---|---|
| **YU250-A02** | 0.36 ± 0.04 | 0.38 ± 0.05 |
| **YU250-H10** | 0.28 ± 0.03 | 0.45 ± 0.06 |

### Example 17: Anti-EGFR IgG YU250-H10 is more potent in inhibiting proliferation of human cancer cell line A431 than Cetuximab, GC1118, Matuzumab, Necitumumab and Panitumumab

Binding of anti-EGFR IgG to cells was analyzed by flow cytometry for A431 (Figure 20 a). To this end, 100,000 cells were harvested and incubated with a serial dilution row of antibodies diluted in PBS + 2% (v/v) FCS + 0.02% NaN₃ (PBA) for 1 h in a U-bottom 96-well plate. After washing of each well three times (harvesting of cells by centrifugation for 3 min at 1,500 rpm / 4°C, discarding of supernatant and re-suspending cells in 175 µL PBA), cells were incubated with a PE-conjugated anti-huFc antibody (1:500 - dianova [109-115-098]) for 1 h. After washing of each well three times, the fluorescence was measured with MACSQuant^{®} VYB (Miltenyi Biotec). Anti-EGFR IgG YU250-A02 and YU250-H10 bound with similar potency to the human cancer cell line A431 as Cetuximab, GC1118, Matuzumab, Necitumumab and Panitumumab (less than 2-fold difference in EC₅₀ values - Table 7).

Inhibition of proliferation was analyzed for A431 (Figure 20 b). 2,000 cells per 96- well were seeded in RPMI + 10% FCS + P/S and incubated for 24 h before starving cells in medium containing 0.2% FCS for another 24 h. Serum-starved cells were treated with 10 nM anti-EGFR IgG for 5 d until quantification of cell number using CellTiter-Glo^{®} 2.0 Cell Viability Assay (Promega [G9242]). Anti-EGFR IgG YU250-H10 led to a statistically significant inhibition of proliferation for serum-starved A431 at 10 nM compared to untreated cells.

Inhibition of EGFR signaling was analyzed by Western blotting for A431 (Figure 20 c). 200,000 cells per 6-well were seeded in RPMI + 10% FCS + P/S and incubated for 24 h before starving cells in medium containing 0.2% FCS for another 24 h. Serum-starved cells were treated with 100 nM anti-EGFR IgG for 1 h prior to stimulation with 50 ng/mL EGF (biotechne [236-EG-200]) for 15 min. After cell lysis (150 mM NaCl, 1% Nonident P-40, 10 mM NaF, 20 mM β-glycero-phosphate, 1 mM EDTA, 1 mM Na₃VO₃, 0.5 mM PMSF, 0.25% deoxycholic acid sodium salt, 0.1% SDS, 50 mM Tris pH 7.5, cOmplete^{™} protease inhibitors (Roche)), the concentration of the whole cell lysates was determined (DC protein assay - Bio-Rad [5000116]), equal amounts loaded onto 4-12% NuPAGE^{™} gels (Thermo Fisher [WG1402BOX] and Western blot was performed using iBlot 2 Dry Blotting System (Thermo Fisher [IB23001]. Blots were blocked (0.5% (v/v) Roche blocking reagent (Merck) in PBS, 0.05% (v/v) Tween-20) and signals detected with Fusion Solo S (Vilber) using primary antibodies directed against EGFR (Santa Cruz [sc-03]), pEGFR Y1068 (Cell Signaling [CS#3777]), ERK (Cell Signaling [CS#9107]), pERK T202/Y204 (Cell Signaling [CS#9101]) and α-tubulin (Sigma [05-829]) as well as secondary HRP-conjugated antibodies directed against mouse IgG (dianova [115-035-062]) or rabbit IgG (dianova [111-035-144]). In contrast to anti-EGFR IgG Matuzumab, the anti-EGFR IgG YU250-A02 and IgG YU250-H10 (sequences as defined in Example 4) lead to a reduction of EGFR phosphorylation at position Y1068 for serum-starved A431 stimulated with 50 ng/mL EGF at 100 nM compared to untreated cells. Only treatment with anti-EGFR IgG YU250-H10 lead to an inhibition of the MAPK pathway for serum-starved A431 stimulated with 50 ng/mL EGF at 100 nM compared to untreated cells.

**Table 7: EC₅₀ values [nM] for binding of anti-EGFR IgG to human cancer cell line A431 analyzed by flow cytometry (n=3).**

| | **A431** |
|---|---|
| **YU250-A02** | 1.24 ± 0.35 |
| **YU250-H10** | 1.56 ± 0.42 |
| **Cetuximab** | 1.20 ± 0.30 |
| **GC1118** | 1.67 ± 0.40 |
| **Matuzumab** | 1.68 ± 0.39 |
| **Necitumumab** | 1.32 ± 0.39 |
| **Panitumumab** | 1.31 ± 0.27 |

### Example 18: Anti-EGFR IgG YU250-A02 and YU250-H10 show more potent inhibitory activity regarding proliferation of human cancer cell line DiFi than GC1118

Binding of anti-EGFR IgG to cells was analyzed by flow cytometry for DiFi (Figure 21 a). 100,000 cells were harvested and incubated with a serial dilution row of antibodies diluted in PBS + 2% (v/v) FCS + 0.02% NaN₃ (PBA) for 1 h in a U-bottom 96-well plate. After washing of each well three times (harvesting of cells by centrifugation for 3 min at 1,500 rpm / 4°C, discarding of supernatant and re-suspending cells in 175 µL PBA), cells were incubated with a PE-conjugated anti-huFc antibody (1:500 - dianova [109-115-098]) for 1 h. After washing of each well three times, the fluorescence was measured with MACSQuant^{®} VYB (Miltenyi Biotec). Anti-EGFR IgG YU250-A02 and YU250-H10 bound as good to the human cancer cell line DiFi analyzed by flow cytometry as Cetuximab, GC1118, Matuzumab, Necitumumab and Panitumumab (less than 2-fold difference in EC₅₀ values - Table 8).

Inhibition of proliferation was analyzed for DiFi (Figure 21 b). 2,000 cells per 96- well were seeded in RPMI + 10% FCS + P/S and incubated for 24 h before starving cells in medium containing 0.2% FCS for another 24 h. Serum-starved cells were treated with 10 nM anti-EGFR IgG for 5 d until quantification of cell number using CellTiter-Glo^{®} 2.0 Cell Viability Assay (Promega [G9242]). In contrast to anti-EGFR IgG GC1118, the anti-EGFR IgG YU250-A02 and YU250-H10 led to a statistically significant inhibition of proliferation for serum-starved DiFi at 10 nM compared to untreated cells.

Inhibition of EGFR signaling was analyzed by Western blotting for DiFi (Figure 21 c). 200,000 cells per 6-well were seeded in RPMI + 10% FCS + P/S and incubated for 24 h before starving cells in medium containing 0.2% FCS for another 24 h. Serum-starved cells were treated with 100 nM anti-EGFR IgG for 1 h prior to stimulation with 50 ng/mL EGF (biotechne [236-EG-200]) for 15 min. After cell lysis (150 mM NaCl, 1% Nonident P-40, 10 mM NaF, 20 mM β-glycero-phosphate, 1 mM EDTA, 1 mM Na₃VO₃, 0.5 mM PMSF, 0.25% deoxycholic acid sodium salt, 0.1% SDS, 50 mM Tris pH 7.5, cOmplete^{™} protease inhibitors (Roche)), the concentration of the whole cell lysates was determined (DC protein assay - Bio-Rad [5000116]), equal amounts loaded onto 4-12% NuPAGE^{™} gels (Thermo Fisher [WG1402BOX] and Western blot was performed using iBlot 2 Dry Blotting System (Thermo Fisher [IB23001]. Blots were blocked (0.5% (v/v) Roche blocking reagent (Merck) in PBS, 0.05% (v/v) Tween-20) and signals detected with Fusion Solo S (Vilber) using primary antibodies directed against EGFR (Santa Cruz [sc-03]), pEGFR Y1068 (Cell Signaling [CS#3777]), ERK (Cell Signaling [CS#9107]), pERK T202/Y204 (Cell Signaling [CS#9101]) and α-tubulin (Sigma [05-829]) as well as secondary HRP-conjugated antibodies directed against mouse IgG (dianova [115-035-062]) or rabbit IgG (dianova [111-035-144]). In contrast to anti-EGFR IgG GC1118, the anti-EGFR IgG YU250-A02 and IgG YU250-H10 (sequences as defined in Example 4) led to an inhibition of the MAPK pathway for serum-starved and non-stimulated DiFi at 100 nM comparable to the inhibitory effect of Cetuximab, Matuzumab, Necitumumab and Panitumumab. In contrast to anti-EGFR IgG Matuzumab, the anti-EGFR IgG YU250-A02 and IgG YU250-H10 led to a reduction of EGFR phosphorylation at position Y1068 for serum-starved DiFi stimulated with 50 ng/mL EGF at 100 nM compared to untreated cells.

**Table 8: EC₅₀ values [nM] for binding of anti-EGFR IgG to human cancer cell line DiFi analyzed by flow cytometry (n=3).**

| | **DiFi** |
|---|---|
| **YU250-A02** | 0.64 ± 0.08 |
| **YU250-H10** | 0.73 ± 0.07 |
| **Cetuximab** | 0.50 ± 0.03 |
| **GC1118** | 1.10 ± 0.08 |
| **Matuzumab** | 0.95 ± 0.06 |
| **Necitumumab** | 0.39 ± 0.05 |
| **Panitumumab** | 0.69 ± 0.08 |

### Example 19: Anti-EGFR IgG YU250-A02 and YU250-H10 show more potent inhibitory activity regarding proliferation of human cancer cell line FaDu than GC1118 and Matuzumab

Binding of anti-EGFR IgG to FaDu was analyzed by flow cytometry (Figure 22 a). 100,000 cells were harvested and incubated with a serial dilution row of antibodies diluted in PBS + 2% (v/v) FCS + 0.02% NaN₃ (PBA) for 1 h in a U-bottom 96-well plate. After washing of each well three times (harvesting of cells by centrifugation for 3 min at 1,500 rpm / 4°C, discarding of supernatant and re-suspending cells in 175 µL PBA), cells were incubated with a PE-conjugated anti-huFc antibody (1:500 - dianova [109-115-098]) for 1 h. After washing of each well three times, the fluorescence was measured with MACSQuant^{®} VYB (Miltenyi Biotec). Anti-EGFR IgG YU250-A02 and YU250-H10 bound as good to the human cancer cell line FaDu analyzed by flow cytometry as Cetuximab, GC1118, Matuzumab, Necitumumab and Panitumumab (less than 2-fold difference in EC₅₀ values - Table 9).

Inhibition of proliferation was analyzed for FaDu (Figure 22 b). 2,000 cells per 96- well were seeded in DMEM + 10% FCS + P/S and incubated for 24 h before starving cells in medium containing 0.2% FCS for another 24 h. Serum-starved cells were treated with 10 nM anti-EGFR IgG for 5 d until quantification of cell number using CellTiter-Glo^{®} 2.0 Cell Viability Assay (Promega [G9242]). In contrast to anti-EGFR IgG GC1118 and Matuzumab, the anti-EGFR IgG YU250-A02 and IgG YU250-H10 (sequences as defined in Example 4) led to a statistically significant inhibition of proliferation for serum-starved FaDu at 10 nM compared to untreated cells.

Inhibition of EGFR signaling was analyzed by Western blot for FaDu (Figure 22 c). 200,000 cells per 6-well were seeded in DMEM + 10% FCS + P/S and incubated for 24 h before starving cells in medium containing 0.2% FCS for another 24 h. Serum-starved cells were treated with 100 nM anti-EGFR IgG for 1 h prior to stimulation with 50 ng/mL EGF (biotechne [236-EG-200]) for 15 min. After cell lysis (150 mM NaCl, 1% Nonident P-40, 10 mM NaF, 20 mM β-glycero-phosphate, 1 mM EDTA, 1 mM Na₃VO₃, 0.5 mM PMSF, 0.25% deoxycholic acid sodium salt, 0.1% SDS, 50 mM Tris pH 7.5, cOmplete^{™} protease inhibitors (Roche)), the concentration of the whole cell lysates was determined (DC protein assay - Bio-Rad [5000116]), equal amounts loaded onto 4-12% NuPAGE^{™} gels (Thermo Fisher [WG1402BOX] and Western blot was performed using iBlot 2 Dry Blotting System (Thermo Fisher [IB23001]. Blots were blocked (0.5% (v/v) Roche blocking reagent (Merck) in PBS, 0.05% (v/v) Tween-20) and signals detected with Fusion Solo S (Vilber) using primary antibodies directed against EGFR (Santa Cruz [sc-03]), pEGFR Y1068 (Cell Signaling [CS#3777]), ERK (Cell Signaling [CS#9107]), pERK T202/Y204 (Cell Signaling [CS#9101]) and α-tubulin (Sigma [05-829]) as well as secondary HRP-conjugated antibodies directed against mouse IgG (dianova [115-035-062]) or rabbit IgG (dianova [111-035-144]). In contrast to anti-EGFR IgG Matuzumab, the anti-EGFR IgG YU250-A02 and IgG YU250-H10 led to a stronger reduction of EGFR phosphorylation at position Y1068 for serum-starved FaDu stimulated with 50 ng/mL EGF at 100 nM compared to untreated cells.

**Table 9: EC₅₀ values [nM] for binding of anti-EGFR IgG to human cancer cell line FaDu analyzed by flow cytometry (n=3).**

| | **FaDu** |
|---|---|
| **YU250-A02** | 0.26 ± 0.06 |
| **YU250-H10** | 0.27 ± 0.08 |
| **Cetuximab** | 0.20 ± 0.06 |
| **GC1118** | 0.47 ± 0.12 |
| **Matuzumab** | 0.34 ± 0.09 |
| **Necitumumab** | 0.15 ± 0.03 |
| **Panitumumab** | 0.31 ± 0.08 |

### Example 20: Anti-EGFR IgG YU250-A02 and YU250-H10 show equal inhibitory activity regarding proliferation of human cancer cell line LIM1215 as Cetuximab, GC1118, Matuzumab, Necitumumab and Panitumumab

Binding of anti-EGFR IgG to cells was analyzed by flow cytometry for LIM1215 (Figure 23 a). 100,000 cells were harvested and incubated with a serial dilution row of antibodies diluted in PBS + 2% (v/v) FCS + 0.02% NaN₃ (PBA) for 1 h in a U-bottom 96-well plate. After washing of each well three times (harvesting of cells by centrifugation for 3 min at 1,500 rpm / 4°C, discarding of supernatant and re-suspending cells in 175 µL PBA), cells were incubated with a PE-conjugated anti-huFc antibody (1:500 - dianova [109-115-098]) for 1 h. After washing of each well three times, the fluorescence was measured with MACSQuant^{®} VYB (Miltenyi Biotec). Anti-EGFR IgG YU250-A02 and IgG YU250-H10 (sequences as defined in Example 4) bound as good to the human cancer cell line LIM1215 analyzed by flow cytometry as Cetuximab, GC1118, Matuzumab, Necitumumab and Panitumumab (less than 2-fold difference in EC₅₀ values - Table 10).

Inhibition of proliferation was analyzed for LIM1215 (Figure 23 b). To this end, 2,000 cells per 96-well were seeded in RPMI + 10% FCS + P/S and incubated for 24 h before starving cells in medium containing 0.2% FCS for another 24 h. Serum-starved cells were treated with 10 nM anti-EGFR IgG for 5 d until quantification of cell number using CellTiter-Glo^{®} 2.0 Cell Viability Assay (Promega [G9242]). Anti-EGFR IgG YU250-A02 and IgG YU250-H10 led to a statistically significant inhibition of proliferation for serum-starved LIM1215 at 10 nM compared to untreated cells comparable to Cetuximab, GC1118, Matuzumab, Necitumumab and Panitumumab.

Inhibition of EGFR signaling was analyzed by Western blot for LIM1215 (Figure 23 c). Therefor, 200,000 cells per 6-well were seeded in RPMI + 10% FCS + P/S and incubated for 24 h before starving cells in medium containing 0.2% FCS for another 24 h. Serum-starved cells were treated with 100 nM anti-EGFR IgG for 1 h prior to stimulation with 50 ng/mL EGF (biotechne [236-EG-200]) for 15 min. After cell lysis (150 mM NaCl, 1% Nonident P-40, 10 mM NaF, 20 mM β-glycero-phosphate, 1 mM EDTA, 1 mM NasVOs, 0.5 mM PMSF, 0.25% deoxycholic acid sodium salt, 0.1% SDS, 50 mM Tris pH 7.5, cOmplete^{™} protease inhibitors (Roche)), the concentration of the whole cell lysates was determined (DC protein assay - Bio-Rad [5000116]), equal amounts loaded onto 4-12% NuPAGE^{™} gels (Thermo Fisher [WG1402BOX] and Western blot was performed using iBlot 2 Dry Blotting System (Thermo Fisher [IB23001]. Blots were blocked (0.5% (v/v) Roche blocking reagent (Merck) in PBS, 0.05% (v/v) Tween-20) and signals detected with Fusion Solo S (Vilber) using primary antibodies directed against EGFR (Santa Cruz [sc-03]), pEGFR Y1068 (Cell Signaling [CS#3777]), ERK (Cell Signaling [CS#9107]), pERK T202/Y204 (Cell Signaling [CS#9101]) and α-tubulin (Sigma [05-829]) as well as secondary HRP-conjugated antibodies directed against mouse IgG (dianova [115-035-062]) or rabbit IgG (dianova [111-035-144]). In contrast to anti-EGFR IgG Matuzumab, the anti-EGFR IgG YU250-A02 and IgG YU250-H10 (sequences as defined in Example 4) led to a stronger reduction of EGFR phosphorylation at position Y1068 for serum-starved LIM1215 stimulated with 50 ng/mL EGF at 100 nM compared to untreated cells.

**Table 10: EC₅₀ values [nM] for binding of anti-EGFR IgG to human cancer cell line LIM1215 analyzed by flow cytometry (n=3).**

| | **LIM1215** |
|---|---|
| **YU250-A02** | 0.09 ± 0.03 |
| **YU250-H10** | 0.09 ± 0.02 |
| **Cetuximab** | 0.06 ± 0.02 |
| **GC1118** | 0.15 ± 0.04 |
| **Matuzumab** | 0.12 ± 0.03 |
| **Necitumumab** | 0.05 ± 0.02 |
| **Panitumumab** | 0.11 ± 0.02 |

### Example 21: Anti-EGFR IgG YU250-A02 and YU250-H10 show more potent inhibitory activity regarding proliferation of EGF- or HB-EGF-stimulated human cancer cell lines DiFi and LIM1215 than Matuzumab

Inhibition of proliferation was analyzed for stimulated DiFi (Figure 24 a / c) and LIM1215 (Figure 24 b / d). 2,000 cells per 96-well were seeded in RPMI + 10% FCS + P/S and incubated for 24 h before starving cells in medium containing 0.2% FCS for another 24 h. Serum-starved cells were treated with 100 nM anti-EGFR IgG for 1 h before being stimulated with 1 ng/mL EGF (Figure 24 a / b)) or 3 ng/mL HB-EGF (Figure 24 c / d). After 5 d, quantification of cell number was performed using CellTiter-Glo^{®} 2.0 Cell Viability Assay (Promega [G9242]). In contrast to anti-EGFR IgG Matuzumab, at 100 nM the anti-EGFR IgG YU250-A02 and IgG YU250-H10 (sequences as defined in Example 4) lead to a statistically significant inhibition of proliferation for serum-starved DiFi and LIM1215 stimulated with 1 ng/mL EGF and for serum-starved LIM1215 stimulated with 3 ng/mL HB-EGF.

### Example 22: Anti-EGFR IgG YU250-A02 and YU250-H10 show cell binding to clinically observed EGFR mutations G465R and S492R located in epitope of approved antibodies Cetuximab, Necitumumab and Panitumumab

Stable murine fibroblast cell lines were generated using a retroviral MSCV-based vector system. The sequence of human EGFR (aa 1 - 1,210) was cloned into a pMSCV- LTR-Puromycin vector and transduced cells were selected using 1 µg/mL Puromycin (Sigma [P8833-25MG]) in DMEM + 10% FCS + P/S. Cell lines for the EGFR wildtype (wt) sequence (SEQ ID NO: 54), two clinically observed variants of EGFR (G465R (SEQ ID NO: 55) and S492R (SEQ ID NO: 56)) as well as an empty vector control containing a multiple cloning site instead of EGFR were generated and analyzed for EGFR expression by Western blot (Figure 25 a). 200,000 cells per 6-well were seeded in DMEM + 10% FCS + P/S and incubated for 24 h before cell lysis (150 mM NaCl, 1% Nonident P-40, 10 mM NaF, 20 mM β-glycero-phosphate, 1 mM EDTA, 1 mM NasVOs, 0.5 mM PMSF, 0.25% deoxycholic acid sodium salt, 0.1% SDS, 50 mM Tris pH 7.5, cOmplete^{™} protease inhibitors (Roche)). After cell lysis, the concentration of the whole cell lysates was determined (DC protein assay - Bio-Rad [5000116]), equal amounts loaded onto 4-12% NuPAGE^{™} gels (Thermo Fisher [WG1402BOX] and Western blot was performed using iBlot 2 Dry Blotting System (Thermo Fisher [IB23001]. Blots were blocked (0.5% (v/v) Roche blocking reagent (Merck) in PBS, 0.05% (v/v) Tween-20) and signals detected with Fusion Solo S (Vilber) using primary antibodies directed against EGFR (Santa Cruz [sc- 03]) and α-tubulin (Sigma [05-829]) as well as secondary HRP-conjugated antibodies directed against mouse IgG (dianova [115-035-062]) or rabbit IgG (dianova [111-035-144]).

Binding of anti-EGFR IgG to cells was analyzed by flow cytometry for NIH-3T3 + EGFR wt (Figure 25 b), NIH-3T3 + EGFR G465R (Figure 25 c) and NIH-3T3 + EGFR S492R (Figure 25 d). To this end, 100,000 cells were harvested and incubated with 10 nM of antibodies diluted in PBS + 2% (v/v) FCS + 0.02% NaN₃ (PBA) for 1 h in a U-bottom 96-well plate. After washing of each well three times (harvesting of cells by centrifugation for 3 min at 1,500 rpm / 4°C, discarding of supernatant and re-suspending cells in 175 µL PBA), cells were incubated with a PE-conjugated anti-huFc antibody (1:500 - dianova [109-115-098]) for 1 h. After washing of each well three times, the fluorescence was measured with MACSQuant^{®} VYB (Miltenyi Biotec). Anti-EGFR IgG YU250-A02 and IgG YU250-H10 (sequences as defined in Example 4) bound to NIH-3T3 + EGFR wt, NIH-3T3 + EGFR G465R and NIH-3T3 + EGFR S492R similar as IgG GC1118 and Matuzumab, while binding of Necitumumab to NIH-3T3 + EGFR G465R was strongly reduced. No binding to NIH-3T3 + EGFR G465R was observed for Cetuximab and Panitumumab and Cetuximab did furthermore not bind to NIH-3T3 + EGFR S492R.

### Example 23: Anti-EGFR IgG YU250-A02 and YU250-H10 effectively inhibit EGFR phosphorylation and MAPK pathway activation in stable murine fibroblast cell lines expressing clinically observed EGFR mutations G465R and S492R

Inhibition of EGFR signaling was analyzed by Western blot for NIH-3T3 + EGFR wt (Figure 26 a / b), NIH-3T3 + EGFR G465R (Figure 26 c) and NIH-3T3 + EGFR S492R (Figure 26 d). To this end, 200,000 cells per 6-well were seeded in DMEM + 10% FCS + P/S and incubated for 24 h before starving cells in medium containing 0.2% FCS for another 24 h. Serum-starved cells were treated with 100 nM anti-EGFR IgG for 1 h prior to stimulation with 50 ng/mL EGF (biotechne [236-EG-200]) for 15 min. After cell lysis (150 mM NaCl, 1% Nonident P-40, 10 mM NaF, 20 mM β-glycero-phosphate, 1 mM EDTA, 1 mM NasVOs, 0.5 mM PMSF, 0.25% deoxycholic acid sodium salt, 0.1% SDS, 50 mM Tris pH 7.5, cOmplete^{™} protease inhibitors (Roche)), the concentration of the whole cell lysates was determined (DC protein assay - Bio-Rad [5000116]), equal amounts loaded onto 4-12% NuPAGE^{™} gels (Thermo Fisher [WG1402BOX] and Western blot was performed using iBlot 2 Dry Blotting System (Thermo Fisher [IB23001]. Blots were blocked (0.5% (v/v) Roche blocking reagent (Merck) in PBS, 0.05% (v/v) Tween-20) and signals detected with Fusion Solo S (Vilber) using primary antibodies directed against EGFR (Santa Cruz [sc-03]), pEGFR Y1068 (Cell Signaling [CS#3777]), ERK (Cell Signaling [CS#9107]), pERK T202/Y204 (Cell Signaling [CS#9101]) and α-tubulin (Sigma [05-829]) as well as secondary HRP-conjugated antibodies directed against mouse IgG (dianova [115-035-062]) or rabbit IgG (dianova [111-035-144]).

In contrast to anti-EGFR IgG Matuzumab, the anti-EGFR IgG YU250-A02 and IgG YU250-H10 (sequences as defined in Example 4) led to a stronger reduction of EGFR phosphorylation at position Y1068 and stronger inhibition of the MAPK pathway for serum-starved NIH-3T3 + EGFR wt stimulated with 50 ng/mL EGF at 100 nM compared to untreated cells.

In contrast to anti-EGFR IgG Cetuximab, the anti-EGFR IgG YU250-A02 and IgG YU250-H10 led to a reduction of EGFR phosphorylation at position Y1068 and inhibition of the MAPK pathway for serum-starved NIH-3T3 + EGFR G465R and NIH-3T3 + EGFR S492R stimulated with 50 ng/mL EGF at 100 nM compared to untreated cells.

In contrast to anti-EGFR IgG Necitumumab and Panitumumab, the anti-EGFR IgG YU250-A02 and IgG YU250-H10 lead to a reduction of EGFR phosphorylation at position Y1068 and inhibition of the MAPK pathway for serum-starved NIH-3T3 + EGFR G465R stimulated with 50 ng/mL EGF at 100 nM compared to untreated cells.

In contrast to anti-EGFR IgG Necitumumab and Panitumumab which still bound with at least 80% of the signal intensity to the NIH-3T3 + EGFR S492R compared to IgG GC1118 analyzed at 10 nM by flow cytometry (Figure 26 d), the anti-EGFR IgG YU250-A02 and IgG YU250-H10 led to a reduction of EGFR phosphorylation at position Y1068 which also translated into inhibition of the MAPK pathway for serum-starved NIH-3T3 + EGFR S492R stimulated with 50 ng/mL EGF at 100 nM compared to untreated cells.

### Example 24: Bispecific EGFRxCD3 antibodies in various formats for YU250-H10 lead to an efficient target cell killing in co-culture assays with PBMCs isolated from a healthy donor

Binding of bispecific EGFRxCD3 antibodies to cells was analyzed by flow cytometry for CD3-expressing Jurkat (Figure 27 a) as well as for EGFR-expressing cancer cells FaDu (Figure 27 b) and LIM1215 (Figure 27 c). To this end, 100,000 cells were harvested and incubated with a serial dilution row of bispecific antibodies diluted in PBS + 2% (v/v) FCS + 0.02% NaN₃ (PBA) for 1 h in a U-bottom 96-well plate. After washing of each well three times (harvesting of cells by centrifugation for 3 min at 1,500 rpm / 4°C, discarding of supernatant and re-suspending cells in 175 µL PBA), cells were incubated with a PE-conjugated anti-His antibody for scDb and scDb-scFv (1:250 - Miltenyi [130-120-718]) or a PE-conjugated anti-huFc antibody for eIg and Fab-eIg (1:500 - dianova [109-115-098]) for 1 h. After washing of each well three times, the fluorescence was measured with MACSQuant^{®} VYB (Miltenyi Biotec). Bispecific EGFRxCD3 antibodies of the formats scDb (SEQ ID NO: 19), scDb-scFv (SEQ ID NO: 20), eIg (SEQ ID NOs: 9, 21 and 23, 24; examples of this format are shown in Figs. 33B and 33C) and Fab-eIg (SEQ ID NOs: 9, 21 and 22, 24; examples of this format are shown Figs. 33D and 33E) containing the variable domains of anti-EGFR antibody YU250-H10 and of the anti-human CD3 antibody huU3 (humanized version of UCHT1) variable domain sequences (V_{H} as set forth in SEQ ID NO: 96, V_{L} as set forth in SEQ ID NO: 97) bound to CD3-expressing Jurkat as well as to EGFR-expressing FaDu and LIM1215 cells.

Cytotoxicity of bispecific EGFRxCD3 antibodies was analyzed on EGFR-expressing FaDu (Figure 27 d) and LIM1215 (Figure 27 e) induced by activation of T-cells isolated as PBMCs from a healthy donor. To this end, 20,000 target cells (FaDu or LIM1215) were seeded per 96-well in DMEM + 10% FCS + P/S (FaDu) or RPMI + 10% FCS + P/S (LIM1215) and incubated for 24 h. PBMCs isolated from a healthy donor by low density gradient centrifugation (PromoCell Lymphocyte Separation Medium 1077 [C-44010]) and stored at -80°C in FCS + 10% DMSO were thawed 24 h prior to usage in cytotoxicity assay and cultured in RPMI + 10% FCS + P/S. Target cells were pre-incubated with dilution row of bispecific antibodies diluted in RPMI + 10% FCS + P/S for 15 min before adding 200,000 PBMCs per 96-well (effector-to-target cell ratio (E:T) - 10:1). Viable cells were quantified by crystal violet staining 72 h after treatment measuring the optical density at 550 nm (TECAN Spark^{®}).

Bispecific, trivalent EGFRxCD3 antibodies containing the variable domains of anti-EGFR antibody YU250-H10 (scDb-scFv: 1.68 ± 0.25 nM / Fab-eIg: 0.76 ± 0.11 nM) showed an increased binding to FaDu compared to bivalent EGFRxCD3 antibodies (scDb: 1.68 ± 0.25 nM / eIg: 2.17 ± 0.26 nM) resulting in a more potent cytotoxicity in a co-culture assay with PBMCs isolated from a healthy donor.

Bispecific, trivalent EGFRxCD3 antibodies containing the variable domains of anti-EGFR antibody YU250-H10 (scDb-scFv: 0.31 ± 0.04 nM / Fab-eIg: 0.27 ± 0.04 nM) showed an increased binding to LIM1215 compared to bivalent EGFRxCD3 antibodies (scDb: 1.02 ± 0.08 nM / eIg: 0.85 ± 0.07 nM) resulting in a more potent cytotoxicity in a co-culture assay with PBMCs isolated from a healthy donor.

**Table 11: EC₅₀ values for cell binding [nM] and IC₅₀ values [pM] for cytotoxicity of bispecific EGFRxCD3 antibodies containing the anti-EGFR antibody YU250-H10**

| | **scDb** | **scDb-scFv** | **eIg** | **Fab-eIg** |
|---|---|---|---|---|
| | **EC₅₀ for cell binding [nM]** | | | |
| **Jurkat** | 7.56 ± 0.45 | >10 | >10 | >10 |
| **FaDu** | 1.68 ± 0.25 | 0.74 ± 0.14 | 2.17 ± 0.26 | 0.76 ± 0.11 |
| **LIM1215** | 1.02 ± 0.08 | 0.31 ± 0.04 | 0.85 ± 0.07 | 0.27 ± 0.04 |

| | **IC₅₀ for cytotoxicity [pM]** | | | |
|---|---|---|---|---|
| **FaDu** | 18.1 ± 3.8 | 4.4 ± 0.9 | 108.8 ± 32.0 | 7.0 ± 1.4 |
| **LIM1215** | 60.2 ± 17.0 | 4.0 ± 1.0 | 66.0 ± 17.1 | 3.0 ± 0.7 |

### Example 25: Bispecific EGFRxCD3 antibodies of the eIg and Fab-eIg format for anti-EGFR antibodies MKU011-A7 and YU250-H10 lead to an efficient target cell killing in co-culture assays with PBMCs isolated from healthy donors

Binding of bispecific EGFRxCD3 antibodies in the two distinct formats eIg and Fab-eIg comprising the variable domains of MKU011-A7 or YU250-H10 to cells was analyzed by flow cytometry for FaDu (Figure 28 a), HCT-116 (Figure 28 c) and SW620 (Figure 28 e). To this end, 100,000 cells were harvested and incubated with a serial dilution row of bispecific antibodies diluted in PBS + 2% (v/v) FCS + 0.02% NaN₃ (PBA) for 1 h in a U-bottom 96-well plate. After washing of each well three times (harvesting of cells by centrifugation for 3 min at 1,500 rpm / 4°C, discarding of supernatant and re-suspending cells in 175 µL PBA), cells were incubated with a PE- conjugated anti-huFc antibody (1:500 - dianova [109-115-098]) for 1 h. After washing of each well three times, the fluorescence was measured with MACSQuant^{®} VYB (Miltenyi Biotec). Bispecific EGFRxCD3 antibodies of the formats eIg and Fab-eIg containing the anti-EGFR antibody MKU011-A7 or YU250-H10 and the anti-human CD3 antibody huU3 (humanized version of UCHT1) variable domain sequences bound to EGFR-expressing FaDu, HCT-116 and SW620 cells.

Cytotoxicity of bispecific EGFRxCD3 antibodies on EGFR-expressing FaDu (Figure 28 b), HCT-116 (Figure 28 d) and SW620 (Figure 28 f) induced by activation of T-cells isolated as PBMCs from healthy donors. To this end, 20,000 target cells (FaDu, HCT-116 or SW620) were seeded per 96-well in DMEM + 10% FCS + P/S (FaDu and SW620) or RPMI + 10% FCS + P/S (HCT-116) and incubated for 24 h. PBMCs isolated from healthy donors by low density gradient centrifugation (PromoCell Lymphocyte Separation Medium 1077 [C-44010]) and stored at -80°C in FCS + 10% DMSO were thawed 24 h prior to usage in cytotoxicity assay and cultured in RPMI + 10% FCS + P/S. Target cells were pre-incubated with dilution row of bispecific antibodies diluted in RPMI + 10% FCS + P/S for 15 min before adding 200,000 PBMCs per 96-well (effector-to-target cell ratio (E:T) - 10:1). Viable cells were quantified by crystal violet staining 72 h after treatment measuring the optical density at 550 nm (TECAN Spark^{®}).

Bispecific, bivalent EGFRxCD3 eIg antibodies containing the variable domains of YU250-H10 (SEQ ID NOs: SEQ ID NOs: 9, 21 and 23, 24) (FaDu: 0.78 ± 0.06 nM / HCT-116: 0.46 ± 0.03 nM) show an increased binding to FaDu and HCT-116 compared to bivalent EGFRxCD3 eIg antibodies containing the variable domains of MKU011-A7 (SEQ ID NOs: 4, 21 and 23, 24) (FaDu: 3.28 ± 0.17 nM/HCT-116: 4.28 ± 0.24 nM) resulting in a more potent cytotoxicity in co-culture assays with PBMCs isolated from healthy donors.

Bispecific, trivalent EGFRxCD3 Fab-eIg antibodies containing the variable domains of YU250-H10 (SEQ ID NOs: 9, 21 and 22, 24) (FaDu: 0.32 ± 0.04 nM / HCT-116: 0.13 ± 0.02 nM) show similar binding to FaDu and HCT-116 compared to trivalent EGFRxCD3 EGFRxCD3 Fab-eIg antibodies containing the variable domains of MKU011-A7 (SEQ ID NOs: 4, 21 and 22, 24) (FaDu: 0.40 ± 0.05 nM / HCT-116: 0.16 ± 0.02 nM) resulting in equivalent cytotoxicity in co-culture assays with PBMCs isolated from healthy donors (less than 2-fold difference in EC₅₀ values for cell binding).

**Table 12: EC₅₀ values for cell binding [nM] and IC₅₀ values [pM] for cytotoxicity of bispecific EGFRxCD3 antibodies containing the anti-EGFR antibody MKU011-A7 or YU250-H10**

| | **MKU011-A7** | | **YU250-H10** | |
|---|---|---|---|---|
| | **eIg** | **Fab-eIg** | **eIg** | **Fab-eIg** |
| | **EC₅₀ for cell binding [nM]** | | | |
| **FaDu** | 3.28 ± 0.17 | 0.40 ± 0.05 | 0.78 ± 0.06 | 0.32 ± 0.04 |
| **HCT-116** | 4.28 ± 0.24 | 0.16 ± 0.02 | 0.46 ± 0.03 | 0.13 ± 0.02 |
| **SW620** | 0.74 ± 0.11 | 0.03 ± 0.01 | 0.21 ± 0.03 | 0.03 ± 0.01 |

| | **ICso for cytotoxicity [pM]** | | | |
|---|---|---|---|---|
| **FaDu** | 140.0 ± 20.8 | 4.7 ± 2.0 | 48.0 ± 73.6 | 4.8 ± 1.7 |
| **HCT-116** | 228.8 ± 132.8 | 4.7 ± 1.2 | 46.6 ± 20.2 | 3.0 ± 0.9 |
| **SW620** | 32.0 ± 42.8 | 16.8 ± 14.2 | 54.2 ± 99.5 | 4.9 ± 4.7 |

### Example 26: Bispecific, trivalent EGFRxCD3 antibody with anti-EGFR antibody YU250-H10 mediate efficient target cell killing for stable murine fibroblast cell lines expressing the clinically observed EGFR mutations G465R and S492R

A bispecific Fab-eIg antibody was generated as a control by combining the binding site of anti-EGFR Cetuximab with a humanized version of anti-human CD3 antibody UCHT1 (huU3). The sequences of the individual chains of each bispecific antibody were cloned and expressed in suspension culture adapted HEK293-6E cells. The protein was purified from the supernatant of transiently transfected cells by Protein A affinity chromatography. Size exclusion chromatography after preparative FPLC using a TSKgel SuperSW mAb HR column (Tosoh) confirmed the integrity of the protein (Figure 29 a).

Cytotoxicity of bispecific EGFRxCD3 Fab-eIg antibodies for NIH-3T3 + empty vector, NIH-3T3 + EGFR wt, NIH-3T3 + EGFR G465R and NIH-3T3 + EGFR S492R induced by activation of T-cells isolated as PBMCs from healthy donors (Figure 29 b). 5,000 target cells were seeded per 96-well in DMEM + 10% FCS + P/S and incubated for 24 h. PBMC's isolated from healthy donors by low density gradient centrifugation (PromoCell Lymphocyte Separation Medium 1077 [C-44010]) and stored at -80°C in FCS + 10% DMSO were thawed 24 h prior to usage in cytotoxicity assay and cultured in RPMI + 10% FCS + P/S. Target cells were pre- incubated with 10 nM of bispecific antibodies diluted in RPMI + 10% FCS + P/S for 15 min before adding 50,000 PBMC's per 96-well (effector-to-target cell ratio (E:T) - 10:1). Viable cells were quantified by crystal violet staining 72 h after treatment measuring the optical density at 550 nm (TECAN Spark^{®}).

At 10 nM, the bispecific EGFRxCD3 Fab-eIg antibody containing the variable domains of YU250-H10 and the anti-human CD3 antibody huU3 (humanized version of UCHT1) variable domain sequences (SEQ ID NOs: 9, 21 and 22, 24), led to a cell killing of >50% for NIH-3T3 + EGFR wt, NIH-3T3 + EGFR G465R and NIH-3T3 + EGFR S492R compared to non-treated cells. At 10 nM, the bispecific EGFRxCD3 Fab-eIg antibody containing the anti-EGFR antibody Cetuximab and the anti-human CD3 antibody huU3 (humanized version of UCHT1) variable domain sequences, led to a cell killing of >50% for NIH-3T3 + EGFR wt compared to non-treated cells, while failing to kill cells expressing the EGFR mutations G465R and S492R.

**Table 13: Cytotoxicity of bispecific EGFRxCD3 antibodies on stable NIH-3T3 cell lines**

| | **Fab-eIg YU250-H10** | **Fab-eIg Cetuximab** |
|---|---|---|
| | **Cell viability at 10 nM [%]** | |
| **NIH-3T3** + **empty vector** | 69.48 ± 20.23 | 85.49 ± 14.96 |
| **NIH-3T3 + EGFR wt** | 26.06 ± 12.84 | 20.54 ± 6.42 |
| **NIH-3T3** + **EGFR G465R** | 36.00 ± 29.13 | 99.67 ± 28.71 |
| **NIH-3T3** + **EGFR S492R** | 44.03 ± 36.68 | 93.40 ± 3.57 |

### Example 27: Bispecific EGFRxCD3 antibodies for YU250-H10 as surrogates for in vivo studies in syngeneic mouse models bind to stable CT-26 + EGFR wt and CD3+ murine spleenocytes isolated from BALB/c

A stable murine cancer cell line was generated using a retroviral MSCV-based vector system. To this end, the sequence of human EGFR (aa 1 - 1,210) (SEQ ID NO: 54) was cloned into a pMSCV-LTR- Puromycin vector and transduced cells were selected using 1 µg/mL Puromycin (Sigma [P8833-25MG]) in RPMI + 10 mM HEPES + 1 mM sodium + 0.1 mM non-essential amino acids + 10% FCS + P/S. Cell lines for the EGFR wildtype (wt) sequence as well as an empty vector control containing a multiple cloning site instead of EGFR were generated. EGFR expression was validated by flow cytometry (Figure 30 a) and Western blot (Figure 30 b). Binding of a murine anti-EGFR antibody (Biolegend [352902]) to CT-26 + empty vector and CT-26 + EGFR wt was analyzed by flow cytometry. To this end, 100,000 cells were harvested and incubated with 1:100 diluted murine anti-EGFR antibody in PBS + 2% (v/v) FCS + 0.02% NaN₃ (PBA) for 1 h at 4°C in a U-bottom 96-well plate. After washing of each well three times (harvesting of cells by centrifugation for 3 min at 1,500 rpm / 4°C, discarding of supernatant and re-suspending cells in 175 µL PBA), cells were incubated with a FITC- conjugated anti-moF(ab')2 antibody contained in the QIFIKIT^{®} (Agilent [K0078]) for 1 h at 4°C. After washing of each well three times, the fluorescence was measured with MACSQuant^{®} Analyzer (Miltenyi Biotec) and receptor expression was quantified using calibration beads contained in the QIFIKIT^{®} (Agilent [K0078]). For Western blot, 200,000 cells per 6-well were seeded in RPMI + 10 mM HEPES + 1 mM sodium + 0.1 mM non-essential amino acids + 10% FCS + P/S and incubated for 24 h before cell lysis (150 mM NaCl, 1% Nonident P-40, 10 mM NaF, 20 mM β-glycero-phosphate, 1 mM EDTA, 1 mM Na₃VO₃, 0.5 mM PMSF, 0.25% deoxycholic acid sodium salt, 0.1% SDS, 50 mM Tris pH 7.5, cOmplete^{™} protease inhibitors (Roche)). After cell lysis, the concentration of the whole cell lysates was determined (DC protein assay - Bio-Rad [5000116]), equal amounts loaded onto 4-12% NuPAGE^{™} gels (Thermo Fisher [WG1402BOX] and Western blot was performed using iBlot 2 Dry Blotting System (Thermo Fisher [IB23001]. Blots were blocked (0.5% (v/v) Roche blocking reagent (Merck) in PBS, 0.05% (v/v) Tween-20) and signals detected with Fusion Solo S (Vilber) using primary antibodies directed against EGFR (Santa Cruz [sc-03]) and α-tubulin (Sigma [05-829]) as well as secondary HRP-conjugated antibodies directed against mouse IgG (dianova [115-035-062]) or rabbit IgG (dianova [111-035-144]).

Binding of bispecific antibodies containing the binding site of anti-EGFR antibody YU250-H10 and the Armenian hamster anti-murine CD3 antibody 2C11 (SEQ ID NOs: 120, 121) was analyzed by flow cytometry for CT-26 + EGFR wt (Figure 30 c). To this end, 100,000 cells were harvested and incubated with a serial dilution row of bispecific antibodies diluted in PBS + 2% (v/v) FCS + 0.02% NaN₃ (PBA) for 1 h at 4°C in a U-bottom 96-well plate. After washing of each well three times (harvesting of cells by centrifugation for 3 min at 1,500 rpm / 4°C, discarding of supernatant and re-suspending cells in 175 µL PBA), cells were incubated with a PE-conjugated anti-huFc antibody (1:500 - dianova [109-115-098]) for 1 h at 4°C. After washing of each well three times, the fluorescence was measured with MACSQuant^{®} VYB (Miltenyi Biotec).

Binding of bispecific antibodies containing the binding site of anti-EGFR antibody YU250-H10 and the Armenian hamster anti-murine CD3 antibody 2C11, was analyzed by flow cytometry for CD3+ spleenocytes (Figure 30 d). To this end, murine spleenocytes were isolated from BALB/c mice and 100,000 cells were incubated with a serial dilution row of bispecific antibodies diluted in PBS + 2% (v/v) FCS + 0.02% NaN₃ (PBA) for 1 h at 4°C in a U-bottom 96-well plate. After washing of each well three times (harvesting of cells by centrifugation for 3 min at 1,500 rpm / 4°C, discarding of supernatant and re-suspending cells in 175 µL PBA), cells were incubated with a PE-conjugated anti-huFc antibody (1:500 - dianova [109-115-098]) for 1 h at 4°C. After washing of each well three times, the cells were stained with an anti-moCD3-FITC antibody (Miltenyi Biotec [130-119-798]) for 30 min at 4°C. After washing of each well one time, the fluorescence was measured with MACSQuant^{®} Analyzer (Miltenyi Biotec).

Bispecific EGFRxCD3 antibodies of the formats eIg (SEQ ID NOs: 25, 26, 27 and 28) and Fab-eIg (SEQ ID NOs: 25, 26, 27 and 29) containing the anti-EGFR antibody YU250-H10 and the anti-murine CD3 antibody 2C11 variable domain sequences bound to CT-26 + EGFR wt and CD3+ murine spleenocytes isolated from BALB/c.

### Example 28: Bispecific EGFRxCD3 antibodies for YU250-H10 as surrogates for in vivo studies in syngeneic mouse models activate and induce proliferation of T-cells of murine spleenocytes isolated from BALB/c

Early activation (Figure 31 a / b) and proliferation (Figure 31 c / d) of CD4⁺/CD8⁺ murine T-cells analyzed by flow cytometry for bispecific antibodies containing the binding site of anti-EGFR antibody YU250-H10 and the Armenian hamster anti-murine CD3 antibody 2C11. 10,000 CT-26-EGFR-wt were seeded per 96-well in RPMI + 10 mM HEPES + 1 mM sodium + 0.1 mM non-essential amino acids + 10% FCS + P/S and incubated for 24 h. Target cells were pre-incubated with a serial dilution row of bispecific antibodies diluted in RPMI + 10% FCS + 50 µM β-ME + P/S for 15 min before adding 100,000 murine spleenocytes isolated from BALB/c per 96-well (effector-to-target cell ratio (E:T) - 10:1). For analysis of T-cell proliferation, the murine spleenocytes were stained with CellTrace^{™} CFSE Cell Proliferation Kit (Thermo Fisher [C34554]). After 24 h (early activation) or 144 h (proliferation), murine spleenocytes were harvested by transfer to U-bottom 96-well plate, centrifugation for 3 min at 1,500 rpm / 4°C and discarding medium. For analysis of early activation, murine spleenocytes were stained with anti-moCD3-FITC (Miltenyi Biotec [130-119-798]), anti-moCD4-VioBlue (Miltenyi Biotec [130-118-696]), anti-moCD8-PE (Miltenyi Biotec [130-123-781]) and anti-moCD69-APC (Miltenyi Biotec [130-115-576]) antibodies. For analysis of proliferation, murine spleenocytes were stained with anti-moCD3-PE-Vio770 (Miltenyi Biotec [130-116-494]), anti-moCD4-VioBlue (Miltenyi Biotec [130-118-696]) and anti-moCD8-PE (Miltenyi Biotec [130-123-781]) antibodies. Antibodies were diluted 1:100 in PBS + 2% (v/v) FCS + 0.02% NaN₃ (PBA), incubated for 30 min at 4°C. After washing of each well one time (harvesting of cells by centrifugation for 3 min at 1,500 rpm / 4°C, discarding of supernatant and re-suspending cells in 175 µL PBA), the fluorescence was measured with MACSQuant^{®} Analyzer (Miltenyi Biotec).

Bispecific EGFRxCD3 antibodies of the formats eIg (SEQ ID NOs: 25, 26, 27 and 28) and Fab-eIg (SEQ ID NOs: 25, 26, 27 and 29) containing the variable domains of YU250-H10 and the anti-murine CD3 antibody 2C11 variable domain sequences activated (CD69⁺) and induced proliferation of CD4⁺/CD8⁺ murine T-cells from BALB/c in co-culture assays with CT-26 + EGFR wt. The activity of the Fab-eIg was strongly increased compared to the eIg.

### Example 29: Bispecific EGFRxCD3 Fab-eIg surrogate for YU250-H10 inhibits tumor growth in a syngeneic mouse model with CT-26 +EGFR wt in BALB/c

Pharmacokinetics (Figure 32 a) for bispecific EGFRxCD3 eIg (SEQ ID NOs: 25, 26, 27 and 28) or Fab-eIg (SEQ ID NOs: 25, 26, 27 and 29) generated by combining the binding site of anti-EGFR YU250-H10 with the Armenian hamster anti-murine CD3 antibody 2C11 was analyzed by i.v. injection of 25 µg (eIg) or 30 µg (Fab-eIg) into tail vein of 8- to 12-week-old BALB/c. Sera were prepared by incubation of blood samples on ice for 20 min followed by centrifugation for 30 min at 4°C / 13,200 rpm and stored at -20°C until analysis. Quantification of antibody concentrations for Fab-eIg was performed by ELISA using immobilized EGFR-moIgG2a-Fc fusion protein comprising the extracellular domain (aa 25 - 645) of human EGFR (SEQ ID NO: 33). The EGFR-moIgG2a-Fc fusion protein was coated onto polystyrene microtiter plates at 3 µg/mL in PBS. Remaining sites were blocked with PBS, 2% skimmed milk (MPBS). Plates were incubated with serum diluted in MPBS. Bound antibodies were detected with an HRP-conjugated anti-huFc antibody (1:5,000) diluted in MPBS. As a substrate for detection, 1% TMB and 0.06% H₂O₂ diluted in 100 mM sodium phosphate buffer (pH 6.0) was used. Quantification of antibody concentrations for eIg was performed by flow cytometry using murine spleenocytes isolated from BALB/c. To this end, 100,000 cells were incubated with serum diluted in PBS + 2% (v/v) FCS + 0.02% NaN₃ (PBA) for 1 h at 4°C in a U-bottom 96-well plate. After washing of each well three times (harvesting of cells by centrifugation for 3 min at 1,500 rpm / 4°C, discarding of supernatant and re-suspending cells in 175 µL PBA), cells were incubated with a PE-conjugated anti-huFc antibody (1:500 - dianova [109-115-098]) for 1 h at 4°C. After washing of each well three times, the cells were stained with an anti-moCD3-FITC antibody (Miltenyi Biotec [130-119-798]) for 30 min at 4°C. After washing of each well one time, the fluorescence was measured with MACSQuant^{®} Analyzer.

Inhibition of tumor growth was analyzed for different doses of bispecific EGFRxCD3 eIg (25 µg / 2.5 µg / 0.25 µg) or Fab-eIg (30 µg / 3 µg / 0.3 µg) in a syngeneic mouse model (Figure 32 b). To this end, 1×10⁶ CT-26 + EGFR wt were injected into left and right flank of 8- to 12-week- old BALB/c. Tumor volumes were measured using a sliding gauge. Mice were treated twice (11 and 15 days after tumor cell inoculation) and tumor volumes are shown after 17 days. Treatment of a syngeneic mouse model (CT-26 + EGFR wt in BALB/c) with 30 µg or 3 µg EGFRxCD3 Fab-eIg surrogate containing the anti-EGFR antibody YU250-H10 in combination with the Armenian hamster anti-murine CD3 antibody 2C11 resulted in a statistically significant reduction of tumor growth compared to treatment with PBS.

## Claims

1. An antigen-binding protein comprising a first antibody variable domain and a second antibody variable domain and wherein the variable domains form a binding site that:
a) specifically binds to a human epidermal growth factor receptor (EGFR) variant having one or more of the mutations V441D, S464L, G465R, and S492R with a KD < 10⁻⁵ molar determined by biolayer interferometry (BLI);
b) does not bind specifically to human EGFR having the mutation Q435P or double mutation F436A/I462A; and
wherein binding of the antigen-binding protein to human EGFR blocks ligand-induced activation of human EGFR.

2. The antigen-binding protein according to claim 1, wherein the variable domains form a binding site that specifically binds to domain III of EGFR (SEQ ID NO: 61).

3. The antigen-binding protein according to claim 1 or 2, wherein the antigen-binding protein is selected from the group consisting of an antibody or an antigen-binding fragment thereof, and a chimeric antigen receptor (CAR);
wherein the antigen-binding protein is preferably selected from the group consisting Fab, Fab', (Fab')2, Fv, disulfide-linked Fv, BsFv, dsFv, (dsFv)2, dsFv-dsFv', scFv, scFv dimer, single chain domain antibody, diabody, ds diabody, bivalent domain antibody.

4. The antigen-binding protein according to claim 3, wherein the antibody is selected from the group consisting of a chimeric antibody, a humanized antibody, a bispecific antibody, and a multispecific antibody; preferably wherein the antibody is a human antibody.

5. The antigen-binding protein according to any one of claims 1 to 4, wherein the first antibody variable domain comprises the light chain complementary determining region (CDRL) 1 (CDRL1), CDRL2, CDRL3 of the variable light chain region of SEQ ID NO: 83, 84 or 85 and/or the second variable domain comprises the CDRH1, CDRH2 and CDRH3 of the heavy chain variable region of SEQ ID NO: 74 or 75, wherein the CDRs are identified by the Kabat definition, the Chothia definition, the IMGT definition or the contact definition and wherein each CDR may comprise one or two amino acid substitutions.

6. The antigen-binding protein according to any one of claims 1 to 5, wherein:
(a) the first antibody variable domain comprises a
CDRL1 of SEQ ID NO: 86,
CDRL2 of SEQ ID NO: 87, and
CDRL3 of SEQ ID NO: 88;
(b) the second antibody variable domain comprises a
CDRH1 of SEQ ID NO: 71
CDRH2 of SEQ ID NO: 72, and
CDRH3 of SEQ ID NO: 73,
preferably wherein
(a) the first antibody variable domain comprises a
CDRL1 of SEQ ID NO: 76 or 77,
CDRL2 of SEQ ID NO: 78 or 79, and
CDRL3 of SEQ ID NO: 80, 81 or 82;
(b) the second antibody variable domain comprises a
CDRH1 of SEQ ID NO: 71,
CDRH2 of SEQ ID NO: 72, and
CDRH3 of SEQ ID NO: 73;
and wherein each CDR may comprise one or two amino acid substitutions.

7. The antigen-binding protein according to any one of claims 1 to 6, wherein the first antibody variable domain comprises one or more of the framework (FR) sequences of SEQ ID NO: 83, 84 or 85, or a variant of the FR sequence having at least 90% sequence identity to the FR sequences of SEQ ID NO: 83, 84 or 85, and/or wherein the second antibody variable domain comprises one or more of the FR sequences of SEQ ID NO: 74 or 75, or a variant of the FR sequence having at least 90% sequence identity to the FR sequences of SEQ ID NO: 74 or 75, wherein the FR are identified by the Kabat definition, the Chothia definition, the IMGT definition or the contact definition, preferably
wherein the first variable domain comprises one of the amino acid sequences according to SEQ ID NO: 83, 84 or 85, and wherein the second variable domain comprises one of the amino acid sequences according to SEQ ID NO: 74 or 75; more preferably
wherein the antigen-binding protein comprises one of the combinations:
a) SEQ ID NO: 74 and SEQ ID NO: 83,
b) SEQ ID NO: 75 and SEQ ID NO: 84, or
c) SEQ ID NO: 74 and SEQ ID NO: 85;
and wherein each CDR may comprise one or two amino acid substitutions.

8. The antigen-binding protein according to any one of claims 1 to 7, comprising the following sequences:
a) CDRH1, CDRH2 and CDRH3 of SEQ ID NOs: 71, 72 and 73, and CDRL1, CDRL2 and CDRL3 of SEQ ID NOs: 76, 78 and 80,
b) CDRH1, CDRH2 and CDRH3 of SEQ ID NOs: 71, 72 and 73, and CDRL1, CDRL2 and CDRL3 of SEQ ID NOs: 77, 79 and 81, or
c) CDRH1, CDRH2 and CDRH3 of SEQ ID NOs: 71, 72 and 73, and CDRL1, CDRL2 and CDRL3 of SEQ ID NOs: 77, 79 and 82;
and wherein each CDR may comprise one or two amino acid substitutions.

9. The antigen-binding protein according to any of claims 1 to 8, wherein the antigen binding protein:
(i) competes for binding to an IgG1 antibody comprising the V_{L} of SEQ ID NO: 83, 84 or 85 and the V_{H} of SEQ ID NO: 74 or 75, or
(ii) binds to the same epitope as an IgG1 antibody comprising the V_{L} of SEQ ID NO: 83, 84 or 85 and the V_{H} of SEQ ID NO: 74 or 75.

10. A nucleic acid or nucleic acids encoding the antigen-binding protein according to any one of claims 1 to 9.

11. A vector comprising the nucleic acid or nuclei acids according to claim 10.

12. A recombinant cell expressing the antigen-binding protein according to any one of claims 1 to 9, the nucleic acid or nucleic acids according to claim 10, or the vector according to claim 11.

13. A pharmaceutical composition comprising the antigen-binding protein according to any one of claims 1 to 9, the nucleic acid or nucleic acids according to claim 10, the vector according to claim 11, or the recombinant cell according to claim 12, and a pharmaceutically acceptable excipient.

14. The antigen-binding protein according to any one of claims 1 to 9, the nucleic acid or nucleic acids according to claim 10, the vector according to claim 11, the recombinant cell according to claim 12, or the pharmaceutical composition according to claim 13, for use in medicine.

15. The antigen-binding protein according to any one of claims 1 to 9, the nucleic acid or nucleic acids according to claim 10, the vector according to claim 11, the recombinant cell according to claim 12, or the pharmaceutical composition according to claim 13, for use in the treatment of cancer, preferably
wherein the cancer is selected from the group consisting of lung cancer, prostate cancer, breast cancer, colon cancer, rectum cancer, head cancer, neck cancer, esophagogastric cancer, liver cancer, glioblastoma, cervix cancer, ovary cancer, bladder cancer, kidney cancer, and pancreas cancer, more preferably
wherein the cancer is selected from the group consisting of head and neck squamous cell cancer, non-small cell lung cancer, metastatic colorectal cancer, recurrent or metastatic squamous cell carcinoma of the head and neck.
